# EUROPEAN PATENT APPLICATION

(11) **EP 2 586 867 A1**
(43) Date of publication of application: **01.05.2013**
(21) Application number: 11306385.3
(22) Date of filing: 26.10.2011
(51) Int. Cl.: C12N 15/867

(54) **Transient expression lentiviral vectors, preparation and uses thereof**

(71) Applicant: Newvectys, 75009 Paris (FR)
(72) Inventor: Altemir, Dorothée, 78910 Tacoignieres (FR); Sarkis, Chamsy, 92100 Boulogne-Billancourt (FR)
(74) Representative: Starck-Loudes, Anne-Caroline

(57) **Abstract**

The present invention describes a recombinant lentiviral vector which cannot by itself achieve complete reverse transcription as well as its uses, in particular for transiently transferring *in vitro,* ex *vivo* or *in vivo* at least one ribonucleic acid sequence of interest in a cell. Such a transient transgene expression is of interest in the context of research, therapy and more generally in the field of biotechnology.

## Description

The present invention describes a recombinant lentiviral vector which cannot by itself achieve complete reverse transcription as well as its uses, in particular for transiently transferring *in vitro,* ex *vivo* or *in vivo* at least one ribonucleic acid sequence of interest in a cell. Such a transient transgene expression is of interest in the context of research, therapy and more generally in the field of biotechnology.

### BACKGROUND

As stated by R Scott McIvor (Molecular Therapy, 2011, May; 19(5):822-3, Therapeutic Delivery of mRNA: The Medium Is the Message), it seems as though every advance in gene transfer and expression needs to somehow provide a solution to the problem of genotoxicity. This rely on developing an alternative to gene transfer methods involving a DNA intermediate, which necessarily present a potential risk of insertional mutagenesis, no matter they are intended to be integrative or not.

Messenger RNA (mRNA) has several advantages over DNA for gene transfer and expression, including the lack of any requirement for nuclear localization or transcription and the nearly negligible possibility of genomic integration of the delivered sequence.

However, the development of mRNA as therapeutic faces the same challenge as any nucleic acid: delivery. It is therefore likely that substantial improvements will be required in the efficiency of mRNA delivery and translation into protein product to reach a level that is of more general therapeutic utility.

The present invention now advantageously addresses these drawbacks and provides a new solution for the use of mRNA as a source of gene product usable *in vitro,* ex *vivo* or *in vivo* for both therapeutic or non-therapeutic (for example research and transgenesis) applications. The present invention more widely answers the long felt need for safe, efficient and transient gene transfer tools as further developed below.

Transiently transferring a nucleic acid or a protein, also herein respectively identified as "recombinant nucleic acid" or "transgene" and "recombinant protein", into a target cell is a major issue in the biotechnological field, in particular in a therapeutic or experimental context.

In the context of therapy, such a transient expression may be mandatory for instance for safety reasons, i.e. in order to avoid deleterious effects of a sustained expression of the recombinant protein in the subject exposed to this therapy or to prevent permanent integration of the recombinant nucleic acid into the host genome by preventing the generation of DNA intermediate forms of the recombinant nucleic acid.

In the context of non-therapeutic uses, the transient expression may be required for example in order to avoid deleterious effects such as positional effects or genomic toxicity. For example, in the context of transgenesis, the transient expression of a DNA modifying enzyme may be advantageous in the zygote or early in the development of the organism to specifically modify a target locus. In this context, avoiding DNA intermediate forms encoding the DNA modifying enzyme in the organism would advantageously prevent potential integration of the genome encoding the DNA modifying enzyme, consequently preventing constitutive expression of said DNA modifying enzyme in said organism.

One may further wish to limit *in vitro,* ex *vivo* or *in vivo* the expression of a recombinant peptide, for example when such expression is only transiently required at a particular moment of a biological process.

Among the technical options currently available to allow the transient delivery of a nucleic acid or of a peptide, the skilled person may select either a non-viral or a viral delivery method. The non-viral delivery method may be a direct peptide delivery method or a direct nucleotide delivery method. The viral delivery method may imply the use of replicative RNA viruses, such as RNA (+) viruses and RNA (-) viruses; or transpackaging of fusion proteins in non-replicative retroviral vectors.

### Non-viral delivery methods

Direct delivery of protein is chosen in specific contexts, such as for the delivery of a ligand of a cell membrane receptor *in vitro* or ex *vivo.* However, in most cases, if the action of the delivered factor is to be intracellular, the efficiency of direct delivery of protein in the culture media may be limiting and other methods should be considered. In addition, direct protein delivery does not allow for targeting a specific cell type, which is a particular issue for *in vivo* applications.

According to another method used in the art, nucleotide sequences (DNA and/or RNA) can be directly delivered to cells by mean of current transfection protocols based on chemical or physical methods, for *in vitro,* ex *vivo* or *in vivo* uses.

The cationic polymers (e.g. diethylaminoethyl (DEAE) -dextran, poly(I-lysine), dendrimers, polyethylenimine (PEI)) and the lipid vectors (e.g. liposomes or lipoplexes such as 1,2-dioleoyl-3-trimethylammonium-propane (DOTAP)) are examples of chemical compounds used to transfer nucleic acids.

Physical methods can also be considered for the transient delivery of nucleic acids into a target cell, such as electroporation. For DNA transfer, the strength of the electric shock required is to be high enough to allow the material to reach the cell nucleus. Such a shock may however be responsible for cell damages.

One can also consider delivering nucleic acid molecules by direct micro-injection into the target cells. This method is mainly applied for transgenesis purpose. It can also be used for gene transfer into poorly permissive cells. Micro-injection is limited to *in vitro* applications and it is a tedious method, highly time consuming (cell by cell) which has not been automated so far for most cells.

These non-viral methods can be of interest under certain circumstances such as transient gene transfer into cell lines *in vitro* or for example in the muscle *in vivo.* Though, they are in most cases of limited efficiency, do not allow targeting a specific cell type and are not easily implemented for they require large amount of highly purified recombinant nucleic acid and some of the recombinant nucleic acid based compositions, complexed with lipid compounds for instance, are particularly unstable and cannot be stored. In addition, in the case of plasmid DNA, bacterial sequences are retained that can have deleterious effects.

### Viral delivery methods of RNA

Viruses are nucleoprotein particles transferring their nucleic information into a target cell and hijacking it for their own replication. For years, they have been modified to be used for gene transfer purpose *in vitro,* ex *vivo* and *in vivo.* "Viral vectors", also herein identified as "virus-derived vectors" or "vector particles", are mostly deprived of virulence factors and non-replicative. They further allow the expression of a sequence of interest: the transgene. They often overcome the lack of efficiency of non-viral gene transfer methods, taking advantage of the strategies viruses have developed over their evolution.

Viruses can be classified in several families, depending on the nature of their nucleic acid content and how it is processed during the viral cycle: RNA viruses contain RNA molecules ((+), (-) or double-stranded), DNA viruses contain DNA molecules (single- or double-stranded) and reverse transcribing viruses have their genome as RNA or DNA depending on the step of the replication cycle.

Among RNA viruses, RNA (+) viruses can be used for transient transfer of RNA because they are never present in the cell as a DNA molecule, i.e. they do not have DNA intermediates, thus precluding in theory any persistence of the viral information and subsequent mutagenic risk. Their genome is processed in the cell cytoplasm like a messenger RNA and is translated by cellular ribosomes for the production of viral proteins. One can thus modify them to carry only the information of interest. Viral vectors have been developed from several RNA (+) viruses including alphavirus (e.g. Sindbis, Semliki forest virus, Venezuelian equine encephalitis), picornavirus (e.g. poliovirus), or flavivirus (e.g. Kunjin virus).

Among those kinds of RNA (+) virus derived vectors, alphaviruses are preferred because of their ability to transduce a large quantity of exogenous proteins into cells of a large range of species. However they exhibit cytopathic effects precluding their use for most therapeutic purposes.

The RNA (-) viruses group comprises most of the main human pathogens, including flu, rabies and measles viruses. The RNA (-) genome is not infectious by its own but has to be associated with a RNA-dependent RNA-polymerase, which will generate the (+) strand.

While several viruses of this family have been used to develop RNA vectors, including measles, the most used one is the Sendai virus (SeV), a paramyxoviridae. The Sendai virus is not genotoxic and can replicate in a large range of mammalian tissues. It is the causative agent of respiratory affections in mice, Guinea pigs, hamsters, rats and in rare cases in pigs, but is not pathogenic for humans. Sendai derived vectors are used in few therapeutic strategies, for instance in cystic fibrosis mouse models.

Nevertheless, one of the major drawbacks of these vectors derived from RNA (+) and RNA (-) viruses is the replication of the RNA genome and the formation of non-transmissible virus like particles (NTVLP) into the host cell, leading to toxicity and cell death in many cases.

### Viral delivery methods of protein (trans-packaging of fusion proteins with HIV-1 derived particles)

An approach to transiently bring a protein into a target cell consists in the trans-packaging of a protein of interest in vector particles derived from HIV-1. This trans-packaging can be achieved by a fusion with naturally encapsidated proteins.

Among the viral proteins investigated as trans-packaging partner is VPR (Viral Protein R), which has various functions in the HIV-1 cycle and pathogenicity. The fusion of a heterologous protein of interest to VPR allows one to encapsidate this protein. Such a strategy has for instance been tested for trans-complementation assays to study the functions of integrase and reverse transcriptase during the retroviral cycle. It can also be used to vectorise therapeutic proteins.

The main drawback of this VPR trans-packaging lies in the primary functions of VPR itself regarding cell cycle arrest and its pro-apoptotic and cytotoxic effects, all properties which originally drove the removal of VPR from the improved generation of HIV-1 vectors and which are retained in the fusion proteins. Another disadvantage is the relatively poor efficiency of trans-packaging, depending on the nature of the fusion protein.

Integrase is another viral protein that has been recently used, with mitigated successes, for trans-packaging of heterologous proteins in HIV-derived particles, to visualize cell/particle interactions (fusion with reporter genes), to develop targeted integration vectors (fusions with specific DNA binding domain such as LexA or ZFN), and to vectorise protein of interest (fusion with p53).

Based on fusion with either VPR or integrase (IN) or any other viral protein, the efficiency of trans-packaging strategy is limited by important factors: the low number of proteins that can be delivered and the functionality of the fused protein. Indeed, although the number of encapsidated VPR protein is high in regard to the particle, the packaging efficiency of a fusion protein is expected to be lower, due to the limited size of the particle; this is also true for IN fusion proteins. This low delivery efficiency can, in particular cases, preclude the use of this method. In addition, a protein, which is fused to VPR or IN may lose its function. In consequence, fusion is to be designed with caution in a way not to alter the protein of interest.

The present invention now offers a solution to the problems of the art and provides new tools and vectors for transiently expressing a transgene *in vitro,* ex *vivo* or *in vivo* with optimal safety.

### BRIEF DRESCRIPTION OF THE INVENTION

Inventors now herein provide new types of RNA vector derived from lentiviruses, in particular vectors as defined in the claims. These vectors are unable to achieve reverse transcription of their genome typically through the alteration of the PBS present in the vector genome or through deletion of said PBS from said vector genome.

The present invention describe lentiviral vectors comprising a mutation of the PBS, typically a deletion of the PBS, that indeed prevent the reverse transcription while preserving encapsidation of the lentiviral RNA genome, thus allowing for an efficient gene transfer of the recombinant lentiviral RNA vector genome.

Inventors herein demonstrate that such vectors, preferably vectors containing a complete deletion of the PBS, allow transferring the vector RNA genome into a host target cell to transiently express a recombinant protein of interest for various purposes such as therapeutic applications or biotechnological applications.

The invention is concerned particularly with a recombinant lentiviral (derived-) vector which cannot by its own achieve complete reverse transcription wherein the vector comprises a recombinant ribonucleic vector genome comprising a 5' LTR retroviral sequence and a 3' LTR retroviral sequence flanking: a functional retroviral psi encapsidation sequence and at least one transgene. Preferably, the recombinant retroviral vector genome is devoid of the gag, *pol* and/or *env* gene, for example of the gag and pol genes, of the gag and *env* genes or of the pol and *env* genes, even more preferably of the *gag, pol* and *env* genes. At least one of the *gag, pol* and *env* genes, as well as any fragments thereof, can intentionally be reintroduced in the vector genome as a "transgene", for example in the context of vaccination. Typically, the gag gene may be reintroduced.

Such lentiviral vectors of the invention, which cannot by their own achieve reverse transcription are also herein identified as reverse transcription deficient or reverse transcription defective vectors.

An example of recombinant lentiviral vector according to the invention is a lentiviral vector, which cannot by its own achieve complete reverse transcription wherein the vector comprises a recombinant ribonucleic vector genome comprising a 5' LTR retroviral sequence and a 3' LTR retroviral sequence preferably flanking: a functional retroviral psi encapsidation sequence, and at least one transgene. The recombinant retroviral vector genome possibly further comprises a RNA nuclear export element such as a Rev responsive element (RRE) from HIV-1, a flap sequence (also herein identified as central polypurine tract - central termination sequence, cPPT-CTS), a splice donor site (SD), a splice acceptor site (SA), and/or a promoter.

Another object of the invention relates to a composition comprising a lentiviral vector according to the invention.
A particular example of such a composition is a pharmaceutical composition (for example a vaccinal composition) comprising a lentiviral vector according to the invention and preferably a pharmaceutically acceptable excipient.

The invention also relates to methods, vectors and compositions for the transient expression of at least one transgene *in vitro,* ex *vivo* or *in vivo.*

The object of the invention is also any method for producing a lentiviral vector as defined above in a producer cell, consisting in particular in the expression of (i) appropriate transcomplementation cassettes, resulting in the production of lentiviral enveloped capsid, and (ii) of an appropriate vector cassette, resulting in the production of a lentiviral recombinant ribonucleic vector genome that will be encapsidated in the lentiviral enveloped capsid.

A method according to the present invention is a method for preparing a lentiviral vector which cannot by its own achieve complete reverse transcription according to the invention, wherein said method comprises expressing within a cell:
a. a vector cassette encoding for a lentiviral recombinant ribonucleic vector genome comprising a 5' LTR retroviral sequence and a 3' LTR retroviral sequence flanking: a functional retroviral psi encapsidation sequence and at least one transgene, and wherein the recombinant ribonucleic vector genome (i) does not comprise a primer binding site (PBS) or (ii) comprises a PBS comprising a mutation or a misplaced PBS, thereby preventing the reverse transcription of said recombinant ribonucleic vector genome, and
b. a transcomplementation envelope cassette encoding for an envelope glycoprotein, and
c. a transcomplementation capsid cassette encoding for sequences derived from a lentiviral genome comprising retroviral gag and *pol* sequences, said transcomplementation capsid sequences lacking any functional psi encapsidation signal, and said transcomplementation capsid sequences being optionally split into several cassettes, and
   recovery of the lentiviral vectors produced.

Another object of the invention is a lentiviral vector obtainable with a method as herein described.

Another object of the invention is a nucleic acid sequence comprising a vector cassette encoding for a lentiviral recombinant ribonucleic vector genome comprising a 5' LTR retroviral sequence and a 3' LTR retroviral sequence flanking: a functional retroviral psi encapsidation sequence and at least one transgene, and wherein the recombinant ribonucleic vector genome (i) does not comprise a primer binding site (PBS) or (ii) comprises a PBS comprising a mutation or a misplaced PBS, thereby preventing the reverse transcription of said recombinant ribonucleic vector genome, and optionally:
- a nucleic acid sequence comprising a transcomplementation envelope cassette encoding for an envelope glycoprotein, and/or
- a nucleic acid sequence comprising a transcomplementation capsid cassette encoding for sequences derived from a lentiviral genome comprising retroviral gag and pol sequences, said transcomplementation capsid sequences lacking any functional psi encapsidation signal, and said transcomplementation capsid sequences being optionally split into several cassettes

The nucleic acid sequence can be selected for example from a linear nucleic acid sequence, a plasmid, an artificial chromosome, a viral vector genome, and a transposon.

A further object of the invention is a cell or cell line comprising a nucleic acid sequence as herein described, in particular a cell or cell line expressing, in a stable or controlled (for example inducible) manner, a lentiviral ribonucleic vector genome to be encapsidated, which lentiviral vector genome does not comprise a primer binding site (PBS) or comprises a PBS comprising a mutation or a misplaced PBS, thereby preventing the reverse transcription of said recombinant ribonucleic vector genome.

Another object of the invention is a composition, for example a pharmaceutical composition, comprising a vector, a nucleic acid sequence, and/or a cell or a cell line as herein described, and optionally a pharmaceutically acceptable excipient.

Another object of the invention is a kit comprising a vector, a nucleic acid sequence, a cell or a cell line, and/or a composition as herein described, and preferably written instructions for using the kit.

A further object of the invention relates to uses of any product as herein described (vector, nucleic acid sequence, cell, cell line, composition and kit of the invention) for transiently expressing at least one transgene *in vitro,* ex *vivo* or *in vivo.*

### DETAILED DESCRIPTION OF THE INVENTION

Unless defined otherwise, all technical and scientific terms used herein have the same meaning as commonly understood by one of ordinary skill in the art (e.g. in cell biology, in molecular biology, nucleic acid chemistry and biochemistry). In order to assist with the understanding of the invention several terms are defined herein.

Unless otherwise indicated, the practice of the present invention employs conventional techniques of chemistry, molecular biology, microbiology, recombinant DNA technology, chemical methods, pharmaceutical formulations and delivery and treatment of patients, which are within the capabilities of a person of ordinary skill in the art. Such techniques are also explained in the literature, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John INiley & Sons, New York, N. Y.); B. Roe, J. Crabtree, and A. Kahn, 1996, DNA Isolation and Sequencing: Essential Techniques, John INiley & Sons; J. M. Polak and James O'D. McGee, 1990, In Situ Hybridisation: Principles and Practice, Oxford University Press; M. J. Gait (Editor), 1984, Oligonucleotide Synthesis: A Practical Approach, IRL Press; and D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

The term **"amino acid"** in the context of the present invention is used in its broadest sense and is meant to include naturally occurring L a-amino acids or residues. The commonly used one and three letter abbreviations for naturally occurring amino acids are used herein: A=Ala; C=Cys; D=Asp; E=Glu; F=Phe; G=Gly; H=His; I=Ile; K=Lys; L=Leu; M=Met; N=Asn; P=Pro; Q=Gln; R=Arg; S=Ser; T=Thr; V=Val; IN=Trp; and Y=Tyr (Lehninger, A. L., (1975) Biochemistry, 2d ed., pp. 71-92, Worth Publishers, New York). The general term 'amino acid' may further include D-amino acids, retro-inverso amino acids as well as chemically modified amino acids such as amino acid analogues, naturally occurring amino acids that are not usually incorporated into proteins such as norleucine, and chemically synthesised compounds having properties known in the art to be characteristic of an amino acid, such as β-amino acids. For example, analogues or mimetics of phenylalanine or proline, which allow the same conformational restriction of the peptide compounds as do natural Phe or Pro, are included within the broad definition of amino acid. Such analogues and mimetics are referred to herein as 'functional equivalents' of the respective amino acid. Other examples of amino acids are listed by Roberts and Vellaccio, The Peptides: Analysis, Synthesis, Biology, Gross and Meiehofer, eds., Vol. 5 p. 341, Academic Press, Inc., N.Y. 1983, which is incorporated herein by reference.

The term **"peptide"** as used herein refers to a plurality of amino acids joined together in a linear or circular chain. The term oligopeptide is typically used to describe peptides having between 2 and about 50 or more amino acids. Peptides larger than about 50 amino acids are often referred to as polypeptides or proteins. For purposes of the present invention, however, the term 'peptide' is not limited to any particular number of amino acids, and is used interchangeably with the terms 'polypeptide' and 'protein'.

The terms **"nucleic acid", "polynucleotide",** and **"oligonucleotide"** are used interchangeably and refer to a deoxyribonucleotide (DNA), ribonucleotide (RNA) polymer, in linear or circular conformation, in either single- or double-stranded form and in any combination thereof (for example a hybrid DNA/RNA molecule). For the purposes of the present invention such DNA or RNA polymers may include natural nucleotides, non-natural or synthetic nucleotides, and mixtures thereof. Non-natural nucleotides may include analogues of natural nucleotides, as well as nucleotides that are modified in the base, sugar and/or phosphate moieties (e.g. phosphorothioate backbones). Examples of modified nucleic acids are PNAs and morpholino nucleic acids. Generally an analogue of a particular nucleotide has the same base-pairing specificity, i.e. an analogue of G will base pair with C. For the purposes of the invention, these terms are not to be considered limiting with respect to the length of a polymer.

A **"gene",** as used herein, is the segment of nucleic acid (typically DNA) that is involved in producing a peptide or ribonucleic acid gene product. It includes regions preceding and following the coding region (leader and trailer) as well as intervening sequences (introns) between individual coding segments (exons). Conveniently, this term also includes the necessary control sequences for gene expression (e.g. enhancers, silencers, promoters, terminators etc.), which may be adjacent to or distant to the relevant coding sequence, as well as the coding and/or transcribed regions encoding the gene product.

The phrase **"genomic sequence"** includes genes and fragments or partial gene sequences. It may refer to non-coding and/or coding gene sequences. Such genomic sequences may be within a gene, or it may be isolated or extracted from its corresponding gene, such as an isolated nucleic acid molecule, vector or plasmid. Genomic sequences include those present in chromosomes whether natural or artificial, and whether in the nucleus or other organelles (e.g., mitochondria and chloroplasts). The genomic sequences may be animal (e.g. mammals, such as humans), or may be viral, parasitic, bacterial or fungal (e.g. yeast).

The expressions ***"in vitro"*** and ***"in vivo"*** each defines the other, more precisely *"in vivo"* is understood as "in the naturally occurring conditions", while *"in vitro"* is understood as "in artificial conditions", said conditions being defined by the subject it refers to. For instance, the "naturally occurring conditions" for a cell is "within an organism"; the term *"in vivo",* when referring to a cell will thus be understood as "within an organism", while the term *"in vitro"* will be understood as "out of an organism", for instance in a Petri dish. As another example, the "naturally occurring conditions" for a retroviral integrase is "in the context of a retroviral particle" or "in the context of a retroviral vector particle" (i.e. respectively within said retroviral particle or a cell being infected by said retroviral particle or within said retroviral vector particle or a cell being transduced by said retroviral vector particle); the term *"in vivo",* when referring to a retroviral integrase will then be understood as for example "in the context of a retroviral (vector) particle" or "in the context of a cell being infected (or transduced) by said retroviral (vector) particle", while the expression *"in vitro"* will be understood as "out of a retroviral (vector) particle or a cell", for example "in a solution".

The expression "***ex vivo**"* refers to *"in vitro",* as previously defined, and usually implies that the subject is transiently taken out of its natural condition before being reintroduced *in vivo,* i.e. in its naturally occurring conditions. A typical example of use of the term "*ex vivo*" is in the context of stem cells manipulation: stem cells are collected from an organism, treated "*ex vivo*", for instance submitted to transduction by retroviral vectors, and then reinfused to the organism, i.e. *"in vivo".*
More specifically in the context of the present invention, when referring to the use of retroviral vector, the term "*in vivo*" will refer to the naturally occurring conditions of the target cells, i.e. within an organism, while *"in vitro"* and "ex *vivo*" will refer to artificial conditions of the target cells, i.e. out of an organism, for instance in a Petri dish.

The term **"PBS"** refers to the **"primer binding site"** in a retroviral genome. The process of reverse transcription is initiated at a particular sequence of the RNA genome: the primer binding site (PBS). The first DNA strand (-) synthesis is initiated by the binding of a specific tRNA on the primer binding site, a sequence of 18 nucleotides, located in 3' from the 5' LTR. For example, the PBS in HIV-1 is recognised by a tRNA Lys3, as another example, the PBS in BIV is recognised by a tRNA Lys1,2. The U5 and R regions of the 5' LTR are synthesised by elongation of the tRNA thanks to the DNA polymerase activity of the reverse transcriptase while the corresponding RNA matrix fragments, with the exception of the PBS region, are degraded thanks to the RNAseH activity of the reverse transcriptase.

As explained previously, there is a need in the art for improved tools and methods allowing the transient expression of a transgene in therapeutic as well as non-therapeutic uses. The present invention provides such improved tools and methods.

In the sense of the invention, the term **"transgene"** generally refers to any nucleic acid sequence of interest. This is typically a sequence encoding a peptide, for example an enzyme, a transcription factor, a growth factor, a trophic factor, an hormone, a cytokine, an antibody, a receptor, a differentiation factor, a colony stimulating factor, a suicide protein, a cell-cycle modifying protein, an anti-proliferative protein, a nuclease, a recombinase, a transposase, a neurotransmitter or a precursor thereof. It can be part of an RNA or of a DNA molecule. Preferably, it is a sequence deriving from a cDNA, a gDNA, a RNA, a synthetic nucleic acid, or a combination thereof. The context will indicate whether the term "transgene" refers to a DNA or a RNA sequence. Typically, the transgene includes a sequence encoding a product of interest. Furthermore, the transgene can include one or more transcription termination regions, typically a polyadenylation signal.
The "transgene" may also typically refer to a non-coding sequence. The transgene can be for example selected from a catalytic nucleic acid (for example a ribozyme), an interfering nucleic acid, an antisense nucleic acid, an aptamer, a miRNA or a decoy RNA.

In the context of the present invention, transient expression vectors relate to retrovirus-derived vectors unable to achieve complete reverse transcription of their genome but able to deliver a RNA and optionally (i.e. when said RNA is a coding RNA) produce a recombinant peptide from the messenger RNA molecules delivered by the vector particles. These lentiviral vectors are advantageously able to transiently transfer the transgene to host target cells or organisms.

**"Lentiviral vectors",** in the context of the invention, are defined as engineered vector particles derived from retroviruses such as oncoretroviruses, lentiviruses or spumaviruses. They consist, as the viruses they are derived from, in (i) an envelope made of cell membrane including an envelope glycoprotein, for example derived from an enveloped virus, possibly from a retrovirus or from any other enveloped virus, for example originating from a rhabdovirus, or a cellular glycoprotein or a synthetic glycoprotein - in this case the retroviral vector is designated as "pseudotyped retroviral vector" ; and (ii) a capsid consisting in gag-derived proteins (gag-polyprotein, matrix, capsid, nucleocapsid) containing a nucleic acid genome, namely the **"retroviral vector genome",** possibly retroviral enzymes derived from the pol gene: protease, integrase and reverse transcriptase, and possibly other retroviral proteins such as regulatory and accessory proteins (for instance *tat, nef, rev,* etc.). They possibly contain any other peptide of interest brought within the vector particle by means of fusion with one of the naturally encapsidated viral peptides. Retroviral vectors of the invention are defined in that the ribonucleic acid vector genome they enclose has been engineered (i) to be deprived of transcomplementation sequences required for replication, namely *gag, pol* and *env* (although fragments thereof may remain, e.g. partial or complete sequence of the gag gene), and (ii) to contain an expression cassette allowing the expression of at least one transgene of interest when applied to target cell, said transgene possibly corresponding to *gag, pol, env* and/or fragments thereof, for instance in a vaccination purpose.

**"Reverse transcription"** is a step of the viral cycle typical of the retroviruses by which their single stranded RNA genome is processed into a double stranded DNA molecule, namely the **"provirus".** This reaction is mediated by the reverse transcriptase, a retroviral enzyme and involves two different catalytic activities: a DNA polymerase activity and an RNaseH activity, both of which require divalent cations, typically Mg2+. In addition, DNA polymerase activity requires deoxynucleotides.

The present invention more particularly relates to lentiviral vectors wherein the recombinant ribonucleic vector genome (i) does not comprise a primer binding site (PBS) or (ii) comprises a PBS comprising a mutation or a misplaced PBS, thereby preventing the reverse transcription of said recombinant ribonucleic vector genome. These new vectors have been successfully tested by inventors in therapeutic and non-therapeutic fields as herein illustrated.

A recombinant lentiviral vector, which cannot by its own achieve complete reverse transcription is thus herein described. Such a lentiviral vector of the invention which cannot by itself achieve reverse transcription is also herein identified as reverse transcription deficient or reverse transcription defective vector. This vector comprises a recombinant ribonucleic vector genome, namely the **"retroviral vector genome",** comprising a 5' LTR retroviral sequence and a 3' LTR retroviral sequence flanking: a functional retroviral psi encapsidation sequence and at least one transgene, for example two, three or four transgenes.

The vector of the invention is a lentiviral vector derived from a lentivirus selected for example from HIV-1, HIV-2, SIV, FIV, EIAV, BIV, VISNA, CAEV and any combination thereof.

In the present invention, the PBS of a lentiviral vector genome is altered so that the reverse transcription process cannot be completed and does not lead to the generation of a full double stranded DNA molecule, namely the lentiviral provirus.

Said alteration consist in mutations of the RNA vector genome in a way modifying its 3D structure or not, and preventing the correct binding of the reverse transcriptase, the correct binding of the tRNA, the initiation of the reverse transcription, the elongation of the primer, the strand jumps, and/or the termination of elongation.

In a preferred embodiment of the invention, the alteration of the PBS in a lentiviral recombinant vector genome derived from HIV-1, HIV-2, SIV, FIV and EIAV affects the PBS sequence 5'-TGGCGCCCGAACAGGGAC-3' (DNA sequence SEQ ID NO:15) or corresponding 5'-UGGCGCCCGAACAGGGAC-3' (RNA sequence SEQ ID NO:17), complementary to tRNA Lys3.

In another preferred embodiment of the invention, the alteration of the PBS in a lentiviral recombinant vector genome derived from CAEV, VISNA and BIV affects the PBS sequence 5'-TGGCGCCCAACGTGGGGC-3' (DNA sequence SEQ ID NO:16) or 5'-UGGCGCCCAACGUGGGGC-3' (RNA sequence SEQ ID NO:18), complementary to tRNA Lys1,2.

In yet another preferred embodiment, the alteration of the PBS in a lentiviral recombinant vector genome is a deletion of 18 bp of sequence 5'-TGGCGCCCGAACAGGGAC-3' (DNA sequence SEQ ID NO:15) or 5'-UGGCGCCCGAACAGGGAC-3' (RNA sequence SEQ ID NO:17) for vector genomes derived from HIV-1, HIV-2, SIV, FIV and EIAV and a deletion of 18 bp of sequence 5'-TGGCGCCCAACGTGGGGC-3' (DNA sequence SEQ ID NO:16) or 5'-UGGCGCCCAACGUGGGGC-3' (RNA sequence SEQ ID NO:18) for vector genomes derived from CAEV, VISNA and BIV.

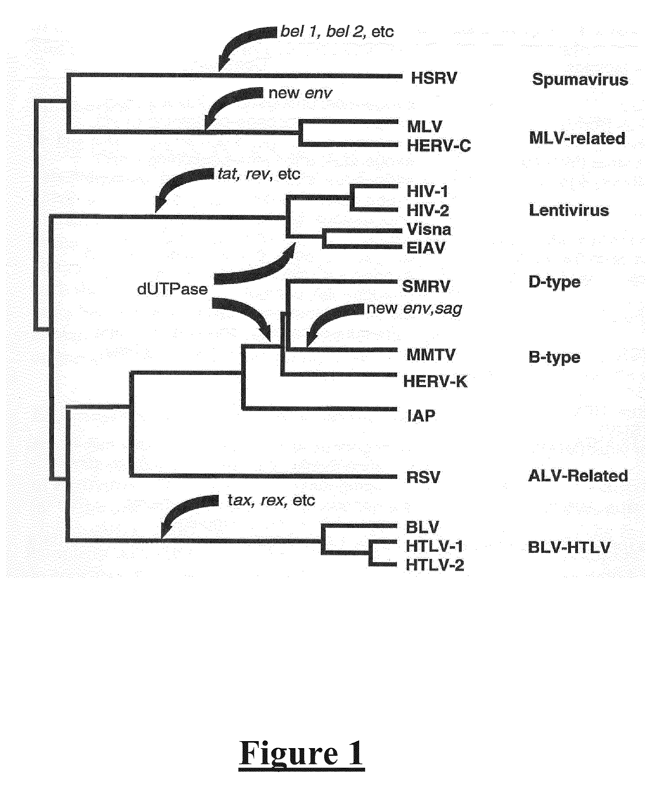

Lentiviruses, from which vectors of the invention are derived, have *gag, pol* and *env* genes flanked by two LTR (Long Terminal Repeat) sequences. Each of these genes encodes for numerous peptides, which are initially expressed in the form of a single precursor polypeptide. The gag gene encodes for the internal structure proteins (matrix, capsid and nucleocapsid). The pol gene encodes for lentiviral enzymes reverse transcriptase, integrase and protease. The *env* gene encodes for viral envelope glycoprotein. Furthermore, the lentivirus genome can contain cis-acting elements, for example elements responsible for exporting out of the nucleus the unspliced viral genomic RNA, which will further be packaged, such as the RRE (Rev Responsive Element) from HIV. The LTR 5' and 3' sequences serve to promote the transcription and also serve as a polyadenylation sequence of the viral RNAs. Sequences necessary for the initiation of reverse transcription of the genome (binding site of the tRNA primer) and for the encapsidation of viral RNA in particles (psi sequence) are adjacent to the LTR 5'. If the sequences necessary for encapsidation are absent from the viral genome, genomic RNA is not actively packaged. Furthermore, the genome of lentiviruses comprises genes encoding for accessory and/or regulatory proteins (distinct from *gag, pol* and *env* proteins) such as, but not limited to (depending on the nature of the virus): src, sag, *Tax, vif, vpr, vpx, vpu, nef, TAT, REV, tmx,* Tas or *Bet* (see **Figure 1****).** For example, the HIV-1 genome contains 7 accessory genes: *vif, vpr, vpx, vpu, nef, TAT* and *REV.*

The lentiviral vector is derived typically from HIV-1, HIV-2, SIV, FIV, EIAV, BIV, VISNA, CAEV. The lentiviral vector may further be derived from a combination of any of the previously mentioned lentiviruses.

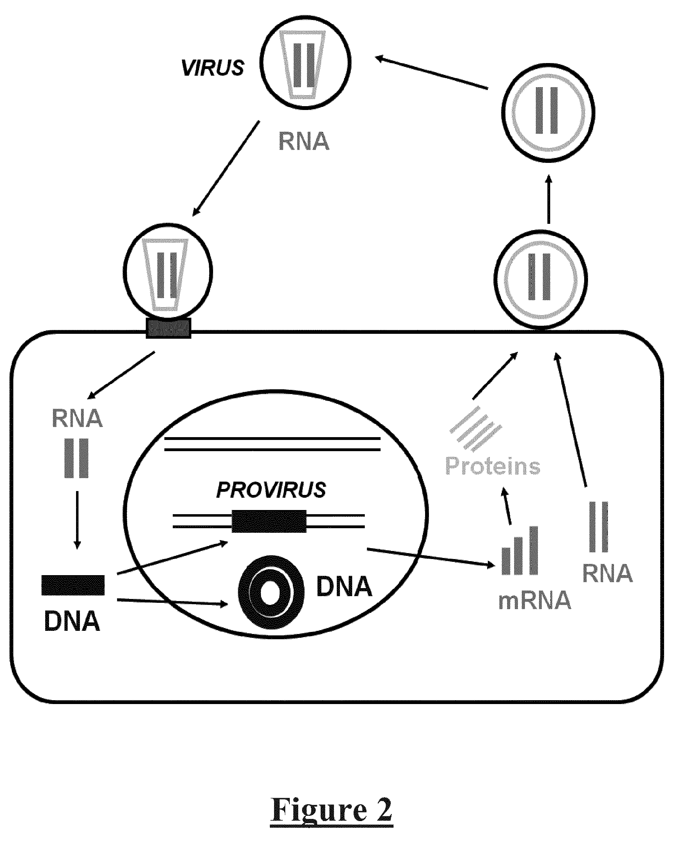

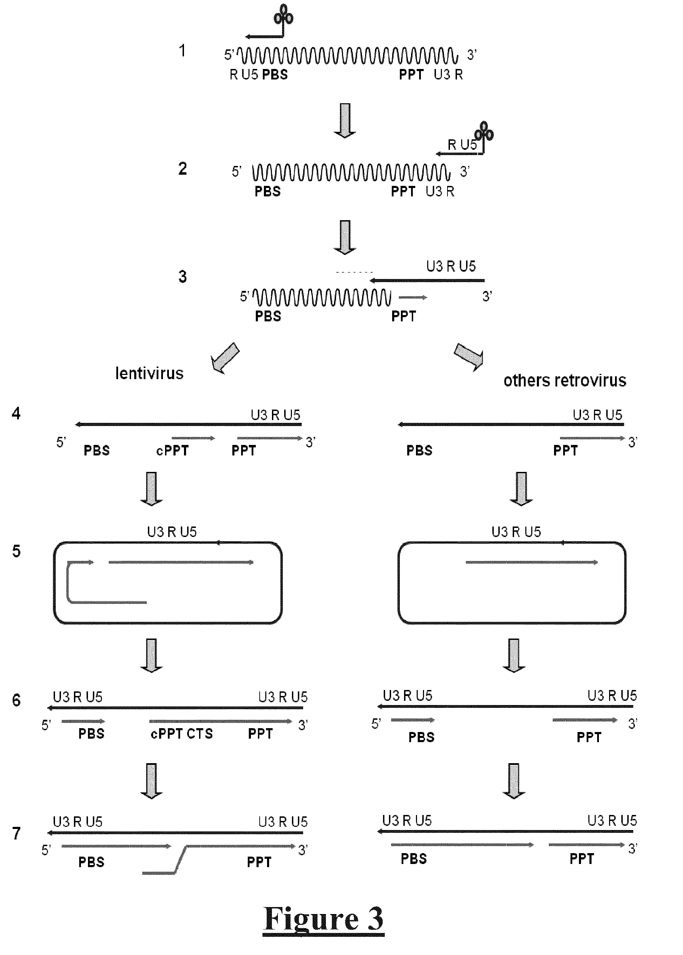

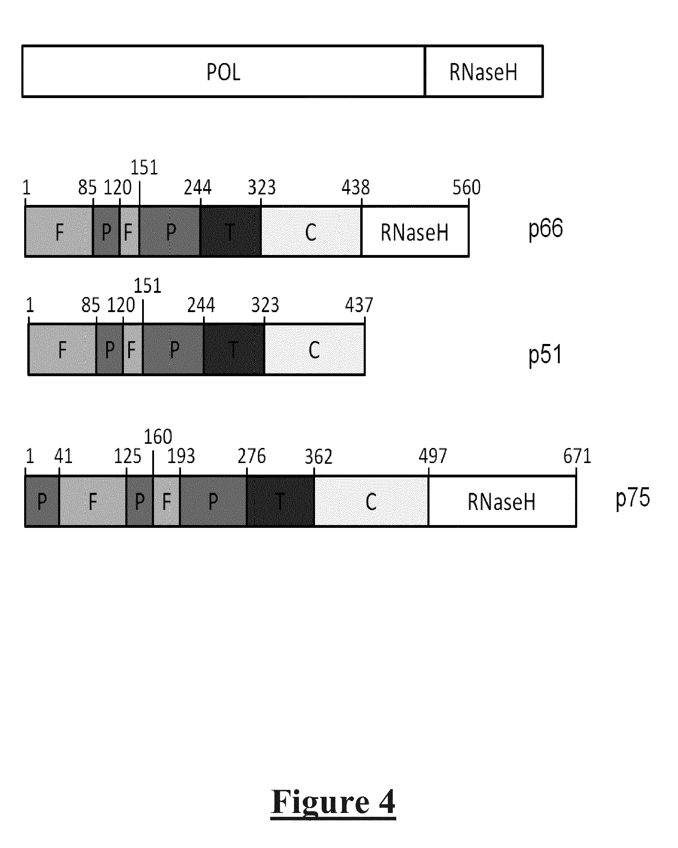

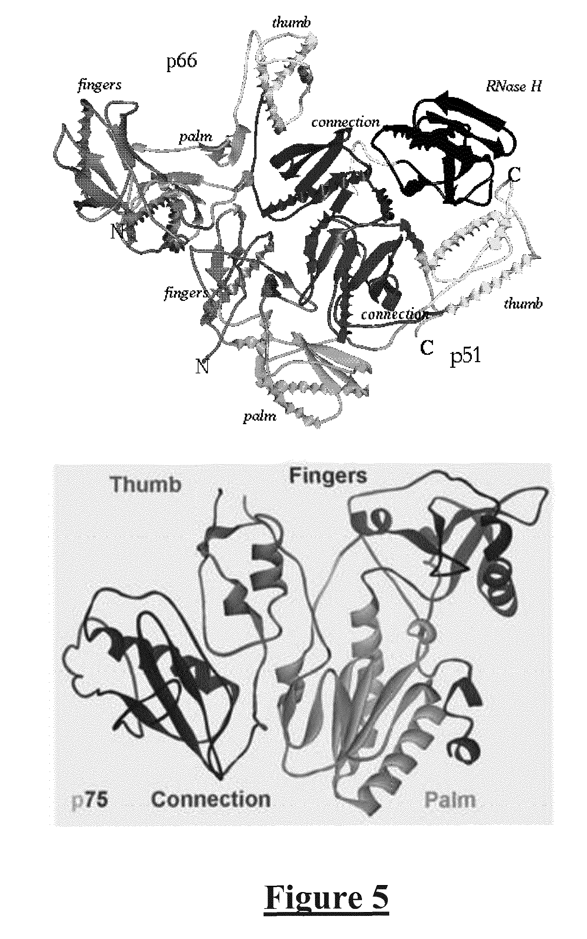

**Reverse transcription** is a typical step of retroviral cycle (see **Figure 2****)** and is normally shared by lentiviruses and lentiviral vectors (see **Figure 3****).** This reaction is mostly cytoplasmic, although it can be initiated within the particle (before the uncoating step) and be completed after nuclear import within the preintegration complex (PIC). It is a multistep process involving three key molecules: the RNA template (with particular nucleic acid sequences and three-dimensional structures), a specific tRNA that serves as a primer for the initiation of the reverse transcription, and the **reverse transcriptase** (RT) viral enzyme which functions as a RNA- and DNA-dependent DNA polymerase (Pol) and a RNA- and DNA-dependent ribonuclease H (RNAseH) (see **Figure 4** and **Figure 5****).** The reverse transcriptase polymerase activity is primer dependent and can transcribe both RNA (RNA-dependent polymerase activity, responsible for the (+) strand synthesis) and DNA (DNA-dependent polymerase activity, responsible for the (-) strand synthesis) templates in a 5'-3' direction. The RNAseH acts on the RNA strand of RNA/DNA duplexes and can catalyse both endo- and exonucleolytic cleavage of such an RNA strand. The RNaseH domain has two particular activities: (a) a polymerase dependent activity, involved in the degradation of the viral RNA simultaneously to the DNA synthesis, and (b) a polymerase independent activity, specifically responsible for the degradation of the polypurine tract (PPT).

In a preferred embodiment, the lentiviral vector of the invention comprise a recombinant ribonucleic vector genome comprising a 5' LTR retroviral sequence and a 3' LTR retroviral sequence flanking: a functional retroviral psi encapsidation sequence and at least one transgene and wherein said recombinant ribonucleic vector genome does not comprise a primer binding site (PBS).
In yet another preferred embodiment, the lentiviral vector of the invention comprises a PBS comprising a mutation or a misplaced PBS, thereby preventing the reverse transcription of said recombinant ribonucleic vector genome.

In the present invention, the PBS can be mutated, displaced or deleted so that the reverse transcription process is altered and cannot be completed, i.e. cannot give rise to full length retroviral double-stranded DNA intermediate molecules in the target cell, and consequently cannot generate a proviral vector genome. This inability to give rise to a full length double stranded DNA molecule is the consequence of alteration(s) of the reverse transcription reaction occurring at the initiation step, the elongation, and/or the termination, the first jump and/or the second jump, and/or during the first strand synthesis and/or the second strand. This inability, in the context of the invention, results from either (i) an absence of a primer binding site (PBS) in the lentiviral vector genome or (ii) a PBS comprising a mutation or a misplaced PBS, thereby preventing the reverse transcription of said recombinant ribonucleic vector genome.

In another embodiment, the recombinant vector genome herein described can further comprises at least one sequence favouring or enhancing the transgene expression, in particular a post-transcriptional regulatory sequence and/or a sequence modifying the stability of mRNAs, for example selected from an intron (natural or synthetic), WPRE, APP 5'UTR, TAU 3'UTR, at least one copy of a miRNA target sequence and any combinations thereof, preferably chosen from INPRE, APP 5'UTR, TAU 3'UTR, and at least one copy of a miRNA target sequence (for example miRNA target sequence of mir 142-3p), even more preferably from two to twelve copies of a miRNA target sequences, typically at least four copies.

In another embodiment, the recombinant vector genome herein described can further comprises other regulatory sequences or elements for example selected from an insulator (MAR, SAR, scs, scs', LCR, etc.), an IRES, a KOZAK or Shine-Dalgarno sequence, and a lentiviral central sequence (cPPT-CTS).

When the vector of the invention is to contain more than one transgene, said transgenes can be linked directly through a fusion construct preferentially including a 2A protease cleavage site, for example originating from the foot-and-mouth disease virus, or alternatively through an IRES sequence.

Transgenes advantageously usable in the context of the present invention can be selected from a catalytic nucleic acid, an aptamer, a miRNA, a decoy RNA, a nucleic acid encoding a biologically active peptide, for example an enzyme, a transcription factor, a growth factor, a trophic factor, a hormone, a cytokine, an antibody, a receptor, a differentiation factor, a colony stimulation factor, a suicide protein, a cell-cycle modifying protein, an anti-proliferative protein, a nuclease, a recombinase, a transposase, and a neurotransmitter or a precursor thereof.

The present invention further includes any composition comprising a vector as herein described, typically a pharmaceutical composition comprising such a vector together with a pharmaceutically acceptable excipient.
Pharmaceutically acceptable excipient or carriers are well known in the art and include but are not limited to saline, buffered saline, dextrose, water, glycerol, sterile isotonic aqueous buffer, and combinations thereof. One example of such an acceptable carrier is a physiologically balanced culture medium containing one or more stabilizing agents such as stabilized, hydrolyzed proteins, lactose, etc. The carrier is preferably sterile. The formulation will be easily selected by the skilled person depending on the chosen mode of administration. Examples of administration methods are further described below.

The lentiviral vectors of the present invention may be administered by injection, for example intravenous injection or subcutaneous injection. The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol), suitable mixtures thereof, and vegetable oils.
The lentiviral vectors of the present invention may also be administered directly to the airways in the form of an aerosol. For use as aerosols, the agents of the present invention in solution or suspension may be packaged in a pressurized aerosol container together with suitable propellants, for example, hydrocarbon propellants like propane, butane, or isobutane with conventional adjuvants. The materials of the present invention also may be administered in a non-pressurized form such as in a nebulizer or atomizer.

Suitable subjects to be treated with a lentiviral vector of the present invention are those requiring the transient delivery of a particular RNA, said RNA being incorporated in the retroviral vector genome as a transgene. Such subjects include human and non-human animals, preferably mammals or avian species. Exemplary mammalian subjects include, without limitation, humans, non-human primates, dogs, cats, rodents, cattle, horses, sheep, and pigs. Exemplary avian subjects include, without limitation chicken, quail, turkey, duck or goose.

The present invention further provides methods for preparing, *in vitro* or *ex vivo,* typically *in vitro,* a lentiviral vector of the invention, which cannot by its own achieve complete reverse transcription.

An example of such a method typically comprises expressing within a cell:
a. a vector cassette encoding for a lentiviral recombinant ribonucleic vector genome comprising a 5' LTR retroviral sequence and a 3' LTR retroviral sequence flanking: a functional retroviral psi encapsidation sequence and at least one transgene, and wherein the recombinant ribonucleic vector genome does not comprise a primer binding site (PBS), and
b. a transcomplementation envelope cassette encoding for an envelope glycoprotein, and
c. a transcomplementation capsid cassette encoding for sequences derived from a lentiviral genome comprising retroviral gag and pol sequences, said transcomplementation capsid sequences lacking any functional psi encapsidation signal, and said transcomplementation capsid sequences being optionally split into several cassettes, and
   recovery of the lentiviral vectors produced.

The term **"cassette"** or **"expression cassette"** in the context of the present invention is to be understood as a nucleic acid sequence possibly involved in the production of one or several RNAs and/or of one or several peptides or polypeptides. The cassette or expression cassette consists in possibly transcribed sequences, for instance but not limited to a gene or a combination of genes, which are possibly associated with functional transcription element(s), namely **"promoter(s)",** it possibly contains additional sequences such as leader and trailer sequences, introns and sequences involved in the regulation or modulation of transcription such as inhibitor, enhancer, stabilizer, internal ribosomal entry site (IRES). The cassette or expression cassette can typically be transferred into target cells for example as part of a plasmid, an artificial chromosome, a viral vector genome (for example a baculoviral or adenoviral vector genome), or of a transposon. This cassette is then possibly expressed in the target cells.
When the cassette or expression cassette is a DNA molecule or part of a DNA molecule, **"expression"** is understood as transcription, i.e. production of a polyadenylated messenger RNA and in case of a coding mRNA, its potential subsequent translation and production of a peptide.
When the cassette is a RNA molecule or part of a RNA molecule, **"expression"** refers to the mRNA translation into a peptide.
Alternatively, when the cassette contains a non-coding RNA (for example a decoy RNA, miRNA, a ribozyme, etc.), "expression" refers to delivery of the RNA into the target cell and its subsequent processing as a catalytic, decoy or interfering RNA.

**"Transcomplementation proteins"** or "Transcomplementation peptides", in the context of the invention, are defined as proteins or peptides that are intended to be part of a retroviral vector particle as a protein but not as nucleic information (coding sequence) and that are expressed in *trans* within a **"producer cell".** Transcomplementation proteins or peptides for example typically include retroviral structural proteins (capsid, nucleocapsid, matrix) encoded by the *gag* gene, retroviral enzymes encoded by the pol gene (protease, reverse transcriptase, integrase), accessory or regulatory peptides (such as *tat, rev* and *net*) and an envelope glycoprotein encoded by the *env* gene. The glycoprotein can be of retroviral origin or can be derived from a non-retroviral enveloped virus or from a cellular glycoprotein or can be a synthetic glycoprotein (such as a chimeric glycoprotein). Genes encoding the transcomplementation proteins or peptides, namely the **"transcomplementation cassettes"** (for example the "transcomplementation envelope cassette" or the "transcomplementation capsid cassette"), are to be present transiently or constitutively (i.e. a transformed cell containing an integrated transcomplementation cassette) and expressed in a constitutive or regulated (for example inducible) manner, in the "producer cells" to produce retroviral vectors typically using a plasmid, an artificial chromosome, a viral vector genome (for example a baculoviral or adenoviral vector genome), a transposon, or any combinations thereof. As an example, plasmids used in the invention to bring the transcomplementation cassettes are also herein identified as **"transcomplementation plasmids",** for example the **"transcomplementation envelope plasmid"** or the **"transcomplementation capsid plasmid".** Retroviral transcomplementation proteins are derived from a single retrovirus or from a combination of different retroviruses, either oncoretroviruses, lentiviruses, or spumaviruses. Expression of the transcomplementation cassettes in producer cells along with the retroviral vector genome cassette leads to the production of the retroviral vector particles.

The **"Retroviral genome",** in the context of the invention, refers to a nucleic acid sequence, which is possibly encapsidated into a retroviral particle, for example (i) when present as a ribonucleic molecule in a **"retroviral particle",** or (ii) when present as a ribonucleic molecule in a cell infected by a **"retrovirus"** and undergoing retroviral replication.
The **"Retroviral vector genome",** also herein identified as **"vector genome"** in the context of the invention refers to a nucleic acid sequence, which is possibly encapsidated into a retroviral vector particle, for example (i) when present in producer cells as a ribonucleic molecule, or when present in the **"retroviral vector particle"** or (ii) when present as a ribonucleic molecule in a cell transduced by a **"retroviral vector".** In the context of the invention, the **"retroviral vector genome"** contains all the cis- and trans-active elements necessary for its proper production and encapsidation, including but not limited to the long terminal repeats (LTR) and the psi encapsidation signal. It also contains one or several cassette(s) encoding the gene of interest or transgene, said cassette possibly containing, as understood in the present invention, any element involved in the transcriptional or post-transcriptional regulation of the coding sequence.
Preferably, the recombinant retroviral vector genome is devoid of the *gag, pol* and/or *env* gene, even more preferably of the *gag, pol* and *env* genes. At least one of the *gag, pol* and *env* genes, as well as any fragments thereof, can intentionally be reintroduced as a "transgene" in the vector genome, for example in the context of vaccination. Typically, the gag gene may be reintroduced.
The **"vector cassette"** in the context of the invention allows the expression of a **"vector genome"** that will be encapsidated in the vector particles in **"producer cells".** The **"vector cassette",** in the context of the invention, is to be present transiently or constitutively (i.e. a transformed cell containing an integrated transcomplementation cassette) and expressed in a constitutive or regulated (for example inducible) manner, in the **"producer cells"** to produce retroviral vector genomes typically using a plasmid, an artificial chromosome, a viral vector genome (for example a baculoviral or adenoviral vector genome), a transposon, or any combinations thereof. As an example, plasmid used in the invention to bring the vector cassette is also herein identified as **"vector plasmid"** or **"transcomplementation vector plasmid".**

**"Producer cells",** in the context of the invention are cells (transiently or constitutively) containing transcomplementation cassettes, which when expressed lead to the production of transcomplementation peptides. Expression of said transcomplementation peptides along with a retroviral vector genome in producer cells allows the production of retroviral vector particles, containing generally two copies of the retroviral vector genome RNA, said retroviral vector particles can be collected in the cell culture medium and further used for *in vitro,* ex *vivo* and/or *in vivo* purposes. Transcomplementation cassettes and vector cassette can be brought into the producer cells by any method known by skilled persons, including but not limited to, plasmid transfection, artificial chromosome transfection, viral vector transduction, for example using adenovirus-derived vectors, baculovirus-derived vectors, herpes simplex virus-derived vectors, or transposon. Transcomplementation cassettes and vector cassette are stably or transiently introduced in the producer cells and are constitutively transcriptionally active or their transcription is dependent on activation of the promoter, for example by a doxycycline-dependent regulation system.

Another example of a method according to the invention comprises expressing within a cell:
a. a vector cassette encoding for a lentiviral recombinant ribonucleic vector genome comprising a 5' LTR retroviral sequence and a 3' LTR retroviral sequence flanking: a functional retroviral psi encapsidation sequence and at least one transgene, and wherein the recombinant ribonucleic vector genome comprises a PBS comprising a mutation or a misplaced PBS, thereby preventing the reverse transcription of said recombinant ribonucleic vector genome, and
b. a transcomplementation envelope cassette encoding for an envelope glycoprotein, and
c. a transcomplementation capsid cassette encoding for sequences derived from a lentiviral genome comprising retroviral gag and pol sequences, said transcomplementation capsid sequences lacking any functional psi encapsidation signal, and said transcomplementation capsid sequences being optionally split into several cassettes, and
   recovery of the lentiviral vectors produced.

In a particular embodiment, a method of the invention for preparing, *in vitro* or ex *vivo,* typically *in vitro,* a lentiviral vector of the invention consists in transient transfection of cells, for example HEK 293T cells, with at least two transcomplementation plasmids as defined previously and one retroviral vector plasmid expressing the ribonucleic retroviral vector genome to be encapsidated (Nat Biotechnol. 1997 Sep;15(9):871-5. Multiply attenuated lentiviral vector achieves efficient gene delivery in vivo. Zufferey R, Nagy D, Mandel RJ, Naldini L, Trono D. Cell. 2000 Apr 14;101(2):173-85. HIV-1 genome nuclear import is mediated by a central DNA flap. Zennou V, Petit C, Guetard D, Nerhbass U, Montagnier L, Charneau P.).

In another particular embodiment, a method of the invention for preparing, *in vitro* or ex *vivo,* typically *in vitro,* a lentiviral vector of the invention consists in stably expressing all or part of the required transcomplementation peptides in a producer cell and further transiently or stably expressing the ribonucleic lentiviral vector genome to be encapsidated, optionally with the other required transcomplementation proteins (J Virol. 1998 Nov;72(11):8463-71. A third-generation lentivirus vector with a conditional packaging system. Dull T, Zufferey R, Kelly M, Mandel RJ, Nguyen M, Trono D, Naldini L.; Mol Ther. 2000 Aug;2(2):170-6. A stable system for the high-titer production of multiply attenuated lentiviral vectors. Klages N, Zufferey R, Trono D.).

Another object of the invention is a nucleic acid sequence comprising a vector cassette encoding for a lentiviral recombinant ribonucleic vector genome comprising a 5' LTR retroviral sequence and a 3' LTR retroviral sequence flanking: a functional retroviral psi encapsidation sequence and at least one transgene, and wherein the recombinant ribonucleic vector genome (i) does not comprise a primer binding site (PBS) or (ii) comprises a PBS comprising a mutation or a misplaced PBS, thereby preventing the reverse transcription of said recombinant ribonucleic vector genome, and optionally:
- a nucleic acid sequence comprising a transcomplementation envelope cassette encoding for an envelope glycoprotein, and/or
- a nucleic acid sequence comprising a transcomplementation capsid cassette encoding for sequences derived from a lentiviral genome comprising retroviral gag and pol sequences, said transcomplementation capsid sequences lacking any functional psi encapsidation signal, and said transcomplementation capsid sequences being optionally split into several cassettes

The nucleic acid sequence can be selected for example from a linear nucleic acid sequence, a plasmid, an artificial chromosome, a viral vector genome (such as a genome derived from a baculovirus, an adenovirus or an herpes virus), and a transposon.

A further object of the invention is a cell or cell line comprising a nucleic acid sequence as herein described, in particular a cell or cell line expressing, in a stable or an inducible manner, a lentiviral ribonucleic vector genome to be encapsidated, which lentiviral vector genome does not comprise a primer binding site (PBS) or comprises a PBS comprising a mutation or a misplaced PBS, thereby preventing the reverse transcription of said recombinant ribonucleic vector genome.

Another object of the invention is a composition, for example a pharmaceutical composition, comprising a vector, a nucleic acid sequence, and/or a cell or a cell line as herein described, and optionally a pharmaceutically acceptable excipient.

Another object of the invention is a kit comprising a vector, a nucleic acid sequence, a cell or a cell line, and/or a composition as herein described, and preferably written instructions for using the kit.

### Applications

Also encompassed by the present invention are any possible uses of a product as herein described (vector, nucleic acid sequence, cell, cell line, composition and kit of the invention) for transiently expressing at least one transgene in *vitro,* ex *vivo* or *in vivo,* in particular of a vector according to the present invention or of a composition comprising such a vector.

There are multiple applications for the transient transfer of nucleic acids, in particular in experimental (e.g. research) and therapeutic fields. Examples of such applications relate to the transfer of functional nucleic acids into target cells and to the transient expression of a protein of interest or transient delivery of a non-coding RNA.

Such a protein of interest may be selected from any protein usable in the context of genetic engineering, of differentiation, of dedifferentiation, of therapy and of research.

In a particular embodiment, the vectors of the invention can be used to transiently express a genetic engineering tool, typically a DNA modifying enzyme. A **"DNA modifying enzyme"** is a natural or synthetic peptide, generally an enzyme, capable of (i) a particular affinity for a DNA molecule or chromatin, possibly a sequence dependent affinity, and (ii) introducing modification in said DNA or chromatin by for example generating a double stranded break in target DNA molecule, removing a sequence from the target DNA molecule, introducing a sequence into a target DNA molecule, modifying the epigenetic imprinting of the target DNA or chromatin, modifying the tridimensional structure and/or ligating ends of one or several DNA molecules.
Such a DNA modifying enzyme can be for example a unidirectional site-specific recombinase, a bidirectional site-specific recombinase, a nuclease (for example Zinc-finger nuclease, meganuclase, TAL effectors), a transposase, a resolvase, a gyrase, a methyl transferase, as well as histone-deacetylase, histone-deacetylase inhibitor, etc.

The use of the vectors of the invention for transiently expressing a DNA modifying enzyme is particularly advantageous, as compared to other typical methods used in the art. For instance, the potential insertion of a recombinant vector genome encoding said DNA modifying enzyme is of potential harm to the cell or the organism. Indeed, DNA modifying enzymes may for example display off-target activities, i.e. non-specific modification of the target DNA or chromatin, leading potentially to genotoxicity, chromosomal aberrations or cell death. The vectors of the invention allow to transiently express the DNA modifying enzyme, thereby preventing the risk of permanent integration of the genome encoding the DNA modifying enzyme, and consequently reducing the risk of off-target activities.

In another particular embodiment, the vectors of the invention can be used to transiently express a differentiation factor in a pluripotent or totipotent cell such as a primary stem cell (embryonic, foetal, juvenile or adult stem cells), an embryonic stem cell line (i.e. ES cells), a progenitor cell, an induced pluripotent stem cell (iPS). Such a differentiation factor can be selected for example from a transcription factor, an epigenetic regulatory factor, a cell cycle modifier, etc., either natural or synthetic.
The use of the vectors of the invention for transiently expressing a differentiation factor is particularly advantageous, as compared to other typical methods used in the art. For instance, the potential insertion of a recombinant vector genome encoding said differentiation factor is of potential harm to the cell or the organism. Indeed, random integration of the recombinant vector genome into the genome of the host cell may induce insertional mutagenesis. Moreover, the vectors of the invention allow to transiently express the differentiation factor, thereby preventing the risk of long-term or constitutive expression of a differentiation factor reducing potential harmful effects of the differentiation factor, and thus allowing to tightly control the duration of the expression of the differentiation factor to the needed time.

In another particular embodiment, the vectors of the invention can be used to transiently express at least one dedifferentiation factor, typically between two and four dedifferentiation factors. According to methods previously described (e.g.: patent application W02009/061442 A1), one can induce the dedifferentiation of a differentiated cell into a stem cell-like cell and for example generate so-called "iPS" typically from animal, preferentially mammal, for example human, non-human primates, swine (porcine), bovine, caprine, canine, or feline.
This dedifferentiation can be achieved by transient delivery of one or several factors such as for example oct-4, sox-2, kfl-4, myc and any combinations thereof, using the vectors of the invention.
The use of the vectors of the invention for transiently expressing a dedifferentiation factor is particularly advantageous, as compared to other typical methods used in the art. For instance, the potential insertion of a recombinant vector genome encoding said dedifferentiation factor is of potential harm to the cell or the organism. Indeed, random integration of the recombinant vector genome into the genome of the host cell may induce insertional mutagenesis. Moreover, the vectors of the invention allow to transiently express the dedifferentiation factor, thereby preventing the risk of long-term or permanent expression of a dedifferentiation factor reducing potential harmful effects of the dedifferentiation factor, and thus allowing to tightly control the duration of the expression of the dedifferentiation factor to the needed time. Indeed, dedifferentiation factors are often oncogenic and their permanent expression may thus induce the generation of cancer cells.

Another field wherein the present invention can be used relates to vaccination. Retroviral vectors of the invention are particularly suitable for use in the field of medicinal treatment where especially an immune response, including a cellular immune response, elicited by endogenously expressed antigen is beneficial or necessary; accordingly, the invention provides tools for the design of vaccination protocols for use in hosts in need of preventive or curative treatment against intracellular pathogenic organisms, including viruses, or more generally against a pathogenic state, including to perform gene therapy *in vivo.* In particular, the invention is appropriate to elicit a cellular immune response, which may be protective when administered to a host, especially when prevention or treatment of viral infection is desired, and possibly to prevent development of pathogenesis associated with the infection.
In the context of eliciting an immune response, preferably a cellular immune response, either for a prophylactic or a therapeutic purpose, retroviral vectors of the invention are intended to transduce antigen presenting cells, and especially dendritic cells. They are thus pseudotyped with a glycoprotein enabling the fusion of said retroviral vector particle of the invention with the target dendritic cells, preferably the VSV glycoprotein, as herein described.
Prophylactic and/or therapeutic vaccination using the vector of the invention can be used to treat human as well as non-human animals, preferably mammals or avian species. Examples of mammalian subjects include, without limitation, humans, non-human primates, dogs, cats, rodents, cattle, horses, sheep, and pigs. Examples of avian subjects include, without limitation chicken, quail, turkey, duck or goose.
The use of retroviral vectors of the invention allows the transient expression of an antigen for inducing an immune reaction along with optimized conditions regarding safety since the uniquely RNA state of the retroviral vector genome prevents any persistence of the genetic information in the target cell. As a consequence, such transient retroviral vectors can be advantageously used for vaccination of a subject against any retrovirus, for example for the vaccination of human subjects against HIV infection, without any risk of recombination of the vector genome with the wild type viral genome, thus precluding any risk of dissemination of the vector genome.

Other advantages and applications of the invention are illustrated in greater detail in the following examples, which must be considered as illustrative and non-restrictive.

### KEYS TO THE FIGURES

**Figure 1****: Relationship of retrovirus groups.**
   The relationships are based on amino acid sequence similarities in the Reverse transcriptase protein of the groups shown. The insertion of sequence from another source is indicated (arrows), typically the accessory and/or regulatory genes, such as bel 1, bel 2, *tat, rev, sag, tax, rex.* Lentiviruses, oncoretroviruses (MLV-related, D-type retroviruses, B-type retroviruses, ALV-related retroviruses, BLV-HTLV group) and Spumaviruses (also herein identified as foamy viruses) are represented. Note that the scale is approximate and not necessarily linear. (HERV-C, an ancient endogenous provirus of humans; SMRV, squirrel monkey retrovirus; HSRV, human spumavirus (also herein identified as human foamy virus); MLV, murine leukemia virus; HIV, human immunodeficiency virus, MMTV, mouse mammary tumor virus; IAP, intracisternal type A particle, a murine endogenous retrovirus; RSV, Rous sarcoma virus; BLV, bovine leukemia virus; HTLV, human T cell leukemia virus.)
**Figure 2****: Retroviral cycle.**
   Retroviral cycle first requires the binding of the particle to the cell membrane, followed by cell entry and uncoating of the particle. The uncoating step leads to the introduction into the cytoplasm of the nucleoproteic core, consisting of gag- and pol-derived proteins, full length genomic RNA (2 molecules) and RT (reverse transcriptase) protein. While this core is progressing toward the cell nucleus the reverse transcription occurs, leading to the generation of a double stranded DNA molecule from the RNA vector genome, namely the provirus. After nuclear import, the nucleoproteic complex is named preintegration complex (PIC). The DNA genome is then processed by the integrase (IN) protein, which mediates the recombination within the host cell chromatin, leading to the stable integration of the genome into a host cell chromosome. Another possible fate of the vector genome is the circularization, this process involves different cellular and viral factors; the genome thus remains in the cell nucleus as an extrachromosomal DNA circle. Both DNA forms, integrated as well as circular extrachromosomal forms, can be transcribed by the host cell machinery, leading to the production of RNA, which can be exported to the cell cytoplasm. These newly formed RNA can subsequently be translated, leading to expression of viral proteins necessary for generating new particles, which encapsidates unspliced RNA genomic molecules and are released from the cell by budding. The maturation of the particle then occurs outside from the cell.
**Figure 3****: Mechanism of reverse transcription in retroviruses.**
   During the reverse transcription, first strand synthesis is similar for all retroviruses. (1) The first strand (-) synthesis is initiated by the binding of a specific tRNA on the Primer Binding Site, a sequence of 18 nucleotides, located in 3' from the 5' LTR. The U5 and R regions of the 5' LTR are synthesised by elongation of the tRNA thanks to the DNA polymerase activity of the reverse transcriptase while the corresponding RNA matrix fragments, with the exception of the PBS region, are degraded thanks to the RNAseH activity of the reverse transcriptase. (2) This first synthetized fragment, called the minus-strand strong stop DNA, jumps on the 3' LTR and the R regions of the DNA and RNA fragments hybridize. (3) The elongation of the DNA fragment is pursued on the U3 region and toward the 5' end of the RNA molecule until the PBS region. The RNAseH activity degrades the RNA matrix after the DNA synthesis. If the RNA matrix is not fully degraded, the second strand synthesis is prevented. A region is however protected from this degradation: the polypurine tract. The RNA fragment spared will be further use a primer for the second strand synthesis. The second strand synthesis (+) occurs in a different manner for oncoretroviruses and other retroviruses. **In oncoretroviruses, (4-7)** the synthesis of the second strand is initiated on the polypurine tract (PPT) sequence located in 5' of the 3' LTR. The elongation progresses toward the U5 region at the 3' end of the genome, using the (-) strand DNA as a template. Meanwhile, minus-strand synthesis continues through the genome, using the (+) strand RNA as a template and removing the RNA template in its wake via RNAseH activity. PPT initiated (+) strand stops after copying the annealed portion of the RNA to generate the (+) strand DNA form of the PBS, forming the (+) strand strong stop product. The tRNA is then removed by the RNAseH activity of the reverse transcriptase. This may facilitate annealing to the PBS complement on the (-) strand DNA, providing the complementarity for the second jump (intramolecular). DNA synthesis then continues. Strand displacement synthesis by reverse transcriptase to the PBS and PPT ends, and/or repair and ligation of a circular intermediate, produces a linear duplex with long terminal repeats (U3 - R - U5) at both ends.
   **In lentiviruses and foamy viruses, (4-7)** the mechanism is quite similar except that initiation occurs on 2 sites: the PPT located in 3' of the first strand and an additional PPT located in the centre of the first strand: the central polypurine tract (cPPT). A central termination sequence is located in 3' of the cPPT and stops the (+) strand synthesis initiated from the 3' PPT. As a consequence, the reverse transcription induces a transient 3 strand-structure overlapping the cPPT and the CTS, named "triplex" **(6).** Although this sequence is dispensable in lentiviral vectors, its presence significantly increases the transduction efficiency of the particles.
**Figure 4****: Retroviral reverse transcriptase structure.**
   Retroviral reverse transcriptases are organized in two domains, each corresponding to the two enzymatic activities of the protein: the DNA polymerase domain (POL) and the RNAseH domain. As an example, detailed organization is shown for HIV-1 and MLV reverse transcriptase. Positions of the two functional domains, as well as sub-domains are figured for HIV-1 p66 and p51 (HIV-1 p51 being generated by protease cleavage of HIV-1 p66) and MLV p75. (F: Finger domain ; P: Palm domain ; T: Thumb domain ; C: Connection domain.)
**Figure 5****: Crystal structure of human immunodeficiency virus type 1 reverse transcriptase heterodimer and MLV p75 reverse transcriptase.**
   **TOP:** This schema figures the p66 (560 residues) and the p51 (440 residues) subunits of HIV-1 reverse transcriptase (top) and the different structural domains: the polymerase domain, which is divided in 4 subdomains: the finger, the palm, the thumb and the connection ; and the RNAseH domain C: C terminus, N: N terminus.
   **BOTTOM:** This schema figures the different structural domains of the MLV reverse transcriptase: the polymerase domain, which is divided in 4 subdomains: the finger, the palm, the thumb and the connection ; and the RNAseH domain (from Retroviral reverse transcriptases. Herschhorn A, Hizi A. Cell Mol Life Sci. 2010 Aug;67(16):2717-47. Epub 2010 Apr 1).

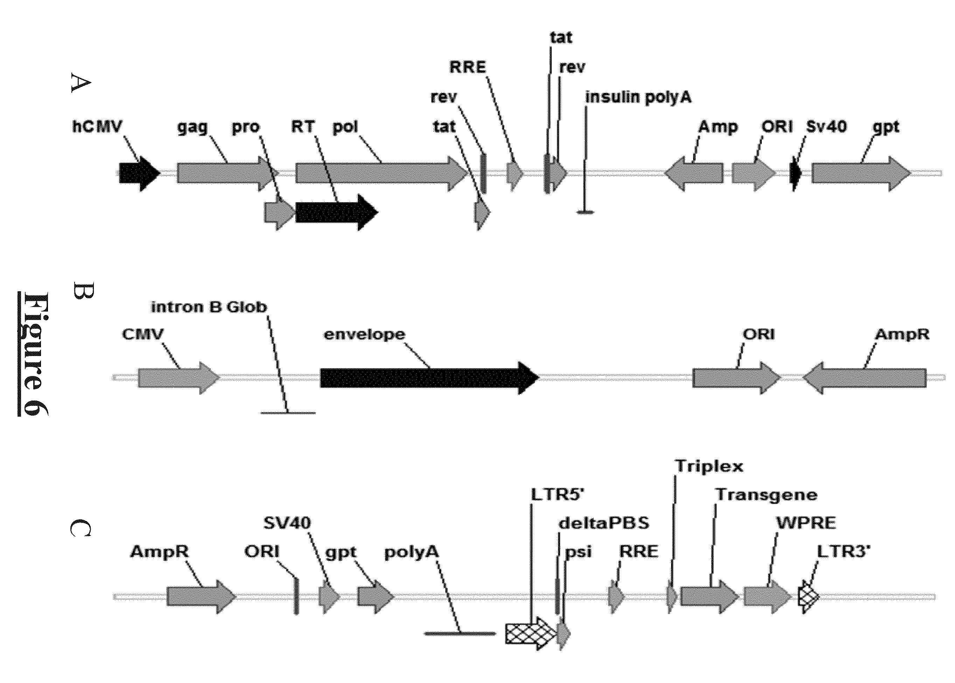

**Figure 6****: Schematic representation of a 3 plasmid-system used for production of HIV-1 lentiviral vectors by transient transfection.**
   **A: the transcomplementation capsid plasmid** contains the backbone bacterial sequences, including the Ampicilin resistance gene (Amp), the bacterial promoter (SV40), the glutamic-pyruvate transaminase coding sequence (gpt), the bacterial origin of replication (ORI), and eukaryote elements: sequences encoding for the structural and enzymatic proteins required for the formation of a particle under the control of a promoter, for example the CMV promoter (hCMV). It particularly contains the gag gene (encoding for the capsid (CA), the matrix (MA), the nucleocapsid (NC)) and the pol gene (encoding for the integrase (IN), the reverse transcriptase (RT) and the protease (PR)), it further comprises the *tat* and *rev* genes. The plasmid also includes a polyadenylation signal (insulin polyA) ;
   **B: the transcomplementation envelope plasmid** contains the backbone bacterial sequences, including the Ampicilin resistance gene AmpR, the bacterial origin of replication (ORI), and sequences active in eukaryote cells: the sequence encoding for a particular envelope glycoprotein envelope under the control of a promoter, for example a CMV promoter, and possibly regulatory elements, such as the beta globin intron (Intron B Glob) ;
   **C: the vector plasmid** contains the backbone bacterial sequences, including the Ampicilin resistance gene (AmpR), the bacterial promoter (SV40), the glutamic-pyruvate transaminase coding sequence (gpt), a polyadenylation signal (polyA), and the retroviral vector elements, including the 5' and the 3' Long Terminal Repeats (LTR5', and Self Inactivating, LTR3' SIN), the Rev Responsive Element (RRE), the triplex or cPPT-CTS (central polypurine tract - central termination sequence), the expression cassette, possibly including a promoter, for example a CMV promoter, and at least one transgene coding sequence and regulatory elements such as WPRE, and an encapsidation signal (Psi), the vector plasmid is further deprived of a functional primer binding site, thus preventing the binding of the tRNA Lys3 and subsequent initiation of reverse transcription.

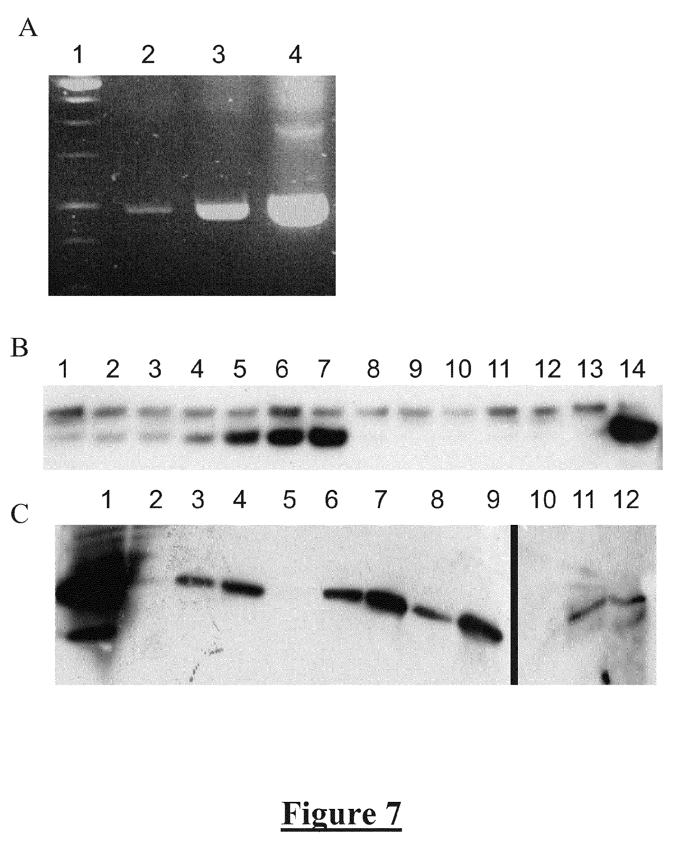

**Figure 7****: Encapsidation of RNA viral genome and transgene expression.**
   **A: Detection of encapsidated RNA genome by RT-PCR.**
      Vector particle preparations are assayed for encapsidation of RNA genome. RNA is extracted and reverse transcribed, generated cDNA are submitted to PCR amplification using primers located in 5' and 3' of the GFP sequence. The PCR product is 979 base pairs. (1: DNA ladder, 2: vector production capsid(2) : vector(2) : envelope(1) ; 3: vector production capsid(2) : vector(4) : envelope(1), 4: PCR positive control).
   **B and C: Detection of GFP and PhiC31 expression by Western blot.**
      Vector particle preparations are used to transduce 293T cells (**B:** 400 000 cells plated 24 hours before transduction with 750 ng p24; **C 2 to 7 and 10 and 11:** 400 000 cells plated 24 hours before transduction with below mentioned doses; **C 8 and 9:** 800 000 cells incubated with below mentioned doses). Cells are collected at different time points after transduction and proteins are extracted and submitted to western blot analysis for the transgene expression **(B and C 1 to 9:** primary antibody: Abcam ab290 ; **C 10 to 12:** primary antibody: PhiC31 specific antibody (gift from M-C Serre) ; revelation ECL+ GE Healthcare) (**B:** DPBS GFP vector, **1**: 4 hours, **2**: 8 hours, **3**: 24 hours, **4**: 32 hours, **5**: 48 hours, **6**: 56 hours, **7**: 5 days, **8**: 9 days, **9**: 12 days, **10**: 16 days, **11**: 19 days, **12:** 23 days, **13**: 26 days, **14**: GFP control ; **C 1 to 9:** DPBS GFP integrase D64V vector, **1**: GFP control, **2-5:** 100ng p24; **3-6:** 500 ng p24, **4- 7-9:** 1000 ng p24, **8:** 200 ng p24, **2-3-4:** 4 hours, **5-6-7:** 8 hours, **8-9:** 18 hours ; **C 10 to 12:** DPBS PHIC31 integrase D64V vector, 1000 ng p24, **10**: 24 hours, **11**: 48 hours, **12**: PHIC31 control).

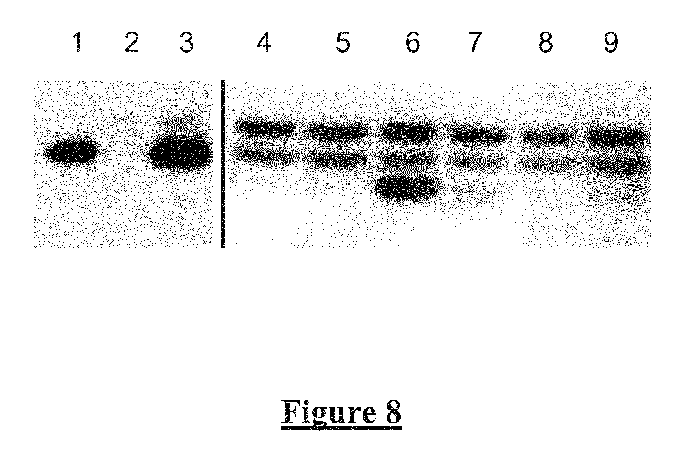

**Figure 8****: Genuine transduction mechanism mediated by reverse transcription deficient vector.**
   293T cells are transduced with different HIV-1 vectors in normal medium or in presence of 10 pM azido thymidine (AZT), an inhibitor of retroviral reverse transcriptase or 0.1 mM chloroquine, an inhibitor of lentiviral uncoating. Cells are grown for 24 hours after transduction. Cells are then collected and proteins are extracted and submitted to western blot analysis to detect GFP expression (primary antibody: Abcam ab290, revelation ECL+ GE Healthcare). **(1**: GFP control, **2-3:** reverse transcription and integration proficient vector, **4-5-6:** reverse transcription proficient integrase deficient vector, **7-8-9:** reverse transcription integrase deficient vector, **2-4:** AZT, **3-6-9:** control medium, **5-8:** chloroquine).

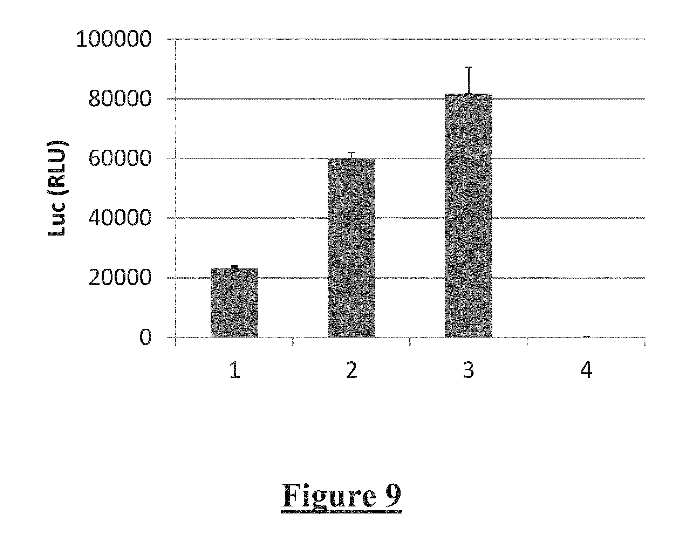

**Figure 9****: Detection of Luciferase activity mediated by reverse transcription deficient HIV-1 vectors.**
   HeLa cells are transduced with increasing doses of vectors deleted for the primer binding site and containing a Luciferase coding sequence. Cells are grown for 24 hours after transduction and then collected and processed with Bright Glow Luciferase kit (Promega), according to manufacturer's protocol (10 µL lysate per well). Luciferase activity is measured by a Luminometer. Results are expressed as Relative Light Unit per 10 pL of lysate and figured as mean of 3 samples per condition +/- standard deviation (SD). (1: 1 µL, 2: 3 µL, 3: 5 µL, 4: control non-transduced cells).

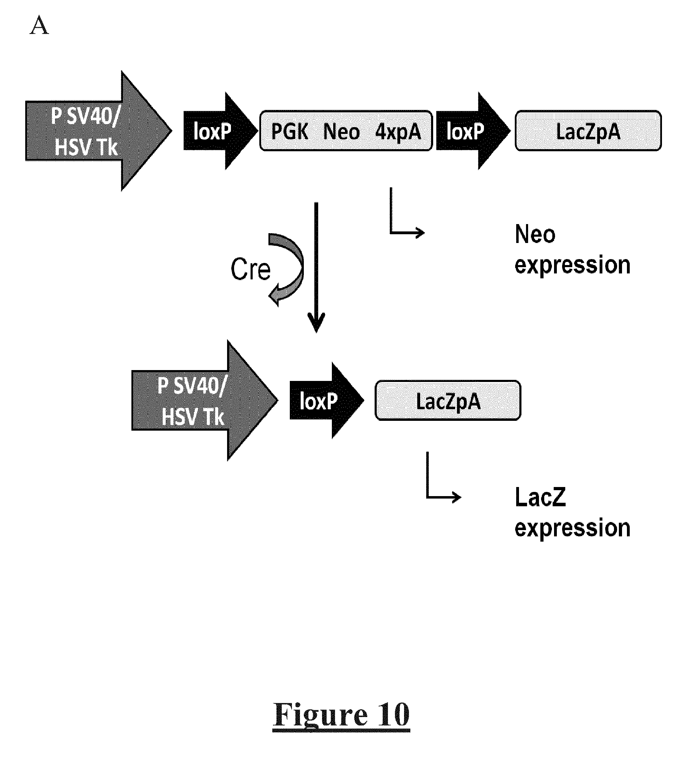

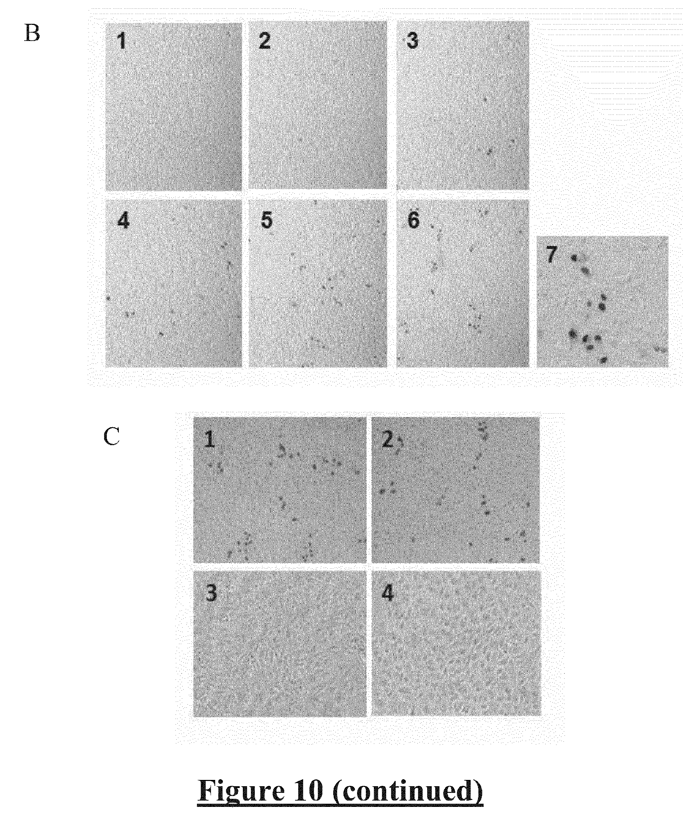

**Figure 10****: The CV-1 5B reporter cell line and LacZ expression in CV1-5B cells after transient expression of Cre recombinase mediated by reverse transcription deficient HIV-1 vectors.**
   **A:** The CV1-5B reporter cell line created by Kellendonk et al.( Kellendonk C., Tronche F., Monaghan A.P., Angrand P.O., Stewart F., Schutz G. Nucleic Acids Res. 1996;24:1404-1411) harbours a stable integration of the pHSVtk/loxNeolox/NLS-lacZ reporter construct. Expression of the *NLS-lacZ* gene is only detected in cells that have undergone Cre-mediated deletion of the IoxP flanked neomycin phosphotransferase gene.
   **B-C:** CV1-5B cells are transduced with increasing doses of reverse transcription deficient vectors encoding for the Cre recombinase. Cells are grown for 72 hours after transduction and then fixed and processed for LacZ activity by a X Gal staining. Positive cells are those having undergone a recombination mediated by Cre. (B1: control non transduced cells, B2: 1 µL, B3: 3 µL, B4: 5 µL, B5: 7 µL, B6: 9 µL, B7: higher magnification of B6; C1: VSV + DNase, C2: VSV wo DNase, C3: wo VSV + DNAse, C4: wo VSV wo DNase).

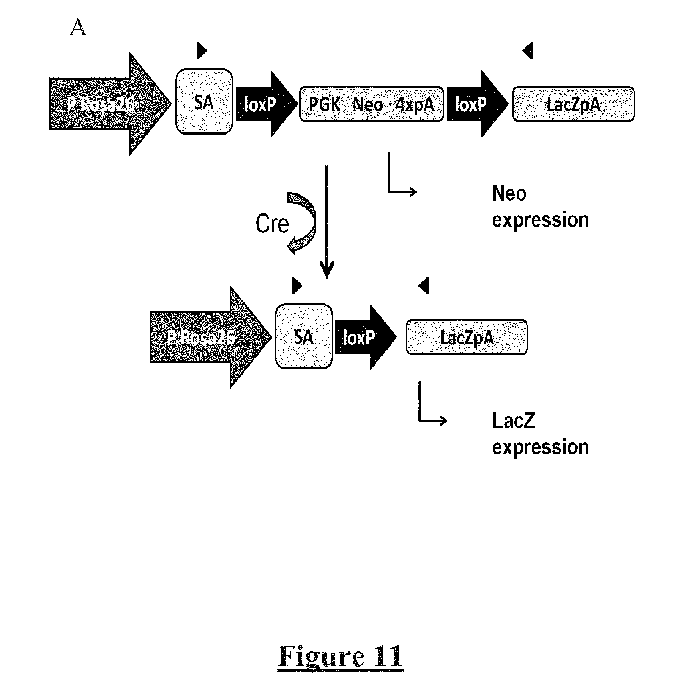

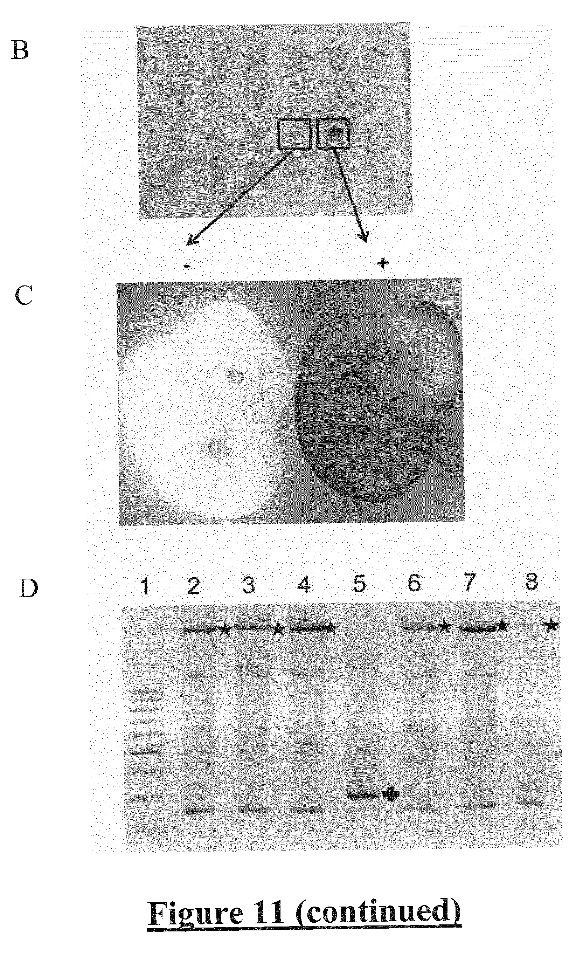

**Figure 11****: The ROSA26 reporter mouse strain and Cre mediated recombination in the ROSA26 reporter mouse strain.**
   **A: The Rosa26 reporter mouse strain**
      The Rosa26 reporter mouse strain created by Soriano et al 1999 (Soriano, P. (1999). Generalized lacZ expression with the ROSA26 Cre reporter strain. Nat Genet 21, 70-71.) harbours a stable integration of the pRosa26/loxNeolox/NLS-lacZ reporter construct. Expression of the *lacZ* gene is only detected in cells that have undergone Cre-mediated deletion of the loxP flanked neomycin phosphotransferase gene. Black arrowheads indicate the position of the primers that are used for the genotyping assay.
   **B to D: Transgenesis in Rosa26 mice and Transient expression of Cre mediated by reverse transcription deficient vector particles.**
      Rosa26 reporter embryos are submitted to lentiviral transgenesis: eggs are treated with reverse transcription deficient HIV-1 vectors carrying a Cre recombinase encoding sequence. Embryos are reimplanted in pseudopregnant females and allowed to develop for 13 days. Embryos are then collected and assayed for LacZ activity (B and C) and genotyping (D). The size of the PCR product for recombined DNA (5, marked with a cross) is expected to be lower compared to non-recombined DNA (2, 3, 4, 6, 7, 8, marked with a star). (1: DNA ladder).

### EXPERIMENTAL PART

### Example 1: Production of a reverse transcription deficient retroviral vector derived from HIV-1 by transient transfection.

According to the present invention, HIV-1 derived vectors deficient for reverse transcription can be produced, for instance by transient transfection of 3 particular plasmids in producer cells (see **Figure 6****).** These three plasmids are (i) the transcomplementation encapsidation plasmid, (ii) the transcomplementation envelope plasmid and (iii) the vector plasmid, which accordingly to the present description contains a modified Primer Binding Site preventing the binding of the tRNA Lys3 and the subsequent initiation of reverse transcription. In the present embodiment, said modification consists in a deletion of the 18 nucleotides of the PBS, ie nucleotides 5577 located 5594 on SEQ ID N0:1, thus generating the plasmid SEQ ID NO:2.

Briefly, the 3 plasmids are transfected by calcium phosphate precipitation method into 293T cells. The transfected cells then produce HIV-1 vector particles including a RNA molecule. These particles can be recovered from the cell culture medium and subsequently used for different purposes, in vitro or in vivo.

In a particular embodiment of the invention, plasmid SEQ ID NO:3, plasmid SEQ ID NO:4 and plasmid SEQ ID NO:2 can be used. The produced vector particles are then characterized in that (1) they are enveloped with VSV glycoprotein, (2) they contain a RNA molecule allowing the expression of GFP and deprived of recognition site for the tRNA Lys3.

In another particular embodiment of the invention, plasmid SEQ ID NO:3, plasmid SEQ ID NO:5 and plasmid SEQ ID NO:6 can be used. The produced vector particles are then characterized in that (1) they are enveloped with Mokola glycoprotein, (2) they contain a RNA molecule allowing the expression of Luciferase and deprived of recognition site for the tRNALys3.

Different combinations of any envelope/capsid/transgene can be used.

### Example 2: Production of a reverse transcription deficient retroviral vectors derived from HIV-1 by using stable producer cell lines.

According to the present invention, HIV-1 derived vectors deficient for reverse transcription can be produced, for instance by using stable producer cell lines. Such cell lines are generated by introducing in a single cell the required transcomplementation elements: envelope and capsid, along with a HIV-1 vector genome, expression of which leading to the formation of ribonucleic vector genome that can be encapsidated but containing a modified PBS preventing the initiation of reverse transcription.

These elements (envelop, capsid and ribonucleic vector genome) are stably introduced into a cell as one or more expression cassettes under the control of promoter (s) which are constitutively or inducible promoters. When the elements are expressed altogether in the cell (constitutively or when the inducible promoters are activated), they form vector particles, which are released by the cells in the surrounding culture medium, similarly as after the transient transfection method described in the previous example. These produced HIV-1 vector particles include a RNA molecule. These particles are collected from the cell culture medium and subsequently used for different purposes, in vitro or in vivo.

In a particular embodiment of the invention, the stable producing cell lines contain the following sequences SEQ ID NO:7, SEQ ID NO:8 and SEQ ID NO:9, and will allow the production of vector particles characterized in that (1) they are enveloped with Mokola glycoprotein, (2) they contain a RNA molecule allowing the expression of Neomycine phosphotransferase. This cell line is generated by transient transfection of 293T cells with 3 plasmids containing the above-mentioned sequences. These cells are then treated with G418, Puromycine and Hygromycine, which allow selecting the cells that have stably incorporated the 3 plasmids: Neo expression cassette brought by the vector plasmid, the Puromycine resistance gene brought by the envelope transcomplementation plasmid and the Hygromycine resistance gene brought by the capsid transcomplementation plasmid. Selected cells are seeded at a very low density to generate homogenous clonal populations. Clonal population are then screened to verify cells that express efficiently all the required elements and allow the highest vector particle titre production.

In another particular embodiment of the invention, the stable producing cell lines contain the following sequences SEQ ID NO:10, SEQ ID NO:9 and SEQ ID N0:11. The produced vector particles are then characterized in that (1) they are enveloped with VSV glycoprotein, (2) they contain a RNA molecule allowing the expression of Neomycine phosphotransferase, and deprived of a functional PBS. To avoid the toxicity of the constitutive expression of VSV glycoprotein for the cells, the VSV gene is under control of a doxycycline inducible promoter activated by a transactivator RtTa2M2 in the presence of doxycycline. The VSV glycoprotein is then expressed when the cells are treated by sufficient dose of doxycycline. In this particular embodiment, the treatment of cells by Hygromycine, G418 8 and Puromycine allows to select for cells respectively containing the inducible envelope transcomplementation cassette along with the constitutive capsid transcomplementation cassette, the vector cassette and the transactivator cassette. The selection of the most efficient clones is then performed in a doxycycline-containing medium.

In another particular embodiment of the invention, the stable cell line allowing the production of HIV-1 vector particles pseudotyped with VSV and expressing the Neo gene and deficient for reverse transcription, does not contain the VSV expressing cassette. Cells constitutively express the genome and the encapsidation elements, leading to incomplete and non-functional particles. The production of complete and functional reverse transcription deficient particles is induced when the VSV is expressed in the cells. The VSV expressing cassette is brought by transient transfection by calcium phosphate. 48 hours after the transfection, HIV-1 vector particles are released from the cells in the cell culture medium and are collected for further use, *in vitro* and/or *in vivo.*

Different combinations of any envelope/capsid/transgene can be used.

### Example 3: GFP expression mediated by HIV-1 reverse transcription deficient vector particles in vitro.

HIV-1 derived vector particles are produced by transient transfection of 293T cells with the envelope transcomplementation plasmid encoding VSV (SEQ ID NO:4), the capsid transcomplementation (SEQ ID NO:3) and the vector plasmid encoding the GFP (SEQ ID NO:2).

In this example, different conditions were tested for the production: the ratio capsid : vector : envelope was 2 : 2 : 1 or 2:4:1.

Vector particles preparations are first assayed for encapsidation of RNA genome. RNA is extracted and submitted to in vitro reverse transcription using SuperScriptll (Invitrogen) and primers located in 5' and 3' of the GFP expression cassette. Results are presented **Figure 7A****.**

The vector particle preparations are used to treat 293T cells (750 ng p24 per 400,000 plated cells). Cells are harvested at different time point after transduction to evaluate the kinetic of transgene expression, analysed by Western blot using GFP specific antibody. Results are shown on **Figure 7****B**.

In another experience, cells are transduced with a single dose of vector (10uL for 80,000 cells) and GFP expression is measured by FACS 36 hours after transduction. The percentage of GFP positive cells is 7.5.

In another embodiment of the invention, HIV-1 derived vector particles deficient for the reverse transcription are produced by transient transfection of 293T cells with the envelope transcomplementation plasmid encoding VSV (SEQ ID NO:4), the capsid transcomplementation encoding for an integrase which has been mutated in the catalytic site (D64V substitution) and is thus impaired for catalysing the integration reaction (SEQ ID N0:12) and the vector plasmid encoding the GFP deleted for the primer binding site (SEQ ID NO:2).

Increasing doses of the vector particle preparations are used to treat 293T cells. Cells are harvested at different time point after transduction to evaluate the kinetic of transgene expression, analysed by Western blot using GFP specific antibody. Results are shown on **Figure 7C****.**

These results show that the reverse transcription deficient particles comprising the sequence encoding the GFP, genuinely encapsidate the recombinant lentiviral ribonucleic vector genome and allow the transient expression of GFP after contact with target cells. Said GFP expression is dose dependent and starts 4 hours after contact with the cell, the maximum level is reached between 56 hours and 5 days after transduction and is no longer detected from 9 days after transduction.

### Example 4: Transient expression of a GFP mediated by HIV-1 reverse transcription deficient vector particles in vitro results from genuine transduction mechanism.

In order to demonstrate that the transgene expression observed after transduction with reverse transcription deficient vectors results from of genuine transduction mechanism (expression from the recombinant RNA vector genome brought by the vector particle) and does not result from contamination of the vector preparation by plasmid used during the production, experience described in **Example 3,** **Figure 7B** is reproduced with or without treatment of the cells with chloroquine, an inhibitor of endosome acidification (VSV pseudotyped retroviral vector particle uncoating pathway).

Results presented on **Figure 8** show that GFP expression is inhibited in cells treated with Chloroquine.

In addition, to verify that the GFP expression is the result of translation of encapsidated RNA genomes and not of transcription of reverse transcribed DNA genomes, cells transduced with GFP expressing DPBS vectors are treated with Azido-thymidine (AZT), an inhibitor of retroviral reverse transcriptases. Cells are collected 24 hours after transduction and protein extracts are submitted to Western blot analysis. As shown on **Figure 8****,** GFP expression mediated by reverse transcription deficient HIV-1 vectors is independent of reverse transcription.

These results show that the reverse transcription deficient particles comprising the sequence encoding the GFP allow the expression of GFP after contact with target cells and said expression is dependent on uncoating of the particle but not on reverse transcriptase activity. Measured GFP expression is thus genuinely resulting from (i) a transduction mechanism and not from pseudo transduction or contamination of the vector production and (ii) direct translation of the recombinant RNA vector genome and it does not involve the reverse transcription of the recombinant RNA vector genome into a DNA proviral genome.

### Example 5: PhiC31 expression mediated by HIV-1 reverse transcription deficient vector particles in vitro.

HIV-1 derived vector particles are produced by transient transfection of 293T cells with the envelope transcomplementation plasmid encoding VSV (SEQ ID NO:4), the capsid transcomplementation (SEQ ID NO:3) and the vector plasmid encoding the PhiC31 phage recombinase (SEQ ID N0:13).

The vector particle preparations are used to treat 293T cells. Cells are harvested at different time point after transduction to evaluate the kinetic of transgene expression, analysed by Western blot using PhiC31 specific antibody. Results are shown on **Figure 7C****.**

These results show that the reverse transcription deficient particles comprising the sequence encoding the PhiC31 recombinase allow the expression of PhiC31 recombinase 48 hours after contact with target cells.

### Example 6: Luciferase expression mediated by HIV-1 reverse transcription deficient vector particles in vitro.

HIV-1 derived vector particles are produced by transient transfection of 293T cells with the envelope transcomplementation plasmid encoding VSV (SEQ ID NO:4), the capsid transcomplementation (SEQ ID NO:3) and the vector plasmid encoding the Luciferase (SEQ ID NO:6).

The vector particle preparations are used to treat 293T cells (0, 1, 3 or 5 µL per 35 000 cells). Cells are harvested 48 hours after transduction and proteins are extracted. Luciferase activity in the protein extracts is measured using Bright Glow Luciferase kit (Promega). Results are shown on Figure 9.

These results show that the reverse transcription deficient particles comprising the sequence encoding the Luciferase allow the expression of Luciferase 48 hours after contact with target cells and said expression is dose dependent.

### Example 7: Transient expression of a DNA modifying enzyme mediated by HIV-1 reverse transcription deficient vector particles in vitro.

In order to demonstrate the potential of reverse transcriptase deficient HIV-1 vectors to transiently express a DNA modifying enzyme such as recombinase in a way that it can induce recombination in cell, Cre expressing vectors are produced by transient transfection of 293T cells with the envelope transcomplementation plasmid encoding VSV (SEQ ID NO:4), the capsid transcomplementation (SEQ ID NO:3) and the vector plasmid encoding the Cre recombinase (SEQ ID NO: 14). The produced vectors are used to transduce CV-1 5B reporter cells.

The CV-1 5B reporter cell line created by Kellendonk et al.(Kellendonk C., Tronche F., Monaghan A.P., Angrand P.O., Stewart F., Schutz G. Nucleic Acids Res. 1996;24:1404-1411) harbours a stable integration of the pHSVtk/loxNeolox/NLS-lacZ reporter construct. Expression of the NLS-/acZgene is only detected in cells that have undergone Cre-mediated deletion of the loxP flanked neomycin phosphotransferase gene, as shown **Figure 10A****.** CV-1 5B cells are transduced with increasing doses of reverse transcription deficient HIV-1 vectors (0 µL, 1 µL, 3 µL, 5 µL, 7 µL, 9 µL per 100,000 cells) and grown for 72 hours in 6 well-plates. Cells are then fixed and LacZ expression is revealed by X-Gal assay. Results are shown **Figure 10****B.**

These results show that the reverse transcription deficient particles comprising the sequence coding for a DNA modifying enzyme, for instance the Cre recombinase, allow the expression of said DNA modifying enzyme. For instance, the Cre recombinase was expressed in a way that it can induce the recombination of the LacZ locus in CV-1 5B target cells and further induce LacZ expression.

### Example 8: Transient expression of a DNA modifying enzyme mediated by HIV-1 reverse transcription deficient vector particles in vitro results from genuine transduction mechanism.

In order to demonstrate that the transgene expression observed after transduction with reverse transcription deficient vectors results from of genuine transduction mechanism (expression from the RNA vector genome brought by the vector particle) and does not result from contamination of the vector preparation by plasmid used during the production, experience described in **Example 7** is reproduced with or without initial treatment of the vector preparation with 1 µg/mL DNase and with or without including the VSV plasmid during the production.

Results presented on **Figure 10C** show that Cre recombination is not dependent on DNAse treatment but dependent on the presence of VSV glycoprotein on the particle envelope.

These results show that the reverse transcription deficient particles comprising the sequence coding for the Cre recombinase allow the expression of Cre recombinase 72 hours after contact with target cells and said expression does not result from pseudo-transduction or contamination with DNA plasmid but results from a genuine transduction mechanism since (i) it is dependent on fusion of the particle with the cell membrane through interaction with VSV glycoprotein and (ii) it is not altered by the treatment of the vector preparation with DNase.

### Example 9: Transient expression of a DNA modifying enzyme mediated by HIV-1 reverse transcription deficient vector particles for genetic engineering of an animal.

Cre expressing vectors as described in **Example 7** are also used in genetic engineering application. In this assay, Cre ROSA 26 (R26R) mice are used. This mouse strain contains a construction similar to the one harboured by the CV1-5B cells and presented on **Figure 11A****.** The LacZ expression requires a recombination induced by the Cre recombinase.

Eggs from R2R6 mice are fertilized and collected. They are microinjected with Cre expressing reverse transcription deficient HIV-1 vectors and transferred into pseudo-pregnant females. Embryos are allowed to develop for 13 days and then collected and LacZ expression is assayed. As showed on the **Figure 11B and 11C****,** one embryo out of 24 that developed in the females after transgenesis, undergone a recombination mediated by Cre (positive for LacZ assay). This result is confirmed by PCR analysis performed on the DNA on the embryos: using primers located both upstream and downstream of the LoxP sequences, as shown on **Figure 11A****,** the recombination is evidenced on the genomic DNA level (see **Figure 11D**)**.**

These results show that the reverse transcription deficient particles comprising the sequence coding for the Cre recombinase allow the expression of Cre recombinase in a way that it can induce the recombination of the LacZ locus during a transgenesis procedure in ROSA26 mice, thus generating genetically modified animals.

## Claims

1. A recombinant lentiviral vector which cannot by its own achieve complete reverse transcription wherein the vector comprises a recombinant ribonucleic vector genome comprising a 5' LTR retroviral sequence and a 3' LTR retroviral sequence flanking: a functional retroviral psi encapsidation sequence and at least one transgene, and wherein the recombinant ribonucleic vector genome (i) does not comprise a primer binding site (PBS) or (ii) comprises a PBS comprising a mutation or a misplaced PBS, thereby preventing the reverse transcription of said recombinant ribonucleic vector genome.

2. The vector according to claim 1, wherein said lentiviral vector is derived from HIV-1, HIV-2, SIV, FIV, EIAV, BIV, VISNA, CAEV and any combination thereof.

3. The vector according to anyone of claims 1 or 2, wherein the mutation in the primer binding site (PBS) preventing reverse transcription of the recombinant ribonucleic vector genome is selected from a deletion, an addition and a substitution of at least one nucleotide, preferably at least 3 nucleotides.

4. The vector according to claim 3, wherein the vector is a HIV-1 derived vector and the mutation is a deletion of the HIV-1 PBS sequence corresponding to SEQ ID NO:15 (DNA) and SEQ ID NO:17 (RNA).

5. The vector according to anyone of claims 1 to 4, wherein the recombinant lentiviral vector genome further comprises a posttranscriptional regulatory sequence chosen from INPRE, APP 5'UTR, TAU 3'UTR, and a miRNA target sequence.

6. The vector according to anyone of the preceding claims, wherein the transgene is selected from a catalytic nucleic acid, an aptamer, a miRNA, a decoy RNA, a nucleic acid encoding a biologically active peptide, for example an enzyme, a transcription factor, a growth factor, a trophic factor, an hormone, a cytokine, an antibody, a receptor, a differentiation factor, a colony stimulation factor, a suicide protein, a cell-cycle modifying protein, an anti-proliferative protein, a nuclease, a recombinase, a transposase, a neurotransmitter or a precursor thereof.

7. A method for preparing a lentiviral vector which cannot by its own achieve complete reverse transcription according to anyone of claims 1 to 6, wherein said method comprises expressing within a cell:
a. a vector cassette encoding for a lentiviral recombinant ribonucleic vector genome comprising a 5' LTR retroviral sequence and a 3' LTR retroviral sequence flanking: a functional retroviral psi encapsidation sequence and at least one transgene, and wherein the recombinant ribonucleic vector genome (i) does not comprise a primer binding site (PBS) or (ii) comprises a PBS comprising a mutation or a misplaced PBS, thereby preventing the reverse transcription of said recombinant ribonucleic vector genome, and
b. a transcomplementation envelope cassette encoding for an envelope glycoprotein, and
c. a transcomplementation capsid cassette encoding for sequences derived from a lentiviral genome comprising retroviral gag and pol sequences, said transcomplementation capsid sequences lacking any functional psi encapsidation signal, and said transcomplementation capsid sequences being optionally split into several cassettes, and
recovery of the retroviral vector produced.

8. A lentiviral vector obtainable with a method according to claim 7.

9. A nucleic acid sequence comprising a vector cassette encoding for a lentiviral recombinant ribonucleic vector genome comprising a 5' LTR retroviral sequence and a 3' LTR retroviral sequence flanking: a functional retroviral psi encapsidation sequence and at least one transgene, and wherein the recombinant ribonucleic vector genome (i) does not comprise a primer binding site (PBS) or (ii) comprises a PBS comprising a mutation or a misplaced PBS, thereby preventing the reverse transcription of said recombinant ribonucleic vector genome, and optionally:
- a nucleic acid sequence comprising a transcomplementation envelope cassette encoding for an envelope glycoprotein, and/or
- a nucleic acid sequence comprising a transcomplementation capsid cassette encoding for sequences derived from a lentiviral genome comprising retroviral gag and pol sequences, said transcomplementation capsid sequences lacking any functional psi encapsidation signal, and said transcomplementation capsid sequences being optionally split into several cassettes

10. The nucleic acid sequence according to claim 9, wherein the sequence is selected from a linear nucleic acid sequence, a plasmid, an artificial chromosome, a viral vector genome, and a transposon.

11. A cell or cell line comprising a nucleic acid sequence according to claim 9 or 10.

12. A composition comprising a vector according to anyone of claims 1 to 6 or 8, a nucleic acid sequence according to claim 9 or 10, and/or a cell or a cell line according to claim 11.

13. A composition according to claim 12, wherein the composition is a pharmaceutical composition comprising a vector according to anyone of claims 1 to 6 or 8, a nucleic acid sequence according to claim 9 or 10, and/or a cell or a cell line according to claim 11, and a pharmaceutically acceptable excipient.

14. A kit comprising a vector according to anyone of claims 1 to 6 or 8, a nucleic acid sequence according to claim 9 or 10, a cell or a cell line according to claim 11 and/or a composition according to claim 12 or 13, and preferably written instructions for using the kit.

15. Use of a vector according to anyone of claims 1 to 6 or 8, a nucleic acid sequence according to claim 9 or 10, a cell or a cell line according to claim 11, a composition according to claim 12 or 13 and/or of a kit according to claim 14, for transiently expressing at least one transgene *in vitro,* ex *vivo* or *in vivo.*


SEQUENCE LISTING

<110>  New Vectys

<120>  TRANSIENT EXPRESSION LENTIVIRAL VECTORS, PREPARATION AND USES
       THEREOF

<130>  B1238

<160>  18

<170>  PatentIn version 3.3

<210>  1
<211>  10374
<212>  DNA
<213>  Artificial sequence

<220>
<223>  SEQ ID NO: 1

<400>  1
   ccagatcctc tacgccggac gcatcgtggc cggcatcacc ggcgccacag gtgcggttgc      60
   tggcgcctat atcgccgaca tcaccgatgg ggaagatcgg gctcgccact tcgggctcat     120
   gagcgcttgt ttcggcgtgg gtatggtggc aggccccgtg gccgggggac tgttgggcgc     180
   catctccttg catgcaccat tccttgcggc ggcggtgctc aacggcctca acctactact     240
   gggctgcttc ctaatgcagg agtcgcataa gggagagcgt cgaatggtgc actctcagta     300
   caatctgctc tgatgccgca tagttaagcc agccccgaca cccgccaaca cccgctgacg     360
   cgccctgacg ggcttgtctg ctcccggcat ccgcttacag acaagctgtg accgtctccg     420
   ggagctgcat gtgtcagagg ttttcaccgt catcaccgaa acgcgcgaga cgaaagggcc     480
   tcgtgatacg cctattttta taggttaatg tcatgataat aatggtttct tagacgtcag     540
   gtggcacttt tcggggaaat gtgcgcggaa cccctatttg tttatttttc taaatacatt     600
   caaatatgta tccgctcatg agacaataac cctgataaat gcttcaataa tattgaaaaa     660
   ggaagagtat gagtattcaa catttccgtg tcgcccttat tccctttttt gcggcatttt     720
   gccttcctgt ttttgctcac ccagaaacgc tggtgaaagt aaaagatgct gaagatcagt     780
   tgggtgcacg agtgggttac atcgaactgg atctcaacag cggtaagatc cttgagagtt     840
   ttcgccccga agaacgtttt ccaatgatga gcacttttaa agttctgcta tgtggcgcgg     900
   tattatcccg tattgacgcc gggcaagagc aactcggtcg ccgcatacac tattctcaga     960
   atgacttggt tgagtactca ccagtcacag aaaagcatct tacggatggc atgacagtaa    1020
   gagaattatg cagtgctgcc ataaccatga gtgataacac tgcggccaac ttacttctga    1080
   caacgatcgg aggaccgaag gagctaaccg cttttttgca caacatgggg gatcatgtaa    1140
   ctcgccttga tcgttgggaa ccggagctga atgaagccat accaaacgac gagcgtgaca    1200
   ccacgatgcc tgtagcaatg gcaacaacgt tgcgcaaact attaactggc gaactactta    1260
   ctctagcttc ccggcaacaa ttaatagact ggatggaggc ggataaagtt gcaggaccac    1320
   ttctgcgctc ggcccttccg gctggctggt ttattgctga taaatctgga gccggtgagc 1380
   gtgggtctcg cggtatcatt gcagcactgg ggccagatgg taagccctcc cgtatcgtag 1440
   ttatctacac gacggggagt caggcaacta tggatgaacg aaatagacag atcgctgaga 1500
   taggtgcctc actgattaag cattggtaac tgtcagacca agtttactca tatatacttt 1560
   agattgattt aaaacttcat ttttaattta aaaggatcta ggtgaagatc ctttttgata 1620
   atctcatgac caaaatccct taacgtgagt tttcgttcca ctgagcgtca gaccccgtag 1680
   aaaagatcaa aggatcttct tgagatcctt tttttctgcg cgtaatctgc tgcttgcaaa 1740
   caaaaaaacc accgctacca gcggtggttt gtttgccgga tcaagagcta ccaactcttt 1800
   ttccgaaggt aactggcttc agcagagcgc agataccaaa tactgtcctt ctagtgtagc 1860
   cgtagttagg ccaccacttc aagaactctg tagcaccgcc tacatacctc gctctgctaa 1920
   tcctgttacc agtggctgct gccagtggcg ataagtcgtg tcttaccggg ttggactcaa 1980
   gacgatagtt accggataag gcgcagcggt cgggctgaac ggggggttcg tgcacacagc 2040
   ccagcttgga gcgaacgacc tacaccgaac tgagatacct acagcgtgag cattgagaaa 2100
   gcgccacgct tcccgaaggg agaaaggcgg acaggtatcc ggtaagcggc agggtcggaa 2160
   caggagagcg cacgagggag cttccagggg gaaacgcctg gtatctttat agtcctgtcg 2220
   ggtttcgcca cctctgactt gagcgtcgat ttttgtgatg ctcgtcaggg gggcggagcc 2280
   tatggaaaaa cgccagcaac gcggcctttt tacggttcct ggccttttgc tggccttttg 2340
   ctcacatgtt ctttcctgcg ttatcccctg attctgtgga taaccgtatt accgcctttg 2400
   agtgagctga taccgctcgc cgcagccgaa cgaccgagcg cagcgagtca gtgagcgagg 2460
   aagcggaaga gcgcccaata cgcaaaccgc ctctccccgc gcgttggccg attcattaat 2520
   gcagctgtgg aatgtgtgtc agttagggtg tggaaagtcc ccaggctccc cagcaggcag 2580
   aagtatgcaa agcatgcatc tcaattagtc agcaaccagg tgtggaaagt ccccaggctc 2640
   cccagcaggc agaagtatgc aaagcatgca tctcaattag tcagcaacca tagtcccgcc 2700
   cctaactccg cccatcccgc ccctaactcc gcccagttcc gcccattctc cgccccatgg 2760
   ctgactaatt ttttttattt atgcagaggc cgaggccgcc tcggcctctg agctattcca 2820
   gaagtagtga ggaggctttt ttggaggcct aggcttttgc aaaaagcttg gacacaagac 2880
   aggcttgcga gatatgtttg agaataccac tttatcccgc gtcagggaga ggcagtgcgt 2940
   aaaaagacgc ggactcatgt gaaatactgg tttttagtgc gccagatctc tataatctcg 3000
   cgcaacctat ttcccctcga acactttttt aagccgtaga taaacaggct gggacacttc 3060
   acatgagcga aaaatacatc gtcacctggg acatgttgca gatccatgca cgtaaactcg 3120
   caagccgact gatgccttct gaacaatgga aaggcattat tgccgtaagc cgtggcggtc 3180
   tgtaccgggt gcgttactgg cgcgtgaact gggtattcgt catgtcgata ccgtttgtat 3240
   ttccagctac gatcacgaca accagcgcga gcttaaagtg ctgaaacgcg cagaaggcga 3300
   tggcgaaggc ttcatcgtta ttgatgacct ggtggatacc ggtggtactg cggttgcgat 3360
   tcgtgaaatg tatccaaaag cgcactttgt caccatcttc gcaaaaccgg ctggtcgtcc 3420
   gctggttgat gactatgttg ttgatatccc gcaagatacc tggattgaac agccgtggga 3480
   tatgggcgtc gtattcgtcc cgccaatctc cggtcgctaa tcttttcaac gcctggcact 3540
   gccgggcgtt gttcttttta acttcaggcg ggttacaata gtttccagta agtattctgg 3600
   aggctgcatc catgacacag gcaaacctga gcgaaaccct gttcaaaccc cgctttaaac 3660
   atcctgaaac ctcgacgcta gtccgccgct ttaatcacgg cgcacaaccg cctgtgcagt 3720
   cggcccttga tggtaaaacc atccctcact ggtatcgcat gattaaccgt ctgatgtgga 3780
   tctggcgcgg cattgaccca cgcgaaatcc tcgacgtcca ggcacgtatt gtgatgagcg 3840
   atgccgaacg taccgacgat gatttatacg atacggtgat tggctaccgt ggcggcaact 3900
   ggatttatga gtgggccccg gatctttgtg aaggaacctt acttctgtgg tgtgacataa 3960
   ttggacaaac tacctacaga gatttaaagc tctaaggtaa atataaaatt tttaagtgta 4020
   taatgtgtta aactactgat tctaattgtt tgtgtatttt agattccaac ctatggaact 4080
   gatgaatggg agcagtggtg gaatgccttt aatgaggaaa acctgttttg ctcagaagaa 4140
   atgccatcta gtgatgatga ggctactgct gactctcaac attctactcc tccaaaaaag 4200
   aagagaaagg tagaagaccc caaggacttt ccttcagaat tgctaagttt tttgagtcat 4260
   gctgtgttta gtaatagaac tcttgcttgc tttgctattt acaccacaaa ggaaaaagct 4320
   gcactgctat acaagaaaat tatggaaaaa tattctgtaa cctttataag taggcataac 4380
   agttataatc ataacatact gttttttctt actccacaca ggcatagagt gtctgctatt 4440
   aataactatg ctcaaaaatt gtgtaccttt agctttttaa tttgtaaagg ggttaataag 4500
   gaatatttga tgtatagtgc cttgactaga gatcataatc agccatacca catttgtaga 4560
   ggttttactt gctttaaaaa acctcccaca cctccccctg aacctgaaac ataaaatgaa 4620
   tgcaattgtt gttgttaact tgtttattgc agcttataat ggttacaaat aaagcaatag 4680
   catcacaaat ttcacaaata aagcattttt ttcactgcat tctagttgtg gtttgtccaa 4740
   actcatcaat gtatcttatc atgtctggat caactggata actcaagcta accaaaatca 4800
   tcccaaactt cccaccccat accctattac cactgccaat tacctagtgg tttcatttac 4860
   tctaaacctg tgattcctct gaattatttt cattttaaag aaattgtatt tgttaaatat 4920
   gtactacaaa cttagtagtt ggaagggcta attcactccc aacgaagaca agatatcctt 4980
   gatctgtgga tctaccacac acaaggctac ttccctgatt agcagaacta cacaccaggg 5040
   ccagggatca gatatccact gacctttgga tggtgctaca agctagtacc agttgagcca 5100
   gagaagttag aagaagccaa caaaggagag aacaccagct tgttacaacc tgtgagcctg 5160
   catgggatgg atgacccgga gagagaagtg ttagagtgga ggtttgacag ccgcctagca 5220
   tttcatcacg gtggcccgag agctgcatcc ggagtacttc aagaactgct gatatcgagc 5280
   ttgctacaag ggactttccg ctggggactt ttccagggag gcgtggcctg ggcgggactg 5340
   gggagtggcg agccctcaga tcctgcatat aagcagctgc tttttgcctg tactgggtct 5400
   ctctggttag accagatctg agcctgggag ctctctggct aactagggaa cccactgctt 5460
   aagcctcaat aaagcttgcc ttgagtgctt caagtagtgt gtgcccgtct gttgtgtgac 5520
   tctggtaact agagatccct cagacccttt tagtcagtgt ggaaaatctc tagcagtggc 5580
   gcccgaacag ggacttgaaa gcgaaaggga aaccagagga gctctctcga cgcaggactc 5640
   ggcttgctga agcgcgcacg gcaagaggcg aggggcggcg actggtgagt acgccaaaaa 5700
   ttttgactag cggaggctag aaggagagag atgggtgcga gagcgtcagt attaagcggg 5760
   ggagaattag atcgcgatgg gaaaaaattc ggttaaggcc agggggaaag aaaaaatata 5820
   aattaaaaca tatagtatgg gcaagcaggg agctagaacg attcgcagtt aatcctggcc 5880
   tgttagaaac atcagaaggc tgtagacaaa tactgggaca gctacaacca tcccttcaga 5940
   caggatcaga agaacttaga tcattatata atacagtagc aaccctctat tgtgtgcatc 6000
   aaaggataga gataaaagac accaaggaag ctttagacaa gatagaggaa gagcaaaaca 6060
   aaagtaagac caccgcacag caagcggccg ctgatcttca gacctggagg aggagatatg 6120
   agggacaatt ggagaagtga attatataaa tataaagtag taaaaattga accattagga 6180
   gtagcaccca ccaaggcaaa gagaagagtg gtgcagagag aaaaaagagc agtgggaata 6240
   ggagctttgt tccttgggtt cttgggagca gcaggaagca ctatgggcgc agcgtcaatg 6300
   acgctgacgg tacaggccag acaattattg tctggtatag tgcagcagca gaacaatttg 6360
   ctgagggcta ttgaggcgca acagcatctg ttgcaactca cagtctgggg catcaagcag 6420
   ctccaggcaa gaatcctggc tgtggaaaga tacctaaagg atcaacagct cctggggatt 6480
   tggggttgct ctggaaaact catttgcacc actgctgtgc cttggaatgc tagttggagt 6540
   aataaatctc tggaacagat ttggaatcac acgacctgga tggagtggga cagagaaatt 6600
   aacaattaca caagcttaat acactcctta attgaagaat cgcaaaacca gcaagaaaag 6660
   aatgaacaag aattattgga attagataaa tgggcaagtt tgtggaattg gtttaacata 6720
   acaaattggc tgtggtatat aaaattattc ataatgatag taggaggctt ggtaggttta 6780
   agaatagttt ttgctgtact ttctatagtg aatagagtta ggcagggata ttcaccatta 6840
   tcgtttcaga cccacctccc aaccccgagg ggacccgaca ggcccgaagg aatagaagaa 6900
   gaaggtggag agagagacag agacagatcc attcgattag tgaacggatc tcgacggtat 6960
   cgccgaattc acaaatggca gtattcatcc acaattttaa aagaaaaggg gggattgggg 7020
   ggtacagtgc aggggaaaga atagtagaca taatagcaac agacatacaa actaaagaat 7080
   tacaaaaaca aattacaaaa attcaaaatt ttcgggttta ttacagggac agcagagatc 7140
   cactttggct gatacgcgga tctacgcgtg gatccaccgg tcgccaccat ggtgagcaag 7200
   ggcgaggagc tgttcaccgg ggtggtgccc atcctggtcg agctggacgg cgacgtaaac 7260
   ggccacaagt tcagcgtgtc cggcgagggc gagggcgatg ccacctacgg caagctgacc 7320
   ctgaagttca tctgcaccac cggcaagctg cccgtgccct ggcccaccct cgtgaccacc 7380
   ctgacctacg gcgtgcagtg cttcagccgc taccccgacc acatgaagca gcacgacttc 7440
   ttcaagtccg ccatgcccga aggctacgtc caggagcgca ccatcttctt caaggacgac 7500
   ggcaactaca agacccgcgc cgaggtgaag ttcgagggcg acaccctggt gaaccgcatc 7560
   gagctgaagg gcatcgactt caaggaggac ggcaacatcc tggggcacaa gctggagtac 7620
   aactacaaca gccacaacgt ctatatcatg gccgacaagc agaagaacgg catcaaggtg 7680
   aacttcaaga tccgccacaa catcgaggac ggcagcgtgc agctcgccga ccactaccag 7740
   cagaacaccc ccatcggcga cggccccgtg ctgctgcccg acaaccacta cctgagcacc 7800
   cagtccgccc tgagcaaaga ccccaacgag aagcgcgatc acatggtcct gctggagttc 7860
   gtgaccgccg ccgggatcac tctcggcatg gacgagctgt acaagtaaag cggccgcggg 7920
   cccatatgat cgatacgtac tagtctcgag ctcaagcttc gaattcccga taatcaacct 7980
   ctggattaca aaatttgtga aagattgact ggtattctta actatgttgc tccttttacg 8040
   ctatgtggat acgctgcttt aatgcctttg tatcatgcta ttgcttcccg tatggctttc 8100
   attttctcct ccttgtataa atcctggttg ctgtctcttt atgaggagtt gtggcccgtt 8160
   gtcaggcaac gtggcgtggt gtgcactgtg tttgctgacg caacccccac tggttggggc 8220
   attgccacca cctgtcagct cctttccggg actttcgctt tccccctccc tattgccacg 8280
   gcggaactca tcgccgcctg ccttgcccgc tgctggacag gggctcggct gttgggcact 8340
   gacaattccg tggtgttgtc ggggaagctg acgtcctttc catggctgct cgcctgtgtt 8400
   gccacctgga ttctgcgcgg gacgtccttc tgctacgtcc cttcggccct caatccagcg 8460
   gaccttcctt cccgcggcct gctgccggct ctgcggcctc ttccgcgtct tcgccttcgc 8520
   cctcagacga gtcggatctc cctttgggcc gcctccccgc atcgggaatt ctgcagtcga 8580
   cggtaccttt aagaccaatg acttacaagg cagctgtaga tcttagccac tttttaaaag 8640
   aaaagggggg actggaaggg ctaattcact cccaacgaag acaaaatcgt cgagagatgc 8700
   tgcatataag cagctgcttt ttgcttgtac tgggtctctc tggttagacc agatctgagc 8760
   ctgggagctc tctggctaac tagggaaccc actgcttaag cctcaataaa gcttgccttg 8820
   agtgcttcaa gtagtgtgtg cccgtctgtt gtgtgactct ggtaactaga gatccctcag 8880
   acccttttag tcagtgtgga aaatctctag cagtagtagt tcatgtcatc ttattattca 8940
   gtatttataa cttgcaaaga aatgaatatc agagagtgag aggccttgac attataatag 9000
   atttagcagg aattgaacta ggagtggagc acacaggcaa agctgcagaa gtacttggaa 9060
   gaagccacca gagatactca cgattctgca catacctggc taatcccaga tcctaaggat 9120
   tacattaagt ttactaacat ttatataatg atttatagtt taaagtataa acttatctaa 9180
   tttactattc tgacagatat taattaatcc tcaaatatca taagagatga ttactattat 9240
   ccccatttaa cacaagagga aactgagagg gaaagatgtt gaagtaattt tcccacaatt 9300
   acagcatccg ttagttacga ctctatgatc ttctgacaca aattccattt actcctcacc 9360
   ctatgactca gtcgaatata tcaaagttat ggacattatg ctaagtaaca aattaccctt 9420
   ttatatagta aatactgagt agattgagag aagaaattgt ttgcaaacct gaatagcttc 9480
   aagaagaaga gaagtgagga taagaataac agttgtcatt taacaagttt taacaagtaa 9540
   cttggttaga aagggattca aatgcataaa gcaagggata aatttttctg gcaacaagac 9600
   tatacaatat aaccttaaat atgacttcaa ataattgttg gaacttgata aaactaatta 9660
   aatattattg aagattatca atattataaa tgtaatttac ttttaaaaag ggaacataga 9720
   aatgtgtatc attagagtag aaaacaatcc ttattatcac aatttgtcaa aacaagtttg 9780
   ttattaacac aagtagaata ctgcattcaa ttaagttgac tgcagatttt gtgttttgtt 9840
   aaaattagaa agagataaca acaatttgaa ttattgaaag taacatgtaa atagttctac 9900
   atacgttctt ttgacatctt gttcaatcat tgatcgaagt tctttatctt ggaagaattt 9960
   gttccaaaga ctctgaaata aggaaaacaa tctattatat agtctcacac ctttgtttta 10020
   cttttagtga tttcaattta ataatgtaaa tggttaaaat ttattcttct ctgagatcat 10080
   ttcacattgc agatagaaaa cctgagactg gggtaatttt tattaaaatc taatttaatc 10140
   tcagaaacac atctttattc taacatcaat ttttccagtt tgatattatc atataaagtc 10200
   agccttcctc atctgcaggt tccacaacaa aaatccaacc aactgtggat caaaaatatt 10260
   gggaaaaaat taaaaatagc aatacaacaa taaaaaaata caaatcagaa aaacagcaca 10320
   gtataacaac tttatttagc atttacaatc tattaggtat tataagtaat ctag 10374

<210> 2
<211> 10356
<212> DNA
<213> Artificial sequence

<220>
<223> SEQ ID NO: 2

<400> 2
ccagatcctc tacgccggac gcatcgtggc cggcatcacc ggcgccacag gtgcggttgc 60

tggcgcctat atcgccgaca tcaccgatgg ggaagatcgg gctcgccact tcgggctcat 120

gagcgcttgt ttcggcgtgg gtatggtggc aggccccgtg gccgggggac tgttgggcgc 180

catctccttg catgcaccat tccttgcggc ggcggtgctc aacggcctca acctactact 240

gggctgcttc ctaatgcagg agtcgcataa gggagagcgt cgaatggtgc actctcagta 300

caatctgctc tgatgccgca tagttaagcc agccccgaca cccgccaaca cccgctgacg 360

cgccctgacg ggcttgtctg ctcccggcat ccgcttacag acaagctgtg accgtctccg 420

ggagctgcat gtgtcagagg ttttcaccgt catcaccgaa acgcgcgaga cgaaagggcc 480

tcgtgatacg cctattttta taggttaatg tcatgataat aatggtttct tagacgtcag 540

gtggcacttt tcggggaaat gtgcgcggaa cccctatttg tttatttttc taaatacatt 600

caaatatgta tccgctcatg agacaataac cctgataaat gcttcaataa tattgaaaaa 660

ggaagagtat gagtattcaa catttccgtg tcgcccttat tccctttttt gcggcatttt 720

gccttcctgt ttttgctcac ccagaaacgc tggtgaaagt aaaagatgct gaagatcagt 780

tgggtgcacg agtgggttac atcgaactgg atctcaacag cggtaagatc cttgagagtt 840

ttcgccccga agaacgtttt ccaatgatga gcacttttaa agttctgcta tgtggcgcgg 900

tattatcccg tattgacgcc gggcaagagc aactcggtcg ccgcatacac tattctcaga 960

atgacttggt tgagtactca ccagtcacag aaaagcatct tacggatggc atgacagtaa 1020

gagaattatg cagtgctgcc ataaccatga gtgataacac tgcggccaac ttacttctga 1080

caacgatcgg aggaccgaag gagctaaccg cttttttgca caacatgggg gatcatgtaa 1140

ctcgccttga tcgttgggaa ccggagctga atgaagccat accaaacgac gagcgtgaca 1200

ccacgatgcc tgtagcaatg gcaacaacgt tgcgcaaact attaactggc gaactactta 1260

ctctagcttc ccggcaacaa ttaatagact ggatggaggc ggataaagtt gcaggaccac 1320

ttctgcgctc ggcccttccg gctggctggt ttattgctga taaatctgga gccggtgagc 1380

gtgggtctcg cggtatcatt gcagcactgg ggccagatgg taagccctcc cgtatcgtag 1440

ttatctacac gacggggagt caggcaacta tggatgaacg aaatagacag atcgctgaga 1500

taggtgcctc actgattaag cattggtaac tgtcagacca agtttactca tatatacttt 1560

agattgattt aaaacttcat ttttaattta aaaggatcta ggtgaagatc ctttttgata 1620

atctcatgac caaaatccct taacgtgagt tttcgttcca ctgagcgtca gaccccgtag 1680

aaaagatcaa aggatcttct tgagatcctt tttttctgcg cgtaatctgc tgcttgcaaa 1740

caaaaaaacc accgctacca gcggtggttt gtttgccgga tcaagagcta ccaactcttt 1800

ttccgaaggt aactggcttc agcagagcgc agataccaaa tactgtcctt ctagtgtagc 1860

cgtagttagg ccaccacttc aagaactctg tagcaccgcc tacatacctc gctctgctaa 1920

tcctgttacc agtggctgct gccagtggcg ataagtcgtg tcttaccggg ttggactcaa 1980

gacgatagtt accggataag gcgcagcggt cgggctgaac ggggggttcg tgcacacagc 2040

ccagcttgga gcgaacgacc tacaccgaac tgagatacct acagcgtgag cattgagaaa 2100

gcgccacgct tcccgaaggg agaaaggcgg acaggtatcc ggtaagcggc agggtcggaa 2160

caggagagcg cacgagggag cttccagggg gaaacgcctg gtatctttat agtcctgtcg 2220

ggtttcgcca cctctgactt gagcgtcgat ttttgtgatg ctcgtcaggg gggcggagcc 2280

tatggaaaaa cgccagcaac gcggcctttt tacggttcct ggccttttgc tggccttttg 2340

ctcacatgtt ctttcctgcg ttatcccctg attctgtgga taaccgtatt accgcctttg 2400

agtgagctga taccgctcgc cgcagccgaa cgaccgagcg cagcgagtca gtgagcgagg 2460

aagcggaaga gcgcccaata cgcaaaccgc ctctccccgc gcgttggccg attcattaat 2520

gcagctgtgg aatgtgtgtc agttagggtg tggaaagtcc ccaggctccc cagcaggcag 2580

aagtatgcaa agcatgcatc tcaattagtc agcaaccagg tgtggaaagt ccccaggctc 2640

cccagcaggc agaagtatgc aaagcatgca tctcaattag tcagcaacca tagtcccgcc 2700

cctaactccg cccatcccgc ccctaactcc gcccagttcc gcccattctc cgccccatgg 2760

ctgactaatt ttttttattt atgcagaggc cgaggccgcc tcggcctctg agctattcca 2820

gaagtagtga ggaggctttt ttggaggcct aggcttttgc aaaaagcttg gacacaagac 2880

aggcttgcga gatatgtttg agaataccac tttatcccgc gtcagggaga ggcagtgcgt 2940

aaaaagacgc ggactcatgt gaaatactgg tttttagtgc gccagatctc tataatctcg 3000

cgcaacctat tttcccctcg aacacttttt aagccgtaga taaacaggct gggacacttc 3060

acatgagcga aaaatacatc gtcacctggg acatgttgca gatccatgca cgtaaactcg 3120

caagccgact gatgccttct gaacaatgga aaggcattat tgccgtaagc cgtggcggtc 3180

tgtaccgggt gcgttactgg cgcgtgaact gggtattcgt catgtcgata ccgtttgtat 3240

ttccagctac gatcacgaca accagcgcga gcttaaagtg ctgaaacgcg cagaaggcga 3300

tggcgaaggc ttcatcgtta ttgatgacct ggtggatacc ggtggtactg cggttgcgat 3360

tcgtgaaatg tatccaaaag cgcactttgt caccatcttc gcaaaaccgg ctggtcgtcc 3420

gctggttgat gactatgttg ttgatatccc gcaagatacc tggattgaac agccgtggga 3480

tatgggcgtc gtattcgtcc cgccaatctc cggtcgctaa tcttttcaac gcctggcact 3540

gccgggcgtt gttcttttta acttcaggcg ggttacaata gtttccagta agtattctgg 3600

aggctgcatc catgacacag gcaaacctga gcgaaaccct gttcaaaccc cgctttaaac 3660

atcctgaaac ctcgacgcta gtccgccgct ttaatcacgg cgcacaaccg cctgtgcagt 3720

cggcccttga tggtaaaacc atccctcact ggtatcgcat gattaaccgt ctgatgtgga 3780

tctggcgcgg cattgaccca cgcgaaatcc tcgacgtcca ggcacgtatt gtgatgagcg 3840

atgccgaacg taccgacgat gatttatacg atacggtgat tggctaccgt ggcggcaact 3900

ggatttatga gtgggccccg gatctttgtg aaggaacctt acttctgtgg tgtgacataa 3960

ttggacaaac tacctacaga gatttaaagc tctaaggtaa atataaaatt tttaagtgta 4020

taatgtgtta aactactgat tctaattgtt tgtgtatttt agattccaac ctatggaact 4080

gatgaatggg agcagtggtg gaatgccttt aatgaggaaa acctgttttg ctcagaagaa 4140

atgccatcta gtgatgatga ggctactgct gactctcaac attctactcc tccaaaaaag 4200

aagagaaagg tagaagaccc caaggacttt ccttcagaat tgctaagttt tttgagtcat 4260

gctgtgttta gtaatagaac tcttgcttgc tttgctattt acaccacaaa ggaaaaagct 4320

gcactgctat acaagaaaat tatggaaaaa tattctgtaa cctttataag taggcataac 4380

agttataatc ataacatact gttttttctt actccacaca ggcatagagt gtctgctatt 4440

aataactatg ctcaaaaatt gtgtaccttt agctttttaa tttgtaaagg ggttaataag 4500

gaatatttga tgtatagtgc cttgactaga gatcataatc agccatacca catttgtaga 4560

ggttttactt gctttaaaaa acctcccaca cctccccctg aacctgaaac ataaaatgaa 4620

tgcaattgtt gttgttaact tgtttattgc agcttataat ggttacaaat aaagcaatag 4680

catcacaaat ttcacaaata aagcattttt ttcactgcat tctagttgtg gtttgtccaa 4740

actcatcaat gtatcttatc atgtctggat caactggata actcaagcta accaaaatca 4800

tcccaaactt cccaccccat accctattac cactgccaat tacctagtgg tttcatttac 4860

tctaaacctg tgattcctct gaattatttt cattttaaag aaattgtatt tgttaaatat 4920

gtactacaaa cttagtagtt ggaagggcta attcactccc aacgaagaca agatatcctt 4980

gatctgtgga tctaccacac acaaggctac ttccctgatt agcagaacta cacaccaggg 5040

ccagggatca gatatccact gacctttgga tggtgctaca agctagtacc agttgagcca 5100

gagaagttag aagaagccaa caaaggagag aacaccagct tgttacaacc tgtgagcctg 5160

catgggatgg atgacccgga gagagaagtg ttagagtgga ggtttgacag ccgcctagca 5220

tttcatcacg gtggcccgag agctgcatcc ggagtacttc aagaactgct gatatcgagc 5280

ttgctacaag ggactttccg ctggggactt tccagggagg cgtggcctgg gcgggactgg 5340

ggagtggcga gccctcagat cctgcatata agcagctgct ttttgcctgt actgggtctc 5400

tctggttaga ccagatctga gcctgggagc tctctggcta actagggaac ccactgctta 5460

agcctcaata aagcttgcct tgagtgcttc aagtagtgtg tgcccgtctg ttgtgtgact 5520

ctggtaacta gagatccctc agaccctttt agtcagtgtg gaaaatctct agcagttgaa 5580

agcgaaaggg aaaccagagg agctctctcg acgcaggact cggcttgctg aagcgcgcac 5640

ggcaagaggc gaggggcggc gactggtgag tacgccaaaa attttgacta gcggaggcta 5700

gaaggagaga gatgggtgcg agagcgtcag tattaagcgg gggagaatta gatcgcgatg 5760

ggaaaaaatt cggttaaggc cagggggaaa gaaaaaatat aaattaaaac atatagtatg 5820

ggcaagcagg gagctagaac gattcgcagt taatcctggc ctgttagaaa catcagaagg 5880

ctgtagacaa atactgggac agctacaacc atcccttcag acaggatcag aagaacttag 5940

atcattatat aatacagtag caaccctcta ttgtgtgcat caaaggatag agataaaaga 6000

caccaaggaa gctttagaca agatagagga agagcaaaac aaaagtaaga ccaccgcaca 6060

gcaagcggcc gctgatcttc agacctggag gaggagatat gagggacaat tggagaagtg 6120

aattatataa atataaagta gtaaaaattg aaccattagg agtagcaccc accaaggcaa 6180

agagaagagt ggtgcagaga gaaaaaagag cagtgggaat aggagctttg ttccttgggt 6240

ttcttgggag cagcaggaag cactatgggc gcagcgtcaa tgacgctgac ggtacaggcc 6300

agacaattat tgtctggtat agtgcagcag cagaacaatt tgctgagggc tattgaggcg 6360

caacagcatc tgttgcaact cacagtctgg ggcatcaagc agctccaggc aagaatcctg 6420

gctgtggaaa gatacctaaa ggatcaacag ctcctgggga tttggggttg ctctggaaaa 6480

ctcatttgca ccactgctgt gccttggaat gctagttgga gtaataaatc tctggaacag 6540

atttggaatc acacgacctg gatggagtgg gacagagaaa ttaacaatta cacaagctta 6600

atacactcct taattgaaga atcgcaaaac cagcaagaaa agaatgaaca agaattattg 6660

gaattagata aatgggcaag tttgtggaat tggtttaaca taacaaattg gctgtggtat 6720

ataaaattat tcataatgat agtaggaggc ttggtaggtt taagaatagt ttttgctgta 6780

ctttctatag tgaatagagt taggcaggga tattcaccat tatcgtttca gacccacctc 6840

ccaaccccga ggggacccga caggcccgaa ggaatagaag aagaaggtgg agagagagac 6900

agagacagat ccattcgatt agtgaacgga tctcgacggt atcgccgaat tcacaaatgg 6960

cagtattcat ccacaatttt aaaagaaaag gggggattgg ggggtacagt gcaggggaaa 7020

gaatagtaga cataatagca acagacatac aaactaaaga attacaaaaa caaattacaa 7080

aaattcaaaa ttttcgggtt tattacaggg acagcagaga tccactttgg ctgatacgcg 7140

gatctacgcg tggatccacc ggtcgccacc atggtgagca agggcgagga gctgttcacc 7200

ggggtggtgc ccatcctggt cgagctggac ggcgacgtaa acggccacaa gttcagcgtg 7260

tccggcgagg gcgagggcga tgccacctac ggcaagctga ccctgaagtt catctgcacc 7320

accggcaagc tgcccgtgcc ctggcccacc ctcgtgacca ccctgaccta cggcgtgcag 7380

tgcttcagcc gctaccccga ccacatgaag cagcacgact tcttcaagtc cgccatgccc 7440

gaaggctacg tccaggagcg caccatcttc ttcaaggacg acggcaacta caagacccgc 7500

gccgaggtga agttcgaggg cgacaccctg gtgaaccgca tcgagctgaa gggcatcgac 7560

ttcaaggagg acggcaacat cctggggcac aagctggagt acaactacaa cagccacaac 7620

gtctatatca tggccgacaa gcagaagaac ggcatcaagg tgaacttcaa gatccgccac 7680

aacatcgagg acggcagcgt gcagctcgcc gaccactacc agcagaacac ccccatcggc 7740

gacggccccg tgctgctgcc cgacaaccac tacctgagca cccagtccgc cctgagcaaa 7800

gaccccaacg agaagcgcga tcacatggtc ctgctggagt tcgtgaccgc cgccgggatc 7860

actctcggca tggacgagct gtacaagtaa agcggccgcg ggcccatatg atcgatacgt 7920

actagtctcg agctcaagct tcgaattccc gataatcaac ctctggatta caaaatttgt 7980

gaaagattga ctggtattct taactatgtt gctcctttta cgctatgtgg atacgctgct 8040

ttaatgcctt tgtatcatgc tattgcttcc cgtatggctt tcattttctc ctccttgtat 8100

aaatcctggt tgctgtctct ttatgaggag ttgtggcccg ttgtcaggca acgtggcgtg 8160

gtgtgcactg tgtttgctga cgcaaccccc actggttggg gcattgccac cacctgtcag 8220

ctcctttccg ggactttcgc tttccccctc cctattgcca cggcggaact catcgccgcc 8280

tgccttgccc gctgctggac aggggctcgg ctgttgggca ctgacaattc cgtggtgttg 8340

tcggggaagc tgacgtcctt tccatggctg ctcgcctgtg ttgccacctg gattctgcgc 8400

gggacgtcct tctgctacgt cccttcggcc ctcaatccag cggaccttcc ttcccgcggc 8460

ctgctgccgg ctctgcggcc tcttccgcgt cttcgccttc gccctcagac gagtcggatc 8520

tccctttggg ccgcctcccc gcatcgggaa ttctgcagtc gacggtacct ttaagaccaa 8580

tgacttacaa ggcagctgta gatcttagcc actttttaaa agaaaagggg ggactggaag 8640

ggctaattca ctcccaacga agacaaaatc gtcgagagat gctgcatata agcagctgct 8700

ttttgcttgt actgggtctc tctggttaga ccagatctga gcctgggagc tctctggcta 8760

actagggaac ccactgctta agcctcaata aagcttgcct tgagtgcttc aagtagtgtg 8820

tgcccgtctg ttgtgtgact ctggtaacta gagatccctc agaccctttt agtcagtgtg 8880

gaaaatctct agcagtagta gttcatgtca tcttattatt cagtatttat aacttgcaaa 8940

gaaatgaata tcagagagtg agaggccttg acattataat agatttagca ggaattgaac 9000

taggagtgga gcacacaggc aaagctgcag aagtacttgg aagaagccac cagagatact 9060

cacgattctg cacatacctg gctaatccca gatcctaagg attacattaa gtttactaac 9120

atttatataa tgatttatag tttaaagtat aaacttatct aatttactat tctgacagat 9180

attaattaat cctcaaatat cataagagat gattactatt atccccattt aacacaagag 9240

gaaactgaga gggaaagatg ttgaagtaat tttcccacaa ttacagcatc cgttagttac 9300

gactctatga tcttctgaca caaattccat ttactcctca ccctatgact cagtcgaata 9360

tatcaaagtt atggacatta tgctaagtaa caaattaccc ttttatatag taaatactga 9420

gtagattgag agaagaaatt gtttgcaaac ctgaatagct tcaagaagaa gagaagtgag 9480

gataagaata acagttgtca tttaacaagt tttaacaagt aacttggtta gaaagggatt 9540

caaatgcata aagcaaggga taaatttttc tggcaacaag actatacaat ataaccttaa 9600

atatgacttc aaataattgt tggaacttga taaaactaat taaatattat tgaagattat 9660

caatattata aatgtaattt acttttaaaa agggaacata gaaatgtgta tcattagagt 9720

agaaaacaat ccttattatc acaatttgtc aaaacaagtt tgttattaac acaagtagaa 9780

tactgcattc aattaagttg actgcagatt ttgtgttttg ttaaaattag aaagagataa 9840

caacaatttg aattattgaa agtaacatgt aaatagttct acatacgttc ttttgacatc 9900

ttgttcaatc attgatcgaa gttctttatc ttggaagaat ttgttccaaa gactctgaaa 9960

taaggaaaac aatctattat atagtctcac acctttgttt tacttttagt gatttcaatt 10020

taataatgta aatggttaaa atttattctt ctctgagatc atttcacatt gcagatagaa 10080

aacctgagac tggggtaatt tttattaaaa tctaatttaa tctcagaaac acatctttat 10140

tctaacatca atttttccag tttgatatta tcatataaag tcagccttcc tcatctgcag 10200

gttccacaac aaaaatccaa ccaactgtgg atcaaaaata ttgggaaaaa attaaaaata 10260

gcaatacaac aataaaaaaa tacaaatcag aaaaacagca cagtataaca actttattta 10320

gcatttacaa tctattaggt attataagta atctag 10356

<210> 3
<211> 12150
<212> DNA
<213> Artificial sequence

<220>
<223> SEQ ID NO: 3

<400> 3
ttgattattg actagttatt aatagtaatc aattacgggg tcattagttc atagcccata 60

tatggagttc cgcgttacat aacttacggt aaatggcccg cctggctgac cgcccaacga 120

cccccgccca ttgacgtcaa taatgacgta tgttcccata gtaacgccaa tagggacttt 180

ccattgacgt caatgggtgg agtatttacg gtaaactgcc cacttggcag tacatcaagt 240

gtatcatatg ccaagtacgc cccctattga cgtcaatgac ggtaaatggc ccgcctggca 300

ttatgcccag tacatgacct tatgggactt tcctacttgg cagtacatct acgtattagt 360

catcgctatt accatggtga tgcggttttg gcagtacatc aatgggcgtg gatagcggtt 420

tgactcacgg ggatttccaa gtctccaccc cattgacgtc aatgggagtt tgttttggca 480

ccaaaatcaa cgggactttc caaaatgtcg taacaactcc gccccattga cgcaaatggg 540

cggtaggcgt gtacggtggg aggtctatat aagcagagct cgtttagtga accgtcagat 600

cgcctggaga cgccatccac gctgttttga cctccataga agacaccggg accgatccag 660

cctccgcggc cgggaacggt gcattggaac gcggattccc cgtgccaaga gtgacgtaag 720

taccgcctat agagtctata ggcccacccc cttggcttct tatgcgacgg atcgatcccg 780

taataagctt cgaggtccgc ggccggccgc gttgacgcgc acggcaagag gcgaggggcg 840

gcgactggtg agagatgggt gcgagagcgt cagtattaag cgggggagaa ttagatcgat 900

gggaaaaaat tcggttaagg ccagggggaa agaaaaaata taaattaaaa catatagtat 960

gggcaagcag ggagctagaa cgattcgcag ttaatcctgg cctgttagaa acatcagaag 1020

gctgtagaca aatactggga cagctacaac catcccttca gacaggatca gaagaactta 1080

gatcattata taatacagta gcaaccctct attgtgtgca tcaaaggata gagataaaag 1140

acaccaagga agctttagac aagatagagg aagagcaaaa caaaagtaag aaaaaagcac 1200

agcaagcagc agctgacaca ggacacagca atcaggtcag ccaaaattac cctatagtgc 1260

agaacatcca ggggcaaatg gtacatcagg ccatatcacc tagaacttta aatgcatggg 1320

taaaagtagt agaagagaag gctttcagcc cagaagtgat acccatgttt tcagcattat 1380

cagaaggagc caccccacaa gatttaaaca ccatgctaaa cacagtgggg ggacatcaag 1440

cagccatgca aatgttaaaa gagaccatca atgaggaagc tgcagaatgg gatagagtgc 1500

atccagtgca tgcagggcct attgcaccag gccagatgag agaaccaagg ggaagtgaca 1560

tagcaggaac tactagtacc cttcaggaac aaataggatg gatgacacat aatccaccta 1620

tcccagtagg agaaatctat aaaagatgga taatcctggg attaaataaa atagtaagaa 1680

tgtatagccc taccagcatt ctggacataa gacaaggacc aaaggaaccc tttagagact 1740

atgtagaccg attctataaa actctaagag ccgagcaagc ttcacaagag gtaaaaaatt 1800

ggatgacaga aaccttgttg gtccaaaatg cgaacccaga ttgtaagact attttaaaag 1860

cattgggacc aggagcgaca ctagaagaaa tgatgacagc atgtcaggga gtggggggac 1920

ccggccataa agcaagagtt ttggctgaag caatgagcca agtaacaaat ccagctacca 1980

taatgataca gaaaggcaat tttaggaacc aaagaaagac tgttaagtgt ttcaattgtg 2040

gcaaagaagg gcacatagcc aaaaattgca gggcccctag gaaaaagggc tgttggaaat 2100

gtggaaagga aggacaccaa atgaaagatt gtactgagag acaggctaat tttttaggga 2160

agatctggcc ttcccacaag ggaaggccag ggaattttct tcagagcaga ccagagccaa 2220

cagccccacc agaagagagc ttcaggtttg gggaagagac aacaactccc tctcagaagc 2280

aggagccgat agacaaggaa ctgtatcctt tagcttccct cagatcactc tttggcagcg 2340

acccctcgtc acaataaaga taggggggca attaaaggaa gctctattag atacaggagc 2400

agatgataca gtattagaag aaatgaattt gccaggaaga tggaaaccaa aaatgatagg 2460

gggaattgga ggttttatca aagtaagaca gtatgatcag atactcatag aaatctgcgg 2520

acataaagct ataggtacag tattagtagg acctacacct gtcaacataa ttggaagaaa 2580

tctgttgact cagattggct gcactttaaa ttttcccatt agtcctattg agactgtacc 2640

agtaaaatta aagccaggaa tggatggccc aaaagttaaa caatggccat tgacagaaga 2700

aaaaataaaa gcattagtag aaatttgtac agaaatggaa aaggaaggaa aaatttcaaa 2760

aattgggcct gaaaatccat acaatactcc agtatttgcc ataaagaaaa aagacagtac 2820

taaatggaga aaattagtag atttcagaga acttaataag agaactcaag atttctggga 2880

agttcaatta ggaataccac atcctgcagg gttaaaacag aaaaaatcag taacagtact 2940

ggatgtgggc gatgcatatt tttcagttcc cttagataaa gacttcagga agtatactgc 3000

atttaccata cctagtataa acaatgagac accagggatt agatatcagt acaatgtgct 3060

tccacaggga tggaaaggat caccagcaat attccagtgt agcatgacaa aaatcttaga 3120

gccttttaga aaacaaaatc cagacatagt catctatcaa tacatggatg atttgtatgt 3180

aggatctgac ttagaaatag ggcagcatag aacaaaaata gaggaactga gacaacatct 3240

gttgaggtgg ggatttacca caccagacaa aaaacatcag aaagaacctc cattcctttg 3300

gatgggttat gaactccatc ctgataaatg gacagtacag cctatagtgc tgccagaaaa 3360

ggacagctgg actgtcaatg acatacagaa attagtggga aaattgaatt gggcaagtca 3420

gatttatgca gggattaaag taaggcaatt atgtaaactt cttaggggaa ccaaagcact 3480

aacagaagta gtaccactaa cagaagaagc agagctagaa ctggcagaaa acagggagat 3540

tctaaaagaa ccggtacatg gagtgtatta tgacccatca aaagacttaa tagcagaaat 3600

acagaagcag gggcaaggcc aatggacata tcaaatttat caagagccat ttaaaaatct 3660

gaaaacagga aagtatgcaa gaatgaaggg tgcccacact aatgatgtga aacaattaac 3720

agaggcagta caaaaaatag ccacagaaag catagtaata tggggaaaga ctcctaaatt 3780

taaattaccc atacaaaagg aaacatggga agcatggtgg acagagtatt ggcaagccac 3840

ctggattcct gagtgggagt ttgtcaatac ccctccctta gtgaagttat ggtaccagtt 3900

agagaaagaa cccataatag gagcagaaac tttctatgta gatggggcag ccaataggga 3960

aactaaatta ggaaaagcag gatatgtaac tgacagagga agacaaaaag ttgtccccct 4020

aacggacaca acaaatcaga agactgagtt acaagcaatt catctagctt tgcaggattc 4080

gggattagaa gtaaacatag tgacagactc acaatatgca ttgggaatca ttcaagcaca 4140

accagataag agtgaatcag agttagtcag tcaaataata gagcagttaa taaaaaagga 4200

aaaagtctac ctggcatggg taccagcaca caaaggaatt ggaggaaatg aacaagtaga 4260

taaattggtc agtgctggaa tcaggaaagt actattttta gatggaatag ataaggccca 4320

agaagaacat gagaaatatc acagtaattg gagagcaatg gctagtgatt ttaacctacc 4380

acctgtagta gcaaaagaaa tagtagccag ctgtgataaa tgtcagctaa aaggggaagc 4440

catgcatgga caagtagact gtagcccagg aatatggcag ctagattgta cacatttaga 4500

aggaaaagtt atcttggtag cagttcatgt agccagtgga tatatagaag cagaagtaat 4560

tccagcagag acagggcaag aaacagcata cttcctctta aaattagcag gaagatggcc 4620

agtaaaaaca gtacatacag acaatggcag caatttcacc agtactacag ttaaggccgc 4680

ctgttggtgg gcggggatca agcaggaatt tggcattccc tacaatcccc aaagtcaagg 4740

agtaatagaa tctatgaata aagaattaaa gaaaattata ggacaggtaa gagatcaggc 4800

tgaacatctt aagacagcag tacaaatggc agtattcatc cacaatttta aaagaaaagg 4860

ggggattggg gggtacagtg caggggaaag aatagtagac ataatagcaa cagacataca 4920

aactaaagaa ttacaaaaac aaattacaaa aattcaaaat tttcgggttt attacaggga 4980

cagcagagat ccagtttgga aaggaccagc aaagctcctc tggaaaggtg aaggggcagt 5040

agtaatacaa gataatagtg acataaaagt agtgccaaga agaaaagcaa agatcatcag 5100

ggattatgga aaacagatgg caggtgatga ttgtgtggca agtagacagg atgaggatta 5160

acacatggaa ttctgcaaca actgctgttt atccatttca gaattgggtg tcgacatagc 5220

agaataggcg ttactcgaca gaggagagca agaaatggag ccagtagatc ctagactaga 5280

gccctggaag catccaggaa gtcagcctaa aactgcttgt accaattgct attgtaaaaa 5340

gtgttgcttt cattgccaag tttgtttcat gacaaaagcc ttaggcatct cctatggcag 5400

gaagaagcgg agacagcgac gaagagctca tcagaacagt cagactcatc aagcttctct 5460

atcaaagcag taagtagtac atgtaatgca acctataata gtagcaatag tagcattagt 5520

agtagcaata ataatagcaa tagttgtgtg gtccatagta atcatagaat ataggaaaat 5580

ggccgctgat cttcagacct ggaggaggag atatgaggga caattggaga agtgaattat 5640

ataaatataa agtagtaaaa attgaaccat taggagtagc acccaccaag gcaaagagaa 5700

gagtggtgca gagagaaaaa agagcagtgg gaataggagc tttgttcctt gggttcttgg 5760

gagcagcagg aagcactatg ggcgcagcgt caatgacgct gacggtacag gccagacaat 5820

tattgtctgg tatagtgcag cagcagaaca atttgctgag ggctattgag gcgcaacagc 5880

atctgttgca actcacagtc tggggcatca agcagctcca ggcaagaatc ctggctgtgg 5940

aaagatacct aaaggatcaa cagctcctgg ggatttgggg ttgctctgga aaactcattt 6000

gcaccactgc tgtgccttgg aatgctagtt ggagtaataa atctctggaa cagatttgga 6060

atcacacgac ctggatggag tgggacagag aaattaacaa ttacacaagc ttaatacact 6120

ccttaattga agaatcgcaa aaccagcaag aaaagaatga acaagaatta ttggaattag 6180

ataaatgggc aagtttgtgg aattggttta acataacaaa ttggctgtgg tatataaaat 6240

tattcataat gatagtagga ggcttggtag gtttaagaat agtttttgct gtactttcta 6300

tagtgaatag agttaggcag ggatattcac cattatcgtt tcagacccac ctcccaaccc 6360

cgaggggacc cgacaggccc gaaggaatag aagaagaagg tggagagaga gacagagaca 6420

gatccattcg attagtgaac ggatccttgg cacttatctg ggacgatctg cggagcctgt 6480

gcctcttcag ctaccaccgc ttgagagact tactcttgat tgtaacgagg attgtggaac 6540

ttctgggacg cagggggtgg gaagccctca aatattggtg gaatctccta caatattgga 6600

gtcaggagct aaagaatagt gctgttagct tgctcaatgc cacagccata gcagtagctg 6660

aggggacaga tagggttata gaagtagtac aaggagcttg tagagctatt cgccacatac 6720

ctagaagaat aagacagggc ttggaaagga ttttgctata agctcgaggc cgccccggtg 6780

accttcagac cttggcactg gaggtggccc ggcagaagcg cggcatcgtg gatcagtgct 6840

gcaccagcat ctgctctctc taccaactgg agaactactg caactaggcc caccactacc 6900

ctgtccaccc ctctgcaatg aataaaacct ttgaaagagc actacaagtt gtgtgtacat 6960

gcgtgcatgt gcatatgtgg tgcgggggga acatgagtgg ggctggctgg agtggcgatg 7020

ataagctgtc aaacatgaga attaattctt gaagacgaaa gggcctcgtg atacgcctat 7080

ttttataggt taatgtcatg ataataatgg tttcttagtc tagaattaat tccgtgtatt 7140

ctatagtgtc acctaaatcg tatgtgtatg atacataagg ttatgtatta attgtagccg 7200

cgttctaacg acaatatgta caagcctaat tgtgtagcat ctggcttact gaagcagacc 7260

ctatcatctc tctcgtaaac tgccgtcaga gtcggtttgg ttggacgaac cttctgagtt 7320

tctggtaacg ccgtcccgca cccggaaatg gtcagcgaac caatcagcag ggtcatcgct 7380

agccagatcc tctacgccgg acgcatcgtg gccggcatca ccggcgccac aggtgcggtt 7440

gctggcgcct atatcgccga catcaccgat ggggaagatc gggctcgcca cttcgggctc 7500

atgagcgctt gtttcggcgt gggtatggtg gcaggccccg tggccggggg actgttgggc 7560

gccatctcct tgcatgcacc attccttgcg gcggcggtgc tcaacggcct caacctacta 7620

ctgggctgct tcctaatgca ggagtcgcat aagggagagc gtcgaatggt gcactctcag 7680

tacaatctgc tctgatgccg catagttaag ccagccccga cacccgccaa cacccgctga 7740

cgcgccctga cgggcttgtc tgctcccggc atccgcttac agacaagctg tgaccgtctc 7800

cgggagctgc atgtgtcaga ggttttcacc gtcatcaccg aaacgcgcga gacgaaaggg 7860

cctcgtgata cgcctatttt tataggttaa tgtcatgata ataatggttt cttagacgtc 7920

aggtggcact tttcggggaa atgtgcgcgg aacccctatt tgtttatttt tctaaataca 7980

ttcaaatatg tatccgctca tgagacaata accctgataa atgcttcaat aatattgaaa 8040

aaggaagagt atgagtattc aacatttccg tgtcgccctt attccctttt ttgcggcatt 8100

ttgccttcct gtttttgctc acccagaaac gctggtgaaa gtaaaagatg ctgaagatca 8160

gttgggtgca cgagtgggtt acatcgaact ggatctcaac agcggtaaga tccttgagag 8220

ttttcgcccc gaagaacgtt ttccaatgat gagcactttt aaagttctgc tatgtggcgc 8280

ggtattatcc cgtattgacg ccgggcaaga gcaactcggt cgccgcatac actattctca 8340

gaatgacttg gttgagtact caccagtcac agaaaagcat cttacggatg gcatgacagt 8400

aagagaatta tgcagtgctg ccataaccat gagtgataac actgcggcca acttacttct 8460

gacaacgatc ggaggaccga aggagctaac cgcttttttg cacaacatgg gggatcatgt 8520

aactcgcctt gatcgttggg aaccggagct gaatgaagcc ataccaaacg acgagcgtga 8580

caccacgatg cctgtagcaa tggcaacaac gttgcgcaaa ctattaactg gcgaactact 8640

tactctagct tcccggcaac aattaataga ctggatggag gcggataaag ttgcaggacc 8700

acttctgcgc tcggcccttc cggctggctg gtttattgct gataaatctg gagccggtga 8760

gcgtgggtct cgcggtatca ttgcagcact ggggccagat ggtaagccct cccgtatcgt 8820

agttatctac acgacgggga gtcaggcaac tatggatgaa cgaaatagac agatcgctga 8880

gataggtgcc tcactgatta agcattggta actgtcagac caagtttact catatatact 8940

ttagattgat ttaaaacttc atttttaatt taaaaggatc taggtgaaga tcctttttga 9000

taatctcatg accaaaatcc cttaacgtga gttttcgttc cactgagcgt cagaccccgt 9060

agaaaagatc aaaggatctt cttgagatcc tttttttctg cgcgtaatct gctgcttgca 9120

aacaaaaaaa ccaccgctac cagcggtggt ttgtttgccg gatcaagagc taccaactct 9180

ttttccgaag gtaactggct tcagcagagc gcagatacca aatactgttc ttctagtgta 9240

gccgtagtta ggccaccact tcaagaactc tgtagcaccg cctacatacc tcgctctgct 9300

aatcctgtta ccagtggctg ctgccagtgg cgataagtcg tgtcttaccg ggttggactc 9360

aagacgatag ttaccggata aggcgcagcg gtcgggctga acggggggtt cgtgcacaca 9420

gcccagcttg gagcgaacga cctacaccga actgagatac ctacagcgtg agctatgaga 9480

aagcgccacg cttcccgaag ggagaaaggc ggacaggtat ccggtaagcg gcagggtcgg 9540

aacaggagag cgcacgaggg agcttccagg gggaaacgcc tggtatcttt atagtcctgt 9600

cgggtttcgc cacctctgac ttgagcgtcg atttttgtga tgctcgtcag gggggcggag 9660

cctatggaaa aacgccagca acgcggcctt tttacggttc ctggcctttt gctggccttt 9720

tgctcacatg ttctttcctg cgttatcccc tgattctgtg gataaccgta ttaccgcctt 9780

tgagtgagct gataccgctc gccgcagccg aacgaccgag cgcagcgagt cagtgagcga 9840

ggaagcggaa gagcgcccaa tacgcaaacc gcctctcccc gcgcgttggc cgattcatta 9900

atgcagctgt ggaatgtgtg tcagttaggg tgtggaaagt ccccaggctc cccagcaggc 9960

agaagtatgc aaagcatgca tctcaattag tcagcaacca ggtgtggaaa gtccccaggc 10020

tccccagcag gcagaagtat gcaaagcatg catctcaatt agtcagcaac catagtcccg 10080

cccctaactc cgcccatccc gcccctaact ccgcccagtt ccgcccattc tccgccccat 10140

ggctgactaa ttttttttat ttatgcagag gccgaggccg cctcggcctc tgagctattc 10200

cagaagtagt gaggaggctt ttttggaggc ctaggctttt gcaaaaagct tggacacaag 10260

acaggcttgc gagatatgtt tgagaatacc actttatccc gcgtcaggga gaggcagtgc 10320

gtaaaaagac gcggactcat gtgaaatact ggtttttagt gcgccagatc tctataatct 10380

cgcgcaacct attttcccct cgaacacttt ttaagccgta gataaacagg ctgggacact 10440

tcacatgagc gaaaaataca tcgtcacctg ggacatgttg cagatccatg cacgtaaact 10500

cgcaagccga ctgatgcctt ctgaacaatg gaaaggcatt attgccgtaa gccgtggcgg 10560

tctgtaccgg gtgcgttact ggcgcgtgaa ctgggtattc gtcatgtcga taccgtttgt 10620

atttccagct acgatcacga caaccagcgc gagcttaaag tgctgaaacg cgcagaaggc 10680

gatggcgaag gcttcatcgt tattgatgac ctggtggata ccggtggtac tgcggttgcg 10740

attcgtgaaa tgtatccaaa agcgcacttt gtcaccatct tcgcaaaacc ggctggtcgt 10800

ccgctggttg atgactatgt tgttgatatc ccgcaagata cctggattga acagccgtgg 10860

gatatgggcg tcgtattcgt cccgccaatc tccggtcgct aatcttttca acgcctggca 10920

ctgccgggcg ttgttctttt taacttcagg cgggttacaa tagtttccag taagtattct 10980

ggaggctgca tccatgacac aggcaaacct gagcgaaacc ctgttcaaac cccgctttaa 11040

acatcctgaa acctcgacgc tagtccgccg ctttaatcac ggcgcacaac cgcctgtgca 11100

gtcggccctt gatggtaaaa ccatccctca ctggtatcgc atgattaacc gtctgatgtg 11160

gatctggcgc ggcattgacc cacgcgaaat cctcgacgtc caggcacgta ttgtgatgag 11220

cgatgccgaa cgtaccgacg atgatttata cgatacggtg attggctacc gtggcggcaa 11280

ctggatttat gagtgggccc cggatctttg tgaaggaacc ttacttctgt ggtgtgacat 11340

aattggacaa actacctaca gagatttaaa gctctaaggt aaatataaaa tttttaaccc 11400

ggatctttgt gaaggaacct tacttctgtg gtgtgacata attggacaaa ctacctacag 11460

agatttaaag ctctaaggta aatataaaat ttttaagtgt ataatgtgtt aaactactga 11520

ttctaattgt ttgtgtattt tagattccaa cctatggaac tgatgaatgg gagcagtggt 11580

ggaatgcctt taatgaggaa aacctgtttt gctcagaaga aatgccatct agtgatgatg 11640

aggctactgc tgactctcaa cattctactc ctccaaaaaa gaagagaaag gtagaagacc 11700

ccaaggactt tccttcagaa ttgctaagtt ttttgagtca tgctgtgttt agtaatagaa 11760

ctcttgcttg ctttgctatt tacaccacaa aggaaaaagc tgcactgcta tacaagaaaa 11820

ttatggaaaa atattctgta acctttataa gtaggcataa cagttataat cataacatac 11880

tgttttttct tactccacac aggcatagag tgtctgctat taataactat gctcaaaaat 11940

tgtgtacctt tagcttttta atttgtaaag gggttaataa ggaatatttg atgtatagtg 12000

ccttgactag agatcataat cagccatacc acatttgtag aggttttact tgctttaaaa 12060

aacctcccac acctccccct gaacctgaaa cataaaatga atgcaattgt tgttgttggg 12120

ctgcaggaat taattcgagc tcgcccgaca 12150

<210> 4
<211> 5824
<212> DNA
<213> Artificial sequence

<220>
<223> SEQ ID NO: 4

<400> 4
ggatcccctg agggggcccc catgggctag aggatccggc ctcggcctct gcataaataa 60

aaaaaattag tcagccatga gcttggccca ttgcatacgt tgtatccata tcataatatg 120

tacatttata ttggctcatg tccaacatta ccgccatgtt gacattgatt attgactagt 180

tattaatagt aatcaattac ggggtcatta gttcatagcc catatatgga gttccgcgtt 240

acataactta cggtaaatgg cccgcctggc tgaccgccca acgacccccg cccattgacg 300

tcaataatga cgtatgttcc catagtaacg ccaataggga ctttccattg acgtcaatgg 360

gtggagtatt tacggtaaac tgcccacttg gcagtacatc aagtgtatca tatgccaagt 420

acgcccccta ttgacgtcaa tgacggtaaa tggcccgcct ggcattatgc ccagtacatg 480

accttatggg actttcctac ttggcagtac atctacgtat tagtcatcgc tattaccatg 540

gtgatgcggt tttggcagta catcaatggg cgtggatagc ggtttgactc acggggattt 600

ccaagtctcc accccattga cgtcaatggg agtttgtttt ggcaccaaaa tcaacgggac 660

tttccaaaat gtcgtaacaa ctccgcccca ttgacgcaaa tgggcggtag gcgtgtacgg 720

tgggaggtct atataagcag agctcgttta gtgaaccgtc agatcgcctg gagacgccat 780

ccacgctgtt ttgacctcca tagaagacac cgggaccgat ccagcctccc ctcgaagctt 840

acatgtggta ccgagctcgg atcctgagaa cttcagggtg agtctatggg acccttgatg 900

ttttctttcc ccttcttttc tatggttaag ttcatgtcat aggaagggga gaagtaacag 960

ggtacacata ttgaccaaat cagggtaatt ttgcatttgt aattttaaaa aatgctttct 1020

tcttttaata tacttttttg tttatcttat ttctaatact ttccctaatc tctttctttc 1080

agggcaataa tgatacaatg tatcatgcct ctttgcacca ttctaaagaa taacagtgat 1140

aatttctggg ttaaggcaat agcaatattt ctgcatataa atatttctgc atataaattg 1200

taactgatgt aagaggtttc atattgctaa tagcagctac aatccagcta ccattctgct 1260

tttattttat ggttgggata aggctggatt attctgagtc caagctaggc ccttttgcta 1320

atcatgttca tacctcttat cttcctccca cagctcctgg gcaacgtgct ggtctgtgtg 1380

ctggcccatc actttggcaa agcacgtgag atctgaattc aacagagatc gatctgtttc 1440

cttgacacta tgaagtgcct tttgtactta gcctttttat tcattggggt gaattgcaag 1500

ttcaccatag tttttccaca caaccaaaaa ggaaactgga aaaatgttcc ttctaattac 1560

cattattgcc cgtcaagctc agatttaaat tggcataatg acttaatagg cacagccata 1620

caagtcaaaa tgcccaagag tcacaaggct attcaagcag acggttggat gtgtcatgct 1680

tccaaatggg tcactacttg tgatttccgc tggtatggac cgaagtatat aacacagtcc 1740

atccgatcct tcactccatc tgtagaacaa tgcaaggaaa gcattgaaca aacgaaacaa 1800

ggaacttggc tgaatccagg cttccctcct caaagttgtg gatatgcaac tgtgacggat 1860

gccgaagcag tgattgtcca ggtgactcct caccatgtgc tggttgatga atacacagga 1920

gaatgggttg attcacagtt catcaacgga aaatgcagca attacatatg ccccactgtc 1980

cataactcta caacctggca ttctgactat aaggtcaaag ggctatgtga ttctaacctc 2040

atttccatgg acatcacctt cttctcagag gacggagagc tatcatccct gggaaaggag 2100

ggcacagggt tcagaagtaa ctactttgct tatgaaactg gaggcaaggc ctgcaaaatg 2160

caatactgca agcattgggg agtcagactc ccatcaggtg tctggttcga gatggctgat 2220

aaggatctct ttgctgcagc cagattccct gaatgcccag aagggtcaag tatctctgct 2280

ccatctcaga cctcagtgga tgtaagtcta attcaggacg ttgagaggat cttggattat 2340

tccctctgcc aagaaacctg gagcaaaatc agagcgggtc ttccaatctc tccagtggat 2400

ctcagctatc ttgctcctaa aaacccagga accggtcctg ctttcaccat aatcaatggt 2460

accctaaaat actttgagac cagatacatc agagtcgata ttgctgctcc aatcctctca 2520

agaatggtcg gaatgatcag tggaactacc acagaaaggg aactgtggga tgactgggca 2580

ccatatgaag acgtggaaat tggacccaat ggagttctga ggaccagttc aggatataag 2640
tttcctttat acatgattgg acatggtatg ttggactccg atcttcatct tagctcaaag 2700
gctcaggtgt tcgaacatcc tcacattcaa gacgctgctt cgcaacttcc tgatgatgag 2760
agtttatttt ttggtgatac tgggctatcc aaaaatccaa tcgagcttgt agaaggttgg 2820
ttcagtagtt ggaaaagctc tattgcctct tttttcttta tcatagggtt aatcattgga 2880
ctattcttgg ttctccgagt tggtatccat ctttgcatta aattaaagca caccaagaaa 2940
agacagattt atacagacat agagatgaac cgacttggaa agtaactcaa atcctgcaca 3000
acagattctt catgtttgga ccaaatcaac ttgtgatacc atgctcaaag aggcctcaat 3060
tatatttgag tttttaattt ttatggaatt caccccacca gtgcaggctg cctatcagaa 3120
agtggtggct ggtgtggcta atgccctggc ccacaagttt cactaagctc gcttccttgc 3180
tgtccaattt ctattaaagg ttccttggtt ccctaagtcc aactactaaa ctgggggata 3240
ttatgaaggg ccttgagcat ctggattctg cctaataaaa aacatttatt ttcattgcaa 3300
tgatgtattt aaattatttc tgaatatttt actaaaaagg gaatgtggga ggtcagtgca 3360
tttaaaacat aaagaaatga agagctagtt caaaccttgg gaaaatacac tatatcttaa 3420
actccatgaa agaaggtgag gctgcaaaca gctaatgcac attggcaaca gccctgatgc 3480
ctatgcctta ttcatccctc agaaaaggat tcaagtagag gcttgatttg gaggttaaag 3540
tttggctatg ctgtatttta cattacttat tgttttagct gtcctcatga atgtcttttc 3600
actacccatt tgcttatcct gcatctctca gccttgactc cactcagttc tcttgcttag 3660
agataccacc tttcccctga agtgttcctt ccatgtttta cggcgagatg gtttctcctc 3720
gcctggccac tcagccttag ttgtctctgt tgtcttatag aggtctactt gaagaaggaa 3780
aaacaggggg catggtttga ctgtcctgtg agcccttctt ccctgcctcc cccactcaca 3840
gtgacccgga atccctcgac atggcagtct agcactagtg cggccgcaga tctgcttcct 3900
cgctcactga ctcgctgcgc tcggtcgttc ggctgcggcg agcggtatca gctcactcaa 3960
aggcggtaat acggttatcc acagaatcag gggataacgc aggaaagaac atgtgagcaa 4020
aaggccagca aaaggccagg aaccgtaaaa aggccgcgtt gctggcgttt ttccataggc 4080
tccgcccccc tgacgagcat cacaaaaatc gacgctcaag tcagaggtgg cgaaacccga 4140
caggactata aagataccag gcgtttcccc ctggaagctc cctcgtgcgc tctcctgttc 4200
cgaccctgcc gcttaccgga tacctgtccg cctttctccc ttcgggaagc gtggcgcttt 4260
ctcaatgctc acgctgtagg tatctcagtt cggtgtaggt cgttcgctcc aagctgggct 4320
gtgtgcacga accccccgtt cagcccgacc gctgcgcctt atccggtaac tatcgtcttg 4380
agtccaaccc ggtaagacac gacttatcgc cactggcagc agccactggt aacaggatta 4440
gcagagcgag gtatgtaggc ggtgctacag agttcttgaa gtggtggcct aactacggct 4500

acactagaag gacagtattt ggtatctgcg ctctgctgaa gccagttacc ttcggaaaaa 4560

gagttggtag ctcttgatcc ggcaaacaaa ccaccgctgg tagcggtggt ttttttgttt 4620

gcaagcagca gattacgcgc agaaaaaaag gatctcaaga agatcctttg atcttttcta 4680

cggggtctga cgctcagtgg aacgaaaact cacgttaagg gattttggtc atgagattat 4740

caaaaaggat cttcacctag atccttttaa attaaaaatg aagttttaaa tcaatctaaa 4800

gtatatatga gtaaacttgg tctgacagtt accaatgctt aatcagtgag gcacctatct 4860

cagcgatctg tctatttcgt tcatccatag ttgcctgact ccccgtcgtg tagataacta 4920

cgatacggga gggcttacca tctggcccca gtgctgcaat gataccgcga gacccacgct 4980

caccggctcc agatttatca gcaataaacc agccagccgg aagggccgag cgcagaagtg 5040

gtcctgcaac tttatccgcc tccatccagt ctattaattg ttgccgggaa gctagagtaa 5100

gtagttcgcc agttaatagt ttgcgcaacg ttgttgccat tgctacaggc atcgtggtgt 5160

cacgctcgtc gtttggtatg gcttcattca gctccggttc ccaacgatca aggcgagtta 5220

catgatcccc catgttgtgc aaaaaagcgg ttagctcctt cggtcctccg atcgttgtca 5280

gaagtaagtt ggccgcagtg ttatcactca tggttatggc agcactgcat aattctctta 5340

ctgtcatgcc atccgtaaga tgcttttctg tgactggtga gtactcaacc aagtcattct 5400

gagaatagtg tatgcggcga ccgagttgct cttgcccggc gtcaatacgg gataataccg 5460

cgccacatag cagaacttta aaagtgctca tcattggaaa acgttcttcg gggcgaaaac 5520

tctcaaggat cttaccgctg ttgagatcca gttcgatgta acccactcgt gcacccaact 5580

gatcttcagc atcttttact ttcaccagcg tttctgggtg agcaaaaaca ggaaggcaaa 5640

atgccgcaaa aaagggaata agggcgacac ggaaatgttg aatactcata ctcttccttt 5700

ttcaatatta ttgaagcatt tatcagggtt attgtctcat gagcggatac atatttgaat 5760

gtatttagaa aaataaacaa ataggggttc cgcgcacatt tccccgaaaa gtgccacctg 5820

acgt 5824

<210> 5
<211> 6466
<212> DNA
<213> Artificial sequence

<220>
<223> SEQ ID NO: 5

<400> 5
gcggccgctc tagagagctt ggcccattgc atacgttgta tccatatcat aatatgtaca 60

tttatattgg ctcatgtcca acattaccgc catgttgaca ttgattattg actagttatt 120

aatagtaatc aattacgggg tcattagttc atagcccata tatggagttc cgcgttacat 180

aacttacggt aaatggcccg cctggctgac cgcccaacga cccccgccca ttgacgtcaa 240

taatgacgta tgttcccata gtaacgccaa tagggacttt ccattgacgt caatgggtgg 300

agtatttacg gtaaactgcc cacttggcag tacatcaagt gtatcatatg ccaagtacgc 360

cccctattga cgtcaatgac ggtaaatggc ccgcctggca ttatgcccag tacatgacct 420

tatgggactt tcctacttgg cagtacatct acgtattagt catcgctatt accatggtga 480

tgcggttttg gcagtacatc aatgggcgtg gatagcggtt tgactcacgg ggatttccaa 540

gtctccaccc cattgacgtc aatgggagtt tgttttggca ccaaaatcaa cgggactttc 600

caaaatgtcg taacaactcc gccccattga cgcaaatggg cggtaggcgt gtacggtggg 660

aggtctatat aagcagagct cgtttagtga accgtcagat cgcctggaga cgccatccac 720

gctgttttga cctccataga agacaccggg accgatccag cctccggtcg accgatcctg 780

agaacttcag ggtgagtttg gggacccttg attgttcttt ctttttcgct attgtaaaat 840

tcatgttata tggagggggc aaagttttca gggtgttgtt tagaatggga agatgtccct 900

tgtatcacca tggaccctca tgataatttt gtttctttca ctttctactc tgttgacaac 960

cattgtctcc tcttattttc ttttcatttt ctgtaacttt ttcgttaaac tttagcttgc 1020

atttgtaacg aatttttaaa ttcacttttg tttatttgtc agattgtaag tactttctct 1080

aatcactttt ttttcaaggc aatcagggta tattatattg tacttcagca cagttttaga 1140

gaacaattgt tataattaaa tgataaggta gaatatttct gcatataaat tctggctggc 1200

gtggaaatat tcttattggt agaaacaact acaccctggt catcatcctg cctttctctt 1260

tatggttaca atgatataca ctgtttgaga tgaggataaa atactctgag tccaaaccgg 1320

gcccctctgc taaccatgtt catgccttct tctctttcct acagctcctg ggcaacgtgc 1380

tggttgttgt gctgtctcat cattttggca aagaattcct cgacggatct gtcgaggtcg 1440

acggtatcga taagcttgat catgaatata ccttgctttg ctgtgatcct cagcttagct 1500

actacacatt ctctgggaga attccccttg tatacgattc ccgagaaaat agagaaatgg 1560

acccccatag acatgatcca tcttagttgc cctaataaca tgctgtctga ggaagaaggt 1620

tgcaatacag agtctccttt cacctacttc gagctcaaga gtggttacct agcccatcag 1680

aaggtcccag gatttacatg cactggggtt gtgaatgagg cagagacata cacaaacttt 1740

gtcggatatg tcaccaccac cttcaaaagg aagcacttta aacctacagt ggctgcttgt 1800

cgtgatgcct acaactggaa agtatcaggg gacccccgat atgaagaatc tctacacacc 1860

ccgtatcccg acagcagctg gttaaggact gtgaccacaa ccaaagaagc ccttcttata 1920

atatcgccaa gcattgtaga gatggacata tatggcagga cccttcactc tcccatgttc 1980

ccttcgggga aatgttccaa gctctatcct tctgtcccct cttgtacaac caaccatgat 2040

tacacattgt ggttgccaga agattctagt ctgagtttga tttgcgacat cttcacttcc 2100

agcagtggac agaaggccat gaatgggtct cgcatctgcg gattcaagga tgaaagggga 2160

ttttacagat ccttgaaggg atcctgtaag ctgacattgt gcgggaaacc tggaattagg 2220

ctgttcgacg gaacttgggt ctcttttaca aagccggacg ttcatgtgtg gtgcactccc 2280

aaccagttag tcaatataca taacgacaga ctagatgagg ttgaacatct gatcgtggac 2340

gatatcatca agaagagaga ggagtgttta gacacgctgg aaactatact tatgtctcaa 2400

tcagttagtt ttagacggtt gagccatttc agaaagttag ttccaggata tggaaaagct 2460

tacactattt tgaacggcag cttaatggaa acaaatgtct actacaaaag agttgacagg 2520

tgggcggaca ttttgccttc taggggatgt ctgaaagtcg gacaacagtg catggaccct 2580

gtcaaagggg tcctcttcaa cggaattatc aagggtccgg atggacaaat attgattcca 2640

gagatgcagt cagagcagct caaacagcat atggatctgt tgaaagcagc tatgtttcct 2700

ctccgtcatc ctttaatcaa cagagaggca gtcttcaaga aggatggaaa tgccgatgat 2760

tttgttgatc tccatatgcc tgatgttcaa aaatctgtgt cggatgtcga cctgggcctg 2820

cctcattggg ggttctggtt gttagtcggg gcaacagtag tagcctttgt ggtcttggcg 2880

tgcttgctcc gtgtatgttg taggagaatg agaaggagaa ggtcactgcg tgccactcag 2940

gatatccccc tcagcgttgc ccctgcccct gtccctcgtg ccaaagtggt gtcatcatgg 3000

gagtcttcta aaggctccca ggtacttgat tgcctttatc atactgcctg acacatagat 3060

cacaacctga ttacctgcct cggcacagat ccactcctca ggtgcaggct gcctatcaga 3120

aggtggtggc tggtgtggcc aatgccctgg ctcacaaata ccactgagat ctttttccct 3180

ctgccaaaaa ttatggggac atcatgaagc cccttgagca tctgacttct ggctaataaa 3240

ggaaatttat tttcattgca atagtgtgtt ggaatttttt gtgtctctca ctcggaagga 3300

catatgggag ggcaaatcat ttaaaacatc agaatgagta tttggtttag agtttggcaa 3360

catatgccca tatgctggct gccatgaaca aaggttggct ataaagaggt catcagtata 3420

tgaaacagcc ccctgctgtc cattccttat tccatagaaa agccttgact tgaggttaga 3480

ttttttttat attttgtttt gtgttatttt tttctttaac atccctaaaa ttttccttac 3540

atgttttact agccagattt ttcctcctct cctgactact cccagtcata gctgtccctc 3600

ttctcttatg gagatccctc gacggatcgg ccgcaattcg taatcatgtc atagctgttt 3660

cctgtgtgaa attgttatcc gctcacaatt ccacacaaca tacgagccgg aagcataaag 3720

tgtaaagcct ggggtgccta atgagtgagc taactcacat taattgcgtt gcgctcactg 3780

cccgctttcc agtcgggaaa cctgtcgtgc cagctgcatt aatgaatcgg ccaacgcgcg 3840

gggagaggcg gtttgcgtat tgggcgctct tccgcttcct cgctcactga ctcgctgcgc 3900

tcggtcgttc ggctgcggcg agcggtatca gctcactcaa aggcggtaat acggttatcc 3960

acagaatcag gggataacgc aggaaagaac atgtgagcaa aaggccagca aaaggccagg 4020

aaccgtaaaa aggccgcgtt gctggcgttt ttccataggc tccgcccccc tgacgagcat 4080

cacaaaaatc gacgctcaag tcagaggtgg cgaaacccga caggactata aagataccag 4140

gcgtttcccc ctggaagctc cctcgtgcgc tctcctgttc cgaccctgcc gcttaccgga 4200

tacctgtccg cctttctccc ttcgggaagc gtggcgcttt ctcatagctc acgctgtagg 4260

tatctcagtt cggtgtaggt cgttcgctcc aagctgggct gtgtgcacga accccccgtt 4320

cagcccgacc gctgcgcctt atccggtaac tatcgtcttg agtccaaccc ggtaagacac 4380

gacttatcgc cactggcagc agccactggt aacaggatta gcagagcgag gtatgtaggc 4440

ggtgctacag agttcttgaa gtggtggcct aactacggct acactagaag aacagtattt 4500

ggtatctgcg ctctgctgaa gccagttacc ttcggaaaaa gagttggtag ctcttgatcc 4560

ggcaaacaaa ccaccgctgg tagcggtggt ttttttgttt gcaagcagca gattacgcgc 4620

agaaaaaaag gatctcaaga agatcctttg atcttttcta cggggtctga cgctcagtgg 4680

aacgaaaact cacgttaagg gattttggtc atgagattat caaaaaggat cttcacctag 4740

atccttttaa attaaaaatg aagttttaaa tcaatctaaa gtatatatga gtaaacttgg 4800

tctgacagtt accaatgctt aatcagtgag gcacctatct cagcgatctg tctatttcgt 4860

tcatccatag ttgcctgact ccccgtcgtg tagataacta cgatacggga gggcttacca 4920

tctggcccca gtgctgcaat gataccgcga gacccacgct caccggctcc agatttatca 4980

gcaataaacc agccagccgg aagggccgag cgcagaagtg gtcctgcaac tttatccgcc 5040

tccatccagt ctattaattg ttgccgggaa gctagagtaa gtagttcgcc agttaatagt 5100

ttgcgcaacg ttgttgccat tgctacaggc atcgtggtgt cacgctcgtc gtttggtatg 5160

gcttcattca gctccggttc ccaacgatca aggcgagtta catgatcccc catgttgtgc 5220

aaaaaagcgg ttagctcctt cggtcctccg atcgttgtca gaagtaagtt ggccgcagtg 5280

ttatcactca tggttatggc agcactgcat aattctctta ctgtcatgcc atccgtaaga 5340

tgcttttctg tgactggtga gtactcaacc aagtcattct gagaatagtg tatgcggcga 5400

ccgagttgct cttgcccggc gtcaatacgg gataataccg cgccacatag cagaacttta 5460

aaagtgctca tcattggaaa acgttcttcg gggcgaaaac tctcaaggat cttaccgctg 5520

ttgagatcca gttcgatgta acccactcgt gcacccaact gatcttcagc atcttttact 5580

ttcaccagcg tttctgggtg agcaaaaaca ggaaggcaaa atgccgcaaa aaagggaata 5640

agggcgacac ggaaatgttg aatactcata ctcttccttt ttcaatatta ttgaagcatt 5700

tatcagggtt attgtctcat gagcggatac atatttgaat gtatttagaa aaataaacaa 5760

ataggggttc cgcgcacatt tccccgaaaa gtgccacctg acgtctaaga aaccattatt 5820

atcatgacat taacctataa aaataggcgt atcacgaggc cctttcgtct cgcgcgtttc 5880

ggtgatgacg gtgaaaacct ctgacacatg cagctcccgg agacggtcac agcttgtctg 5940

taagcggatg ccgggagcag acaagcccgt cagggcgcgt cagcgggtgt tggcgggtgt 6000

cggggctggc ttaactatgc ggcatcagag cagattgtac tgagagtgca ccatatgcgg 6060

tgtgaaatac cgcacagatg cgtaaggaga aaataccgca tcaggcgcca ttcgccattc 6120

ggaaagccgg cgaacgtggc gagaaaggaa gggaagaaag cgaaaggagc gggcgctagg 6180

gcgctggcaa gtgtagcggt cacgctgcgc gtaaccacca cacccgccgc gcttaatgcg 6240

ccgctacagg gcgcgtccca ttcgccattc aggctgcgca actgttggga agggcgatcg 6300

gtgcgggcct cttcgctatt acgccagctg gcgaaagggg gatgtgctgc aaggcgatta 6360

agttgggtaa cgccagggtt ttcccagtca cgacgttgta aaacgacggc cagtgagcgc 6420

gcgtaatacg actcactata gggcgaattg gagctccacc gcggtg 6466

<210> 6
<211> 11346
<212> DNA
<213> Artificial sequence

<220>
<223> SEQ ID NO: 6

<400> 6
   gggtcaccat ctagatggcc atatgatcga tacgtactag tctcgagctc aagcttcgaa 60
   ttcccgataa tcaacctctg gattacaaaa tttgtgaaag attgactggt attcttaact 120
   atgttgctcc ttttacgcta tgtggatacg ctgctttaat gcctttgtat catgctattg 180
   cttcccgtat ggctttcatt ttctcctcct tgtataaatc ctggttgctg tctctttatg 240
   aggagttgtg gcccgttgtc aggcaacgtg gcgtggtgtg cactgtgttt gctgacgcaa 300
   cccccactgg ttggggcatt gccaccacct gtcagctcct ttccgggact ttcgctttcc 360
   ccctccctat tgccacggcg gaactcatcg ccgcctgcct tgcccgctgc tggacagggg 420
   ctcggctgtt gggcactgac aattccgtgg tgttgtcggg gaagctgacg tcctttccat 480
   ggctgctcgc ctgtgttgcc acctggattc tgcgcgggac gtccttctgc tacgtccctt 540
   cggccctcaa tccagcggac cttccttccc gcggcctgct gccggctctg cggcctcttc 600
   cgcgtcttcg ccttcgccct cagacgagtc ggatctccct ttgggccgcc tccccgcatc 660
   gggaattctg cagtcgacgg tacctttaag accaatgact tacaaggcag ctgtagatct 720
   tagccacttt ttaaaagaaa aggggggact ggaagggcta attcactccc aacgaagaca 780
   aaatcgtcga gagatgctgc atataagcag ctgctttttg cttgtactgg gtctctctgg 840
   ttagaccaga tctgagcctg ggagctctct ggctaactag ggaacccact gcttaagcct 900
   caataaagct tgccttgagt gcttcaagta gtgtgtgccc gtctgttgtg tgactctggt 960
   aactagagat ccctcagacc cttttagtca gtgtggaaaa tctctagcag tagtagttca 1020
   tgtcatctta ttattcagta tttataactt gcaaagaaat gaatatcaga gagtgagagg 1080
   ccttgacatt ataatagatt tagcaggaat tgaactagga gtggagcaca caggcaaagc 1140
   tgcagaagta cttggaagaa gccaccagag atactcacga ttctgcacat acctggctaa 1200
   tcccagatcc taaggattac attaagttta ctaacattta tataatgatt tatagtttaa 1260
   agtataaact tatctaattt actattctga cagatattaa ttaatcctca aatatcataa 1320
   gagatgatta ctattatccc catttaacac aagaggaaac tgagagggaa agatgttgaa 1380
   gtaattttcc cacaattaca gcatccgtta gttacgactc tatgatcttc tgacacaaat 1440
   tccatttact cctcacccta tgactcagtc gaatatatca aagttatgga cattatgcta 1500
   agtaacaaat taccctttta tatagtaaat actgagtaga ttgagagaag aaattgtttg 1560
   caaacctgaa tagcttcaag aagaagagaa gtgaggataa gaataacagt tgtcatttaa 1620
   caagttttaa caagtaactt ggttagaaag ggattcaaat gcataaagca agggataaat 1680
   ttttctggca acaagactat acaatataac cttaaatatg acttcaaata attgttggaa 1740
   cttgataaaa ctaattaaat attattgaag attatcaata ttataaatgt aatttacttt 1800
   taaaaaggga acatagaaat gtgtatcatt agagtagaaa acaatcctta ttatcacaat 1860
   ttgtcaaaac aagtttgtta ttaacacaag tagaatactg cattcaatta agttgactgc 1920
   agattttgtg ttttgttaaa attagaaaga gataacaaca atttgaatta ttgaaagtaa 1980
   catgtaaata gttctacata cgttcttttg acatcttgtt caatcattga tcgaagttct 2040
   ttatcttgga agaatttgtt ccaaagactc tgaaataagg aaaacaatct attatatagt 2100
   ctcacacctt tgttttactt ttagtgattt caatttaata atgtaaatgg ttaaaattta 2160
   ttcttctctg agatcatttc acattgcaga tagaaaacct gagactgggg taatttttat 2220
   taaaatctaa tttaatctca gaaacacatc tttattctaa catcaatttt tccagtttga 2280
   tattatcata taaagtcagc cttcctcatc tgcaggttcc acaacaaaaa tccaaccaac 2340
   tgtggatcaa aaatattggg aaaaaattaa aaatagcaat acaacaataa aaaaatacaa 2400
   atcagaaaaa cagcacagta taacaacttt atttagcatt tacaatctat taggtattat 2460
   aagtaatcta gccagatcct ctacgccgga cgcatcgtgg ccggcatcac cggcgccaca 2520
   ggtgcggttg ctggcgccta tatcgccgac atcaccgatg gggaagatcg ggctcgccac 2580
   ttcgggctca tgagcgcttg tttcggcgtg ggtatggtgg caggccccgt ggccggggga 2640
   ctgttgggcg ccatctcctt gcatgcacca ttccttgcgg cggcggtgct caacggcctc 2700
   aacctactac tgggctgctt cctaatgcag gagtcgcata agggagagcg tcgaatggtg 2760
   cactctcagt acaatctgct ctgatgccgc atagttaagc cagccccgac acccgccaac 2820
   acccgctgac gcgccctgac gggcttgtct gctcccggca tccgcttaca gacaagctgt 2880
   gaccgtctcc gggagctgca tgtgtcagag gttttcaccg tcatcaccga aacgcgcgag 2940
   acgaaagggc ctcgtgatac gcctattttt ataggttaat gtcatgataa taatggtttc 3000
   ttagacgtca ggtggcactt ttcggggaaa tgtgcgcgga acccctattt gtttattttt 3060
   ctaaatacat tcaaatatgt atccgctcat gagacaataa ccctgataaa tgcttcaata 3120
   atattgaaaa aggaagagta tgagtattca acatttccgt gtcgccctta ttcccttttt 3180
   tgcggcattt tgccttcctg tttttgctca cccagaaacg ctggtgaaag taaaagatgc 3240
   tgaagatcag ttgggtgcac gagtgggtta catcgaactg gatctcaaca gcggtaagat 3300
   ccttgagagt tttcgccccg aagaacgttt tccaatgatg agcactttta aagttctgct 3360
   atgtggcgcg gtattatccc gtattgacgc cgggcaagag caactcggtc gccgcataca 3420
   ctattctcag aatgacttgg ttgagtactc accagtcaca gaaaagcatc ttacggatgg 3480
   catgacagta agagaattat gcagtgctgc cataaccatg agtgataaca ctgcggccaa 3540
   cttacttctg acaacgatcg gaggaccgaa ggagctaacc gcttttttgc acaacatggg 3600
   ggatcatgta actcgccttg atcgttggga accggagctg aatgaagcca taccaaacga 3660
   cgagcgtgac accacgatgc ctgtagcaat ggcaacaacg ttgcgcaaac tattaactgg 3720
   cgaactactt actctagctt cccggcaaca attaatagac tggatggagg cggataaagt 3780
   tgcaggacca cttctgcgct cggcccttcc ggctggctgg tttattgctg ataaatctgg 3840
   agccggtgag cgtgggtctc gcggtatcat tgcagcactg gggccagatg gtaagccctc 3900
   ccgtatcgta gttatctaca cgacggggag tcaggcaact atggatgaac gaaatagaca 3960
   gatcgctgag ataggtgcct cactgattaa gcattggtaa ctgtcagacc aagtttactc 4020
   atatatactt tagattgatt taaaacttca tttttaattt aaaaggatct aggtgaagat 4080
   cctttttgat aatctcatga ccaaaatccc ttaacgtgag ttttcgttcc actgagcgtc 4140
   agaccccgta gaaaagatca aaggatcttc ttgagatcct ttttttctgc gcgtaatctg 4200
   ctgcttgcaa acaaaaaaac caccgctacc agcggtggtt tgtttgccgg atcaagagct 4260
   accaactctt tttccgaagg taactggctt cagcagagcg cagataccaa atactgtcct 4320
   tctagtgtag ccgtagttag gccaccactt caagaactct gtagcaccgc ctacatacct 4380
   cgctctgcta atcctgttac cagtggctgc tgccagtggc gataagtcgt gtcttaccgg 4440
   gttggactca agacgatagt taccggataa ggcgcagcgg tcgggctgaa cggggggttc 4500
   gtgcacacag cccagcttgg agcgaacgac ctacaccgaa ctgagatacc tacagcgtga 4560
   gcattgagaa agcgccacgc ttcccgaagg gagaaaggcg gacaggtatc cggtaagcgg 4620
   cagggtcgga acaggagagc gcacgaggga gcttccaggg ggaaacgcct ggtatcttta 4680
   tagtcctgtc gggtttcgcc acctctgact tgagcgtcga tttttgtgat gctcgtcagg 4740
   ggggcggagc ctatggaaaa acgccagcaa cgcggccttt ttacggttcc tggccttttg 4800
   ctggcctttt gctcacatgt tctttcctgc gttatcccct gattctgtgg ataaccgtat 4860
   taccgccttt gagtgagctg ataccgctcg ccgcagccga acgaccgagc gcagcgagtc 4920
   agtgagcgag gaagcggaag agcgcccaat acgcaaaccg cctctccccg cgcgttggcc 4980
   gattcattaa tgcagctgtg gaatgtgtgt cagttagggt gtggaaagtc cccaggctcc 5040
   ccagcaggca gaagtatgca aagcatgcat ctcaattagt cagcaaccag gtgtggaaag 5100
   tccccaggct ccccagcagg cagaagtatg caaagcatgc atctcaatta gtcagcaacc 5160
   atagtcccgc ccctaactcc gcccatcccg cccctaactc cgcccagttc cgcccattct 5220
   ccgccccatg gctgactaat tttttttatt tatgcagagg ccgaggccgc ctcggcctct 5280
   gagctattcc agaagtagtg aggaggcttt tttggaggcc taggcttttg caaaaagctt 5340
   ggacacaaga caggcttgcg agatatgttt gagaatacca ctttatcccg cgtcagggag 5400
   aggcagtgcg taaaaagacg cggactcatg tgaaatactg gtttttagtg cgccagatct 5460
   ctataatctc gcgcaaccta ttttcccctc gaacactttt taagccgtag ataaacaggc 5520
   tgggacactt cacatgagcg aaaaatacat cgtcacctgg gacatgttgc agatccatgc 5580
   acgtaaactc gcaagccgac tgatgccttc tgaacaatgg aaaggcatta ttgccgtaag 5640
   ccgtggcggt ctgtaccggg tgcgttactg gcgcgtgaac tgggtattcg tcatgtcgat 5700
   accgtttgta tttccagcta cgatcacgac aaccagcgcg agcttaaagt gctgaaacgc 5760
   gcagaaggcg atggcgaagg cttcatcgtt attgatgacc tggtggatac cggtggtact 5820
   gcggttgcga ttcgtgaaat gtatccaaaa gcgcactttg tcaccatctt cgcaaaaccg 5880
   gctggtcgtc cgctggttga tgactatgtt gttgatatcc cgcaagatac ctggattgaa 5940
   cagccgtggg atatgggcgt cgtattcgtc ccgccaatct ccggtcgcta atcttttcaa 6000
   cgcctggcac tgccgggcgt tgttcttttt aacttcaggc gggttacaat agtttccagt 6060
   aagtattctg gaggctgcat ccatgacaca ggcaaacctg agcgaaaccc tgttcaaacc 6120
   ccgctttaaa catcctgaaa cctcgacgct agtccgccgc tttaatcacg gcgcacaacc 6180
   gcctgtgcag tcggcccttg atggtaaaac catccctcac tggtatcgca tgattaaccg 6240
   tctgatgtgg atctggcgcg gcattgaccc acgcgaaatc ctcgacgtcc aggcacgtat 6300
   tgtgatgagc gatgccgaac gtaccgacga tgatttatac gatacggtga ttggctaccg 6360
   tggcggcaac tggatttatg agtgggcccc ggatctttgt gaaggaacct tacttctgtg 6420
   gtgtgacata attggacaaa ctacctacag agatttaaag ctctaaggta aatataaaat 6480
   ttttaagtgt ataatgtgtt aaactactga ttctaattgt ttgtgtattt tagattccaa 6540
   cctatggaac tgatgaatgg gagcagtggt ggaatgcctt taatgaggaa aacctgtttt 6600
   gctcagaaga aatgccatct agtgatgatg aggctactgc tgactctcaa cattctactc 6660
   ctccaaaaaa gaagagaaag gtagaagacc ccaaggactt tccttcagaa ttgctaagtt 6720
   ttttgagtca tgctgtgttt agtaatagaa ctcttgcttg ctttgctatt tacaccacaa 6780
   aggaaaaagc tgcactgcta tacaagaaaa ttatggaaaa atattctgta acctttataa 6840
   gtaggcataa cagttataat cataacatac tgttttttct tactccacac aggcatagag 6900
   tgtctgctat taataactat gctcaaaaat tgtgtacctt tagcttttta atttgtaaag 6960
   gggttaataa ggaatatttg atgtatagtg ccttgactag agatcataat cagccatacc 7020
   acatttgtag aggttttact tgctttaaaa aacctcccac acctccccct gaacctgaaa 7080
   cataaaatga atgcaattgt tgttgttaac ttgtttattg cagcttataa tggttacaaa 7140
   taaagcaata gcatcacaaa tttcacaaat aaagcatttt tttcactgca ttctagttgt 7200
   ggtttgtcca aactcatcaa tgtatcttat catgtctgga tcaactggat aactcaagct 7260
   aaccaaaatc atcccaaact tcccacccca taccctatta ccactgccaa ttacctagtg 7320
   gtttcattta ctctaaacct gtgattcctc tgaattattt tcattttaaa gaaattgtat 7380
   ttgttaaata tgtactacaa acttagtagt tggaagggct aattcactcc caacgaagac 7440
   aagatatcct tgatctgtgg atctaccaca cacaaggcta cttccctgat tagcagaact 7500
   acacaccagg gccagggatc agatatccac tgacctttgg atggtgctac aagctagtac 7560
   cagttgagcc agagaagtta gaagaagcca acaaaggaga gaacaccagc ttgttacaac 7620
   ctgtgagcct gcatgggatg gatgacccgg agagagaagt gttagagtgg aggtttgaca 7680
   gccgcctagc atttcatcac ggtggcccga gagctgcatc cggagtactt caagaactgc 7740
   tgatatcgag cttgctacaa gggactttcc gctggggact tttccaggga ggcgtggcct 7800
   gggcgggact ggggagtggc gagccctcag atcctgcata taagcagctg ctttttgcct 7860
   gtactgggtc tctctggtta gaccagatct gagcctggga gctctctggc taactaggga 7920
   acccactgct taagcctcaa taaagcttgc cttgagtgct tcaagtagtg tgtgcccgtc 7980
   tgttgtgtga ctctggtaac tagagatccc tcagaccctt ttagtcagtg tggaaaatct 8040
   ctagcagttg aaagcgaaag ggaaaccaga ggagctctct cgacgcagga ctcggcttgc 8100
   tgaagcgcgc acggcaagag gcgaggggcg gcgactggtg agtacgccaa aaattttgac 8160
   tagcggaggc tagaaggaga gagatgggtg cgagagcgtc agtattaagc gggggagaat 8220
   tagatcgcga tgggaaaaaa ttcggttaag gccaggggga aagaaaaaat ataaattaaa 8280
   acatatagta tgggcaagca gggagctaga acgattcgca gttaatcctg gcctgttaga 8340
   aacatcagaa ggctgtagac aaatactggg acagctacaa ccatcccttc agacaggatc 8400
   agaagaactt agatcattat ataatacagt agcaaccctc tattgtgtgc atcaaaggat 8460
   agagataaaa gacaccaagg aagctttaga caagatagag gaagagcaaa acaaaagtaa 8520
   gaccaccgca cagcaagcgg ccgctgatct tcagacctgg aggaggagat atgagggaca 8580
   attggagaag tgaattatat aaatataaag tagtaaaaat tgaaccatta ggagtagcac 8640
   ccaccaaggc aaagagaaga gtggtgcaga gagaaaaaag agcagtggga ataggagctt 8700
   tgttccttgg gttcttggga gcagcaggaa gcactatggg cgcagcgtca atgacgctga 8760
   cggtacaggc cagacaatta ttgtctggta tagtgcagca gcagaacaat ttgctgaggg 8820
   ctattgaggc gcaacagcat ctgttgcaac tcacagtctg gggcatcaag cagctccagg 8880
   caagaatcct ggctgtggaa agatacctaa aggatcaaca gctcctgggg atttggggtt 8940
   gctctggaaa actcatttgc accactgctg tgccttggaa tgctagttgg agtaataaat 9000
   ctctggaaca gatttggaat cacacgacct ggatggagtg ggacagagaa attaacaatt 9060
   acacaagctt aatacactcc ttaattgaag aatcgcaaaa ccagcaagaa aagaatgaac 9120
   aagaattatt ggaattagat aaatgggcaa gtttgtggaa ttggtttaac ataacaaatt 9180
   ggctgtggta tataaaatta ttcataatga tagtaggagg cttggtaggt ttaagaatag 9240
   tttttgctgt actttctata gtgaatagag ttaggcaggg atattcacca ttatcgtttc 9300
   agacccacct cccaaccccg aggggacccg acaggcccga aggaatagaa gaagaaggtg 9360
   gagagagaga cagagacaga tccattcgat tagtgaacgg atctcgacgg tatcgccgaa 9420
   ttcacaaatg gcagtattca tccacaattt taaaagaaaa ggggggattg gggggtacag 9480
   tgcaggggaa agaatagtag acataatagc aacagacata caaactaaag aattacaaaa 9540
   acaaattaca aaaattcaaa attttcgggt ttattacagg gacagcagag atccactttg 9600
   gctgatacgc ggatctacgc gtggatccgc ggcccgggaa ttcgatctaa gctagctagg 9660
   ccgccaccat ggaagacgcc aaaaacataa agaaaggccc ggcgccattc tatccgctgg 9720
   aagatggaac cgctggagag caactgcata aggctatgaa gagatacgcc ctggttcctg 9780
   gaacaattgc ttttacagat gcacatatcg aggtggacat cacttacgct gagtacttcg 9840
   aaatgtccgt tcggttggca gaagctatga aacgatatgg gctgaataca aatcacagaa 9900
   tcgtcgtatg cagtgaaaac tctcttcaat tctttatgcc ggtgttgggc gcgttattta 9960
   tcggagttgc agttgcgccc gcgaacgaca tttataatga acgtgaattg ctcaacagta 10020
   tgggcatttc gcagcctacc gtggtgttcg tttccaaaaa ggggttgcaa aaaattttga 10080
   acgtgcaaaa aaagctccca atcatccaaa aaattattat catggattct aaaacggatt 10140
   accagggatt tcagtcgatg tacacgttcg tcacatctca tctacctccc ggttttaatg 10200
   aatacgattt tgtgccagag tccttcgata gggacaagac aattgcactg atcatgaact 10260
   cctctggatc tactggtctg cctaaaggtg tcgctctgcc tcatagaact gcctgcgtga 10320
   gattctcgca tgccagagat cctatttttg gcaatcaaat cattccggat actgcgattt 10380
   taagtgttgt tccattccat cacggttttg gaatgtttac tacactcgga tatttgatat 10440
   gtggatttcg agtcgtctta atgtatagat ttgaagaaga gctgtttctg aggagccttc 10500
   aggattacaa gattcaaagt gcgctgctgg tgccaaccct attctccttc ttcgccaaaa 10560
   gcactctgat tgacaaatac gatttatcta atttacacga aattgcttct ggtggcgctc 10620
   ccctctctaa ggaagtcggg gaagcggttg ccaagaggtt ccatctgcca ggtatcaggc 10680
   aaggatatgg gctcactgag actacatcag ctattctgat tacacccgag ggggatgata 10740
   aaccgggcgc ggtcggtaaa gttgttccat tttttgaagc gaaggttgtg gatctggata 10800
   ccgggaaaac gctgggcgtt aatcaaagag gcgaactgtg tgtgagaggt cctatgatta 10860
   tgtccggtta tgtaaacaat ccggaagcga ccaacgcctt gattgacaag gatggatggc 10920
   tacattctgg agacatagct tactgggacg aagacgaaca cttcttcatc gttgaccgcc 10980
   tgaagtctct gattaagtac aaaggctatc aggtggctcc cgctgaattg gaatccatct 11040
   tgctccaaca ccccaacatc ttcgacgcag gtgtcgcagg tcttcccgac gatgacgccg 11100
   gtgaacttcc cgccgccgtt gttgttttgg agcacggaaa gacgatgacg gaaaaagaga 11160
   tcgtggatta cgtcgccagt caagtaacaa ccgcgaaaaa gttgcgcgga ggagttgtgt 11220
   ttgtggacga agtaccgaaa ggtcttaccg gaaaactcga cgcaagaaaa atcagagaga 11280
   tcctcataaa ggccaagaag ggcggaaaga tcgccgtgta attcctgcag gctcacgtgc 11340
   gcatca 11346

<210> 7
<211> 13784
<212> DNA
<213> Artificial sequence

<220>
<223> SEQ ID NO: 7

<400> 7
cagttagaga aagaacccat aataggagca gaaactttct atgtagatgg ggcagccaat 60

agggaaacta aattaggaaa agcaggatat gtaactgaca gaggaagaca aaaagttgtc 120

cccctaacgg acacaacaaa tcagaagact gagttacaag caattcatct agctttgcag 180

gattcgggat tagaagtaaa catagtgaca gactcacaat atgcattggg aatcattcaa 240

gcacaaccag ataagagtga atcagagtta gtcagtcaaa taatagagca gttaataaaa 300

aaggaaaaag tctacctggc atgggtacca gcacacaaag gaattggagg aaatgaacaa 360

gtagataaat tggtcagtgc tggaatcagg aaagtactat ttttagatgg aatagataag 420

gcccaagaag aacatgagaa atatcacagt aattggagag caatggctag tgattttaac 480

ctaccacctg tagtagcaaa agaaatagta gccagctgtg ataaatgtca gctaaaaggg 540

gaagccatgc atggacaagt agactgtagc ccaggaatat ggcagctaga ttgtacacat 600

ttagaaggaa aagttatctt ggtagcagtt catgtagcca gtggatatat agaagcagaa 660

gtaattccag cagagacagg gcaagaaaca gcatacttcc tcttaaaatt agcaggaaga 720

tggccagtaa aaacagtaca tacagacaat ggcagcaatt tcaccagtac tacagttaag 780

gccgcctgtt ggtgggcggg gatcaagcag gaatttggca ttccctacaa tccccaaagt 840

caaggagtaa tagaatctat gaataaagaa ttaaagaaaa ttataggaca ggtaagagat 900

caggctgaac atcttaagac agcagtacaa atggcagtat tcatccacaa ttttaaaaga 960

aaagggggga ttggggggta cagtgcaggg gaaagaatag tagacataat agcaacagac 1020

atacaaacta aagaattaca aaaacaaatt acaaaaattc aaaattttcg ggtttattac 1080

agggacagca gagatccagt ttggaaagga ccagcaaagc tcctctggaa aggtgaaggg 1140

gcagtagtaa tacaagataa tagtgacata aaagtagtgc caagaagaaa agcaaagatc 1200

atcagggatt atggaaaaca gatggcaggt gatgattgtg tggcaagtag acaggatgag 1260

gattaacaca tggaattctg caacaactgc tgtttatcca tttcagaatt gggtgtcgac 1320

atagcagaat aggcgttact cgacagagga gagcaagaaa tggagccagt agatcctaga 1380

ctagagccct ggaagcatcc aggaagtcag cctaaaactg cttgtaccaa ttgctattgt 1440

aaaaagtgtt gctttcattg ccaagtttgt ttcatgacaa aagccttagg catctcctat 1500

ggcaggaaga agcggagaca gcgacgaaga gctcatcaga acagtcagac tcatcaagct 1560

tctctatcaa agcagtaagt agtacatgta atgcaaccta taatagtagc aatagtagca 1620

ttagtagtag caataataat agcaatagtt gtgtggtcca tagtaatcat agaatatagg 1680

aaaatggccg ctgatcttca gacctggagg aggagatatg agggacaatt ggagaagtga 1740

attatataaa tataaagtag taaaaattga accattagga gtagcaccca ccaaggcaaa 1800

gagaagagtg gtgcagagag aaaaaagagc agtgggaata ggagctttgt tccttgggtt 1860

cttgggagca gcaggaagca ctatgggcgc agcgtcaatg acgctgacgg tacaggccag 1920

acaattattg tctggtatag tgcagcagca gaacaatttg ctgagggcta ttgaggcgca 1980

acagcatctg ttgcaactca cagtctgggg catcaagcag ctccaggcaa gaatcctggc 2040

tgtggaaaga tacctaaagg atcaacagct cctggggatt tggggttgct ctggaaaact 2100

catttgcacc actgctgtgc cttggaatgc tagttggagt aataaatctc tggaacagat 2160

ttggaatcac acgacctgga tggagtggga cagagaaatt aacaattaca caagcttaat 2220

acactcctta attgaagaat cgcaaaacca gcaagaaaag aatgaacaag aattattgga 2280

attagataaa tgggcaagtt tgtggaattg gtttaacata acaaattggc tgtggtatat 2340

aaaattattc ataatgatag taggaggctt ggtaggttta agaatagttt ttgctgtact 2400

ttctatagtg aatagagtta ggcagggata ttcaccatta tcgtttcaga cccacctccc 2460

aaccccgagg ggacccgaca ggcccgaagg aatagaagaa gaaggtggag agagagacag 2520

agacagatcc attcgattag tgaacggatc cttggcactt atctgggacg atctgcggag 2580

cctgtgcctc ttcagctacc accgcttgag agacttactc ttgattgtaa cgaggattgt 2640

ggaacttctg ggacgcaggg ggtgggaagc cctcaaatat tggtggaatc tcctacaata 2700

ttggagtcag gagctaaaga atagtgctgt tagcttgctc aatgccacag ccatagcagt 2760

agctgagggg acagataggg ttatagaagt agtacaagga gcttgtagag ctattcgcca 2820

catacctaga agaataagac agggcttgga aaggattttg ctataagctc gaggccgccc 2880

cgcccctctc cctccccccc ccctaacgtt actggccgaa gccgcttgga ataaggccgg 2940

tgtgcgtttg tctatatgtt attttccacc atattgccgt cttttggcaa tgtgagggcc 3000

cggaaacctg gccctgtctt cttgacgagc attcctaggg gtctttcccc tctcgccaaa 3060

ggaatgcaag gtctgttgaa tgtcgtgaag gaagcagttc ctctggaagc ttcttgaaga 3120

caaacaacgt ctgtagcgac cctttgcagg cagcggaacc ccccacctgg cgacaggtgc 3180

ctctgcggcc aaaagccacg tgtataagat acacctgcaa aggcggcaca accccagtgc 3240

cacgttgtga gttggatagt tgtggaaaga gtcaaatggc tctcctcaag cgtattcaac 3300

aaggggctga aggatgccca gaaggtaccc cattgtatgg gatctgatct ggggcctcgg 3360

tgcacatgct ttacatgtgt ttagtcgagg ttaaaaaaac gtctaggccc cccgaaccac 3420

ggggacgtgg ttttcctttg aaaaacacga tgataagctt gccacaaccc gtaccaaaga 3480

tggatagatc cggaaagcct gaactcaccg cgacgtctgt cgagaagttt ctgatcgaaa 3540

agttcgacag cgtctccgac ctgatgcagc tctcggaggg cgaagaatct cgtgctttca 3600

gcttcgatgt aggagggcgt ggatatgtcc tgcgggtaaa tagctgcgcc gatggtttct 3660

acaaagatcg ttatgtttat cggcactttg catcggccgc gctcccgatt ccggaagtgc 3720

ttgacattgg ggaattcagc gagagcctga cctattgcat ctcccgccgt gcacagggtg 3780

tcacgttgca agacctgcct gaaaccgaac tgcccgctgt tctgcagccg gtcgcggagg 3840

ccatggatgc gatcgctgcg gccgatctta gccagacgag cgggttcggc ccattcggac 3900

cgcaaggaat cggtcaatac actacatggc gtgatttcat atgcgcgatt gctgatcccc 3960

atgtgtatca ctggcaaact gtgatggacg acaccgtcag tgcgtccgtc gcgcaggctc 4020

tcgatgagct gatgctttgg gccgaggact gccccgaagt ccggcacctc gtgcacgcgg 4080

atttcggctc caacaatgtc ctgacggaca atggccgcat aacagcggtc attgactgga 4140

gcgaggcgat gttcggggat tcccaatacg aggtcgccaa catcttcttc tggaggccgt 4200

ggttggcttg tatggagcag cagacgcgct acttcgagcg gaggcatccg gagcttgcag 4260

gatcgccgcg gctccgggcg tatatgctcc gcattggtct tgaccaactc tatcagagct 4320

tggttgacgg caatttcgat gatgcagctt gggcgcaggg tcgatgcgac gcaatcgtcc 4380

gatccggagc cgggactgtc gggcgtacac aaatcgcccg cagaagcgcg gccgtctgga 4440

ccgatggctg tgtagaagta ctcgccgata gtggaaaccg acgccccagc actcgtccga 4500

gggcaaagga atagcggtga ccttcagacc ttggcactgg aggtggcccg gcagaagcgc 4560

ggcatcgtgg atcagtgctg caccagcatc tgctctctct accaactgga gaactactgc 4620

aactaggccc accactaccc tgtccacccc tctgcaatga ataaaacctt tgaaagagca 4680

ctacaagttg tgtgtacatg cgtgcatgtg catatgtggt gcggggggaa catgagtggg 4740

gctggctgga gtggcgatga taagctgtca aacatgagaa ttaattcttg aagacgaaag 4800

ggcctcgtga tacgcctatt tttataggtt aatgtcatga taataatggt ttcttagtct 4860

agaattaatt ccgtgtattc tatagtgtca cctaaatcgt atgtgtatga tacataaggt 4920

tatgtattaa ttgtagccgc gttctaacga caatatgtac aagcctaatt gtgtagcatc 4980

tggcttactg aagcagaccc tatcatctct ctcgtaaact gccgtcagag tcggtttggt 5040

tggacgaacc ttctgagttt ctggtaacgc cgtcccgcac ccggaaatgg tcagcgaacc 5100

aatcagcagg gtcatcgcta gccagatcct ctacgccgga cgcatcgtgg ccggcatcac 5160

cggcgccaca ggtgcggttg ctggcgccta tatcgccgac atcaccgatg gggaagatcg 5220

ggctcgccac ttcgggctca tgagcgcttg tttcggcgtg ggtatggtgg caggccccgt 5280

ggccggggga ctgttgggcg ccatctcctt gcatgcacca ttccttgcgg cggcggtgct 5340

caacggcctc aacctactac tgggctgctt cctaatgcag gagtcgcata agggagagcg 5400

tcgaatggtg cactctcagt acaatctgct ctgatgccgc atagttaagc cagccccgac 5460

acccgccaac acccgctgac gcgccctgac gggcttgtct gctcccggca tccgcttaca 5520

gacaagctgt gaccgtctcc gggagctgca tgtgtcagag gttttcaccg tcatcaccga 5580

aacgcgcgag acgaaagggc ctcgtgatac gcctattttt ataggttaat gtcatgataa 5640

taatggtttc ttagacgtca ggtggcactt ttcggggaaa tgtgcgcgga acccctattt 5700

gtttattttt ctaaatacat tcaaatatgt atccgctcat gagacaataa ccctgataaa 5760

tgcttcaata atattgaaaa aggaagagta tgagtattca acatttccgt gtcgccctta 5820

ttcccttttt tgcggcattt tgccttcctg tttttgctca cccagaaacg ctggtgaaag 5880

taaaagatgc tgaagatcag ttgggtgcac gagtgggtta catcgaactg gatctcaaca 5940

gcggtaagat ccttgagagt tttcgccccg aagaacgttt tccaatgatg agcactttta 6000

aagttctgct atgtggcgcg gtattatccc gtattgacgc cgggcaagag caactcggtc 6060

gccgcataca ctattctcag aatgacttgg ttgagtactc accagtcaca gaaaagcatc 6120

ttacggatgg catgacagta agagaattat gcagtgctgc cataaccatg agtgataaca 6180

ctgcggccaa cttacttctg acaacgatcg gaggaccgaa ggagctaacc gcttttttgc 6240

acaacatggg ggatcatgta actcgccttg atcgttggga accggagctg aatgaagcca 6300

taccaaacga cgagcgtgac accacgatgc ctgtagcaat ggcaacaacg ttgcgcaaac 6360

tattaactgg cgaactactt actctagctt cccggcaaca attaatagac tggatggagg 6420

cggataaagt tgcaggacca cttctgcgct cggcccttcc ggctggctgg tttattgctg 6480

ataaatctgg agccggtgag cgtgggtctc gcggtatcat tgcagcactg gggccagatg 6540

gtaagccctc ccgtatcgta gttatctaca cgacggggag tcaggcaact atggatgaac 6600

gaaatagaca gatcgctgag ataggtgcct cactgattaa gcattggtaa ctgtcagacc 6660

aagtttactc atatatactt tagattgatt taaaacttca tttttaattt aaaaggatct 6720

aggtgaagat cctttttgat aatctcatga ccaaaatccc ttaacgtgag ttttcgttcc 6780

actgagcgtc agaccccgta gaaaagatca aaggatcttc ttgagatcct ttttttctgc 6840

gcgtaatctg ctgcttgcaa acaaaaaaac caccgctacc agcggtggtt tgtttgccgg 6900

atcaagagct accaactctt tttccgaagg taactggctt cagcagagcg cagataccaa 6960

atactgttct tctagtgtag ccgtagttag gccaccactt caagaactct gtagcaccgc 7020

ctacatacct cgctctgcta atcctgttac cagtggctgc tgccagtggc gataagtcgt 7080

gtcttaccgg gttggactca agacgatagt taccggataa ggcgcagcgg tcgggctgaa 7140

cggggggttc gtgcacacag cccagcttgg agcgaacgac ctacaccgaa ctgagatacc 7200

tacagcgtga gctatgagaa agcgccacgc ttcccgaagg gagaaaggcg gacaggtatc 7260

cggtaagcgg cagggtcgga acaggagagc gcacgaggga gcttccaggg ggaaacgcct 7320

ggtatcttta tagtcctgtc gggtttcgcc acctctgact tgagcgtcga tttttgtgat 7380

gctcgtcagg ggggcggagc ctatggaaaa acgccagcaa cgcggccttt ttacggttcc 7440

tggccttttg ctggcctttt gctcacatgt tctttcctgc gttatcccct gattctgtgg 7500

ataaccgtat taccgccttt gagtgagctg ataccgctcg ccgcagccga acgaccgagc 7560

gcagcgagtc agtgagcgag gaagcggaag agcgcccaat acgcaaaccg cctctccccg 7620

cgcgttggcc gattcattaa tgcagctgtg gaatgtgtgt cagttagggt gtggaaagtc 7680

cccaggctcc ccagcaggca gaagtatgca aagcatgcat ctcaattagt cagcaaccag 7740

gtgtggaaag tccccaggct ccccagcagg cagaagtatg caaagcatgc atctcaatta 7800

gtcagcaacc atagtcccgc ccctaactcc gcccatcccg cccctaactc cgcccagttc 7860

cgcccattct ccgccccatg gctgactaat tttttttatt tatgcagagg ccgaggccgc 7920

ctcggcctct gagctattcc agaagtagtg aggaggcttt tttggaggcc taggcttttg 7980

caaaaagctt ggacacaaga caggcttgcg agatatgttt gagaatacca ctttatcccg 8040

cgtcagggag aggcagtgcg taaaaagacg cggactcatg tgaaatactg gtttttagtg 8100

cgccagatct ctataatctc gcgcaaccta ttttcccctc gaacactttt taagccgtag 8160

ataaacaggc tgggacactt cacatgagcg aaaaatacat cgtcacctgg gacatgttgc 8220

agatccatgc acgtaaactc gcaagccgac tgatgccttc tgaacaatgg aaaggcatta 8280

ttgccgtaag ccgtggcggt ctgtaccggg tgcgttactg gcgcgtgaac tgggtattcg 8340

tcatgtcgat accgtttgta tttccagcta cgatcacgac aaccagcgcg agcttaaagt 8400

gctgaaacgc gcagaaggcg atggcgaagg cttcatcgtt attgatgacc tggtggatac 8460

cggtggtact gcggttgcga ttcgtgaaat gtatccaaaa gcgcactttg tcaccatctt 8520

cgcaaaaccg gctggtcgtc cgctggttga tgactatgtt gttgatatcc cgcaagatac 8580

ctggattgaa cagccgtggg atatgggcgt cgtattcgtc ccgccaatct ccggtcgcta 8640

atcttttcaa cgcctggcac tgccgggcgt tgttcttttt aacttcaggc gggttacaat 8700

agtttccagt aagtattctg gaggctgcat ccatgacaca ggcaaacctg agcgaaaccc 8760

tgttcaaacc ccgctttaaa catcctgaaa cctcgacgct agtccgccgc tttaatcacg 8820

gcgcacaacc gcctgtgcag tcggcccttg atggtaaaac catccctcac tggtatcgca 8880

tgattaaccg tctgatgtgg atctggcgcg gcattgaccc acgcgaaatc ctcgacgtcc 8940

aggcacgtat tgtgatgagc gatgccgaac gtaccgacga tgatttatac gatacggtga 9000

ttggctaccg tggcggcaac tggatttatg agtgggcccc ggatctttgt gaaggaacct 9060

tacttctgtg gtgtgacata attggacaaa ctacctacag agatttaaag ctctaaggta 9120

aatataaaat ttttaacccg gatctttgtg aaggaacctt acttctgtgg tgtgacataa 9180

ttggacaaac tacctacaga gatttaaagc tctaaggtaa atataaaatt tttaagtgta 9240

taatgtgtta aactactgat tctaattgtt tgtgtatttt agattccaac ctatggaact 9300

gatgaatggg agcagtggtg gaatgccttt aatgaggaaa acctgttttg ctcagaagaa 9360

atgccatcta gtgatgatga ggctactgct gactctcaac attctactcc tccaaaaaag 9420

aagagaaagg tagaagaccc caaggacttt ccttcagaat tgctaagttt tttgagtcat 9480

gctgtgttta gtaatagaac tcttgcttgc tttgctattt acaccacaaa ggaaaaagct 9540

gcactgctat acaagaaaat tatggaaaaa tattctgtaa cctttataag taggcataac 9600

agttataatc ataacatact gttttttctt actccacaca ggcatagagt gtctgctatt 9660

aataactatg ctcaaaaatt gtgtaccttt agctttttaa tttgtaaagg ggttaataag 9720

gaatatttga tgtatagtgc cttgactaga gatcataatc agccatacca catttgtaga 9780

ggttttactt gctttaaaaa acctcccaca cctccccctg aacctgaaac ataaaatgaa 9840

tgcaattgtt gttgttgggc tgcaggaatt aattcgagct cgcccgacat tgattattga 9900

ctagttatta atagtaatca attacggggt cattagttca tagcccatat atggagttcc 9960

gcgttacata acttacggta aatggcccgc ctggctgacc gcccaacgac ccccgcccat 10020

tgacgtcaat aatgacgtat gttcccatag taacgccaat agggactttc cattgacgtc 10080

aatgggtgga gtatttacgg taaactgccc acttggcagt acatcaagtg tatcatatgc 10140

caagtacgcc ccctattgac gtcaatgacg gtaaatggcc cgcctggcat tatgcccagt 10200

acatgacctt atgggacttt cctacttggc agtacatcta cgtattagtc atcgctatta 10260

ccatggtgat gcggttttgg cagtacatca atgggcgtgg atagcggttt gactcacggg 10320

gatttccaag tctccacccc attgacgtca atgggagttt gttttggcac caaaatcaac 10380

gggactttcc aaaatgtcgt aacaactccg ccccattgac gcaaatgggc ggtaggcgtg 10440

tacggtggga ggtctatata agcagagctc gtttagtgaa ccgtcagatc gcctggagac 10500

gccatccacg ctgttttgac ctccatagaa gacaccggga ccgatccagc ctccgcggcc 10560

gggaacggtg cattggaacg cggattcccc gtgccaagag tgacgtaagt accgcctata 10620

gagtctatag gcccaccccc ttggcttctt atgcgacgga tcgatcccgt aataagcttc 10680

gaggtccgcg gccggccgcg ttgacgcgca cggcaagagg cgaggggcgg cgactggtga 10740

gagatgggtg cgagagcgtc agtattaagc gggggagaat tagatcgatg ggaaaaaatt 10800

cggttaaggc cagggggaaa gaaaaaatat aaattaaaac atatagtatg ggcaagcagg 10860

gagctagaac gattcgcagt taatcctggc ctgttagaaa catcagaagg ctgtagacaa 10920

atactgggac agctacaacc atcccttcag acaggatcag aagaacttag atcattatat 10980

aatacagtag caaccctcta ttgtgtgcat caaaggatag agataaaaga caccaaggaa 11040

gctttagaca agatagagga agagcaaaac aaaagtaaga aaaaagcaca gcaagcagca 11100
gctgacacag gacacagcaa tcaggtcagc caaaattacc ctatagtgca gaacatccag 11160
gggcaaatgg tacatcaggc catatcacct agaactttaa atgcatgggt aaaagtagta 11220
gaagagaagg ctttcagccc agaagtgata cccatgtttt cagcattatc agaaggagcc 11280
accccacaag atttaaacac catgctaaac acagtggggg gacatcaagc agccatgcaa 11340
atgttaaaag agaccatcaa tgaggaagct gcagaatggg atagagtgca tccagtgcat 11400
gcagggccta ttgcaccagg ccagatgaga gaaccaaggg gaagtgacat agcaggaact 11460
actagtaccc ttcaggaaca aataggatgg atgacacata atccacctat cccagtagga 11520
gaaatctata aaagatggat aatcctggga ttaaataaaa tagtaagaat gtatagccct 11580
accagcattc tggacataag acaaggacca aaggaaccct ttagagacta tgtagaccga 11640
ttctataaaa ctctaagagc cgagcaagct tcacaagagg taaaaaattg gatgacagaa 11700
accttgttgg tccaaaatgc gaacccagat tgtaagacta ttttaaaagc attgggacca 11760
ggagcgacac tagaagaaat gatgacagca tgtcagggag tggggggacc cggccataaa 11820
gcaagagttt tggctgaagc aatgagccaa gtaacaaatc cagctaccat aatgatacag 11880
aaaggcaatt ttaggaacca aagaaagact gttaagtgtt tcaattgtgg caaagaaggg 11940
cacatagcca aaaattgcag ggcccctagg aaaaagggct gttggaaatg tggaaaggaa 12000
ggacaccaaa tgaaagattg tactgagaga caggctaatt ttttagggaa gatctggcct 12060
tcccacaagg gaaggccagg gaattttctt cagagcagac cagagccaac agccccacca 12120
gaagagagct tcaggtttgg ggaagagaca acaactccct ctcagaagca ggagccgata 12180
gacaaggaac tgtatccttt agcttccctc agatcactct ttggcagcga cccctcgtca 12240
caataaagat aggggggcaa ttaaaggaag ctctattaga tacaggagca gatgatacag 12300
tattagaaga aatgaatttg ccaggaagat ggaaaccaaa aatgataggg ggaattggag 12360
gttttatcaa agtaagacag tatgatcaga tactcataga aatctgcgga cataaagcta 12420
taggtacagt attagtagga cctacacctg tcaacataat tggaagaaat ctgttgactc 12480
agattggctg cactttaaat tttcccatta gtcctattga gactgtacca gtaaaattaa 12540
agccaggaat ggatggccca aaagttaaac aatggccatt gacagaagaa aaaataaaag 12600
cattagtaga aatttgtaca gaaatggaaa aggaaggaaa aatttcaaaa attgggcctg 12660
aaaatccata caatactcca gtatttgcca taaagaaaaa agacagtact aaatggagaa 12720
aattagtaga tttcagagaa cttaataaga gaactcaaga tttctgggaa gttcaattag 12780
gaataccaca tcctgcaggg ttaaaacaga aaaaatcagt aacagtactg gatgtgggcg 12840
atgcatattt ttcagttccc ttagataaag acttcaggaa gtatactgca tttaccatac 12900
ctagtataaa caatgagaca ccagggatta gatatcagta caatgtgctt ccacagggat 12960
ggaaaggatc accagcaata ttccagtgta gcatgacaaa aatcttagag ccttttagaa 13020

aacaaaatcc agacatagtc atctatcaat acatggatga tttgtatgta ggatctgact 13080

tagaaatagg gcagcataga acaaaaatag aggaactgag acaacatctg ttgaggtggg 13140

gatttaccac accagacaaa aaacatcaga aagaacctcc attcctttgg atgggttatg 13200

aactccatcc tgataaatgg acagtacagc ctatagtgct gccagaaaag gacagctgga 13260

ctgtcaatga catacagaaa ttagtgggaa aattgaattg ggcaagtcag atttatgcag 13320

ggattaaagt aaggcaatta tgtaaacttc ttaggggaac caaagcacta acagaagtag 13380

taccactaac agaagaagca gagctagaac tggcagaaaa cagggagatt ctaaaagaac 13440

cggtacatgg agtgtattat gacccatcaa aagacttaat agcagaaata cagaagcagg 13500

ggcaaggcca atggacatat caaatttatc aagagccatt taaaaatctg aaaacaggaa 13560

agtatgcaag aatgaagggt gcccacacta atgatgtgaa acaattaaca gaggcagtac 13620

aaaaaatagc cacagaaagc atagtaatat ggggaaagac tcctaaattt aaattaccca 13680

tacaaaagga aacatgggaa gcatggtgga cagagtattg gcaagccacc tggattcctg 13740

agtgggagtt tgtcaatacc cctcccttag tgaagttatg gtac 13784

<210> 8
<211> 7586
<212> DNA
<213> Artificial sequence

<220>
<223> SEQ ID NO: 8

<400> 8
gcggccgctc tagagagctt ggcccattgc atacgttgta tccatatcat aatatgtaca 60

tttatattgg ctcatgtcca acattaccgc catgttgaca ttgattattg actagttatt 120

aatagtaatc aattacgggg tcattagttc atagcccata tatggagttc cgcgttacat 180

aacttacggt aaatggcccg cctggctgac cgcccaacga cccccgccca ttgacgtcaa 240

taatgacgta tgttcccata gtaacgccaa tagggacttt ccattgacgt caatgggtgg 300

agtatttacg gtaaactgcc cacttggcag tacatcaagt gtatcatatg ccaagtacgc 360

cccctattga cgtcaatgac ggtaaatggc ccgcctggca ttatgcccag tacatgacct 420

tatgggactt tcctacttgg cagtacatct acgtattagt catcgctatt accatggtga 480

tgcggttttg gcagtacatc aatgggcgtg gatagcggtt tgactcacgg ggatttccaa 540

gtctccaccc cattgacgtc aatgggagtt tgttttggca ccaaaatcaa cgggactttc 600

caaaatgtcg taacaactcc gccccattga cgcaaatggg cggtaggcgt gtacggtggg 660

aggtctatat aagcagagct cgtttagtga accgtcagat cgcctggaga cgccatccac 720

gctgttttga cctccataga agacaccggg accgatccag cctccggtcg accgatcctg 780

agaacttcag ggtgagtttg gggacccttg attgttcttt ctttttcgct attgtaaaat 840

tcatgttata tggagggggc aaagttttca gggtgttgtt tagaatggga agatgtccct 900

tgtatcacca tggaccctca tgataatttt gtttctttca ctttctactc tgttgacaac 960

cattgtctcc tcttattttc ttttcatttt ctgtaacttt ttcgttaaac tttagcttgc 1020

atttgtaacg aatttttaaa ttcacttttg tttatttgtc agattgtaag tactttctct 1080

aatcactttt ttttcaaggc aatcagggta tattatattg tacttcagca cagttttaga 1140

gaacaattgt tataattaaa tgataaggta gaatatttct gcatataaat tctggctggc 1200

gtggaaatat tcttattggt agaaacaact acaccctggt catcatcctg cctttctctt 1260

tatggttaca atgatataca ctgtttgaga tgaggataaa atactctgag tccaaaccgg 1320

gcccctctgc taaccatgtt catgccttct tctctttcct acagctcctg ggcaacgtgc 1380

tggttgttgt gctgtctcat cattttggca aagaattcct cgacggatct gtcgaggtcg 1440

acggtatcga taagcttgat catgaatata ccttgctttg ctgtgatcct cagcttagct 1500

actacacatt ctctgggaga attccccttg tatacgattc ccgagaaaat agagaaatgg 1560

acccccatag acatgatcca tcttagttgc cctaataaca tgctgtctga ggaagaaggt 1620

tgcaatacag agtctccttt cacctacttc gagctcaaga gtggttacct agcccatcag 1680

aaggtcccag gatttacatg cactggggtt gtgaatgagg cagagacata cacaaacttt 1740

gtcggatatg tcaccaccac cttcaaaagg aagcacttta aacctacagt ggctgcttgt 1800

cgtgatgcct acaactggaa agtatcaggg gacccccgat atgaagaatc tctacacacc 1860

ccgtatcccg acagcagctg gttaaggact gtgaccacaa ccaaagaagc ccttcttata 1920

atatcgccaa gcattgtaga gatggacata tatggcagga cccttcactc tcccatgttc 1980

ccttcgggga aatgttccaa gctctatcct tctgtcccct cttgtacaac caaccatgat 2040

tacacattgt ggttgccaga agattctagt ctgagtttga tttgcgacat cttcacttcc 2100

agcagtggac agaaggccat gaatgggtct cgcatctgcg gattcaagga tgaaagggga 2160

ttttacagat ccttgaaggg atcctgtaag ctgacattgt gcgggaaacc tggaattagg 2220

ctgttcgacg gaacttgggt ctcttttaca aagccggacg ttcatgtgtg gtgcactccc 2280

aaccagttag tcaatataca taacgacaga ctagatgagg ttgaacatct gatcgtggac 2340

gatatcatca agaagagaga ggagtgttta gacacgctgg aaactatact tatgtctcaa 2400

tcagttagtt ttagacggtt gagccatttc agaaagttag ttccaggata tggaaaagct 2460

tacactattt tgaacggcag cttaatggaa acaaatgtct actacaaaag agttgacagg 2520

tgggcggaca ttttgccttc taggggatgt ctgaaagtcg gacaacagtg catggaccct 2580

gtcaaagggg tcctcttcaa cggaattatc aagggtccgg atggacaaat attgattcca 2640

gagatgcagt cagagcagct caaacagcat atggatctgt tgaaagcagc tatgtttcct 2700

ctccgtcatc ctttaatcaa cagagaggca gtcttcaaga aggatggaaa tgccgatgat 2760

tttgttgatc tccatatgcc tgatgttcaa aaatctgtgt cggatgtcga cctgggcctg 2820

cctcattggg ggttctggtt gttagtcggg gcaacagtag tagcctttgt ggtcttggcg 2880

tgcttgctcc gtgtatgttg taggagaatg agaaggagaa ggtcactgcg tgccactcag 2940

gatatccccc tcagcgttgc ccctgcccct gtccctcgtg ccaaagtggt gtcatcatgg 3000

gagtcttcta aagggctccc aggtacttga tgtgtctata tgtgattttc caccatattg 3060

ccgtcttttg gcaatgtgag ggcccggaaa cctggccctg tcttcttgac gagcattcct 3120

aggggtcttt cccctctcgc caaaggaatg caaggtctgt tgaatgtcgt gaaggaagca 3180

gttcctctgg aagcttcttg aagacaaaca acgtctgtag cgaccctttg caggcagcgg 3240

aaccccccac ctggcgacag gtgcctctgc ggccaaaagc cacgtgtata agatacacct 3300

gcaaaggcgg cacaacccca gtgccacgtt gtgagttgga tagttgtgga aagagtcaaa 3360

tggctctcct caagcgtatt caacaagggg ctgaaggatg cccagaaggt accccattgt 3420

atgggatctg atctggggcc tcggtgcaca tgctttacat gtgtttagtc gaggttaaaa 3480

aaacgtctag gccccccgaa ccacggggac gtggttttcc tttgaaaaac acgatgataa 3540

gctcaagctt accatgaccg agtacaagcc cacggtgcgc ctcgccaccc gcgacgacgt 3600

ccccagggcc gtacgcaccc tcgccgccgc gttcgccgac taccccgcca cgcgccacac 3660

cgtcgatccg gaccgccaca tcgagcgggt caccgagctg caagaactct tcctcacgcg 3720

cgtcgggctc gacatcggca aggtgtgggt cgcggacgac ggcgccgcgg tggcggtctg 3780

gaccacgccg gagagcgtcg aagcgggggc ggtgttcgcc gagatcggcc cgcgcatggc 3840

cgagttgagc ggttcccggc tggccgcgca gcaacagatg gaaggcctcc tggcgccgca 3900

ccggcccaag gagcccgcgt ggttcctggc caccgtcggc gtctcgcccg accaccaggg 3960

caagggtctg ggcagcgccg tcgtgctccc cggagtggag gcggccgagc gcgccggggt 4020

gcccgccttc ctggagacct ccgcgccccg caacctcccc ttctacgagc ggctcggctt 4080

caccgtcacc gccgacgtcg aggtgcccga aggaccgcgc acctggtgca tgacccgcaa 4140

gcccggtgcc tgactttatc atactgcctg acacatagat cacaacctga ttacctgcct 4200

cggcacagat ccactcctca ggtgcaggct gcctatcaga aggtggtggc tggtgtggcc 4260

aatgccctgg ctcacaaata ccactgagat ctttttccct ctgccaaaaa ttatggggac 4320

atcatgaagc cccttgagca tctgacttct ggctaataaa ggaaatttat tttcattgca 4380

atagtgtgtt ggaatttttt gtgtctctca ctcggaagga catatgggag ggcaaatcat 4440

ttaaaacatc agaatgagta tttggtttag agtttggcaa catatgccca tatgctggct 4500

gccatgaaca aaggttggct ataaagaggt catcagtata tgaaacagcc ccctgctgtc 4560

cattccttat tccatagaaa agccttgact tgaggttaga ttttttttat attttgtttt 4620

gtgttatttt tttctttaac atccctaaaa ttttccttac atgttttact agccagattt 4680

ttcctcctct cctgactact cccagtcata gctgtccctc ttctcttatg gagatccctc 4740

gacggatcgg ccgcaattcg taatcatgtc atagctgttt cctgtgtgaa attgttatcc 4800

gctcacaatt ccacacaaca tacgagccgg aagcataaag tgtaaagcct ggggtgccta 4860

atgagtgagc taactcacat taattgcgtt gcgctcactg cccgctttcc agtcgggaaa 4920

cctgtcgtgc cagctgcatt aatgaatcgg ccaacgcgcg gggagaggcg gtttgcgtat 4980

tgggcgctct tccgcttcct cgctcactga ctcgctgcgc tcggtcgttc ggctgcggcg 5040

agcggtatca gctcactcaa aggcggtaat acggttatcc acagaatcag gggataacgc 5100

aggaaagaac atgtgagcaa aaggccagca aaaggccagg aaccgtaaaa aggccgcgtt 5160

gctggcgttt ttccataggc tccgcccccc tgacgagcat cacaaaaatc gacgctcaag 5220

tcagaggtgg cgaaacccga caggactata aagataccag gcgtttcccc ctggaagctc 5280

cctcgtgcgc tctcctgttc cgaccctgcc gcttaccgga tacctgtccg cctttctccc 5340

ttcgggaagc gtggcgcttt ctcatagctc acgctgtagg tatctcagtt cggtgtaggt 5400

cgttcgctcc aagctgggct gtgtgcacga accccccgtt cagcccgacc gctgcgcctt 5460

atccggtaac tatcgtcttg agtccaaccc ggtaagacac gacttatcgc cactggcagc 5520

agccactggt aacaggatta gcagagcgag gtatgtaggc ggtgctacag agttcttgaa 5580

gtggtggcct aactacggct acactagaag aacagtattt ggtatctgcg ctctgctgaa 5640

gccagttacc ttcggaaaaa gagttggtag ctcttgatcc ggcaaacaaa ccaccgctgg 5700

tagcggtggt ttttttgttt gcaagcagca gattacgcgc agaaaaaaag gatctcaaga 5760

agatcctttg atcttttcta cggggtctga cgctcagtgg aacgaaaact cacgttaagg 5820

gattttggtc atgagattat caaaaaggat cttcacctag atccttttaa attaaaaatg 5880

aagttttaaa tcaatctaaa gtatatatga gtaaacttgg tctgacagtt accaatgctt 5940

aatcagtgag gcacctatct cagcgatctg tctatttcgt tcatccatag ttgcctgact 6000

ccccgtcgtg tagataacta cgatacggga gggcttacca tctggcccca gtgctgcaat 6060

gataccgcga gacccacgct caccggctcc agatttatca gcaataaacc agccagccgg 6120

aagggccgag cgcagaagtg gtcctgcaac tttatccgcc tccatccagt ctattaattg 6180

ttgccgggaa gctagagtaa gtagttcgcc agttaatagt ttgcgcaacg ttgttgccat 6240

tgctacaggc atcgtggtgt cacgctcgtc gtttggtatg gcttcattca gctccggttc 6300

ccaacgatca aggcgagtta catgatcccc catgttgtgc aaaaaagcgg ttagctcctt 6360

cggtcctccg atcgttgtca gaagtaagtt ggccgcagtg ttatcactca tggttatggc 6420

agcactgcat aattctctta ctgtcatgcc atccgtaaga tgcttttctg tgactggtga 6480

gtactcaacc aagtcattct gagaatagtg tatgcggcga ccgagttgct cttgcccggc 6540

gtcaatacgg gataataccg cgccacatag cagaacttta aaagtgctca tcattggaaa 6600

acgttcttcg gggcgaaaac tctcaaggat cttaccgctg ttgagatcca gttcgatgta 6660

acccactcgt gcacccaact gatcttcagc atcttttact ttcaccagcg tttctgggt 6720

gagcaaaaac aggaaggcaa aatgccgcaa aaaagggaat aagggcgaca cggaaatgtt 6780

gaatactcat actcttcctt tttcaatatt attgaagcat ttatcagggt tattgtctca 6840

tgagcggata catatttgaa tgtatttaga aaaataaaca aataggggtt ccgcgcacat 6900

ttccccgaaa agtgccacct aaattgtaag cgttaatatt ttgttaaaat tcgcgttaaa 6960

tttttgttaa atcagctcat tttttaacca ataggccgaa atcggcaaaa tcccttataa 7020

atcaaaagaa tagaccgaga tagggttgag tgttgttcca gtttggaaca agagtccact 7080

attaaagaac gtggactcca acgtcaaagg gcgaaaaacc gtctatcagg gcgatggccc 7140

actacgtgaa ccatcaccct aatcaagttt tttggggtcg aggtgccgta aagcactaaa 7200

tcggaaccct aaagggagcc cccgatttag agcttgacgg ggaaagccgg cgaacgtggc 7260

gagaaaggaa gggaagaaag cgaaaggagc gggcgctagg gcgctggcaa gtgtagcggt 7320

cacgctgcgc gtaaccacca cacccgccgc gcttaatgcg ccgctacagg gcgcgtccca 7380

ttcgccattc aggctgcgca actgttggga agggcgatcg gtgcgggcct cttcgctatt 7440

acgccagctg gcgaaagggg gatgtgctgc aaggcgatta agttgggtaa cgccagggtt 7500

ttcccagtca cgacgttgta aaacgacggc cagtgagcgc gcgtaatacg actcactata 7560

gggcgaattg gagctccacc gcggtg 7586

<210> 9
<211> 11070
<212> DNA
<213> Artificial sequence

<220>
<223> SEQ ID NO: 9

<400> 9
agggcctcgt gatacgccta tttttatagg ttaatgtcat gataataatg gtttcttaga 60

cgtcaggtgg cacttttcgg ggaaatgtgc gcggaacccc tatttgttta tttttctaaa 120

tacattcaaa tatgtatccg ctcatgagac aataaccctg ataaatgctt caataatatt 180

gaaaaaggaa gagtatgagt attcaacatt tccgtgtcgc ccttattccc ttttttgcgg 240

cattttgcct tcctgttttt gctcacccag aaacgctggt gaaagtaaaa gatgctgaag 300

atcagttggg tgcacgagtg ggttacatcg aactggatct caacagcggt aagatccttg 360

agagttttcg ccccgaagaa cgttttccaa tgatgagcac ttttaaagtt ctgctatgtg 420

gcgcggtatt atcccgtatt gacgccgggc aagagcaact cggtcgccgc atacactatt 480

ctcagaatga cttggttgag tactcaccag tcacagaaaa gcatcttacg gatggcatga 540

cagtaagaga attatgcagt gctgccataa ccatgagtga taacactgcg gccaacttac 600

ttctgacaac gatcggagga ccgaaggagc taaccgcttt tttgcacaac atgggggatc 660

atgtaactcg ccttgatcgt tgggaaccgg agctgaatga agccatacca aacgacgagc 720

gtgacaccac gatgcctgta gcaatggcaa caacgttgcg caaactatta actggcgaac 780

tacttactct agcttcccgg caacaattaa tagactggat ggaggcggat aaagttgcag 840

gaccacttct gcgctcggcc cttccggctg gctggtttat tgctgataaa tctggagccg 900

gtgagcgtgg gtctcgcggt atcattgcag cactggggcc agatggtaag ccctcccgta 960

tcgtagttat ctacacgacg gggagtcagg caactatgga tgaacgaaat agacagatcg 1020

ctgagatagg tgcctcactg attaagcatt ggtaactgtc agaccaagtt tactcatata 1080

tactttagat tgatttaaaa cttcattttt aatttaaaag gatctaggtg aagatccttt 1140

ttgataatct catgaccaaa atcccttaac gtgagttttc gttccactga gcgtcagacc 1200

ccgtagaaaa gatcaaagga tcttcttgag atcctttttt tctgcgcgta atctgctgct 1260

tgcaaacaaa aaaaccaccg ctaccagcgg tggtttgttt gccggatcaa gagctaccaa 1320

ctctttttcc gaaggtaact ggcttcagca gagcgcagat accaaatact gtccttctag 1380

tgtagccgta gttaggccac cacttcaaga actctgtagc accgcctaca tacctcgctc 1440

tgctaatcct gttaccagtg gctgctgcca gtggcgataa gtcgtgtctt accgggttgg 1500

actcaagacg atagttaccg gataaggcgc agcggtcggg ctgaacgggg ggttcgtgca 1560

cacagcccag cttggagcga acgacctaca ccgaactgag atacctacag cgtgagcatt 1620

gagaaagcgc cacgcttccc gaagggagaa aggcggacag gtatccggta agcggcaggg 1680

tcggaacagg agagcgcacg agggagcttc cagggggaaa cgcctggtat ctttatagtc 1740

ctgtcgggtt tcgccacctc tgacttgagc gtcgattttt gtgatgctcg tcaggggggc 1800

ggagcctatg gaaaaacgcc agcaacgcgg cctttttacg gttcctggcc ttttgctggc 1860

cttttgctca catgttcttt cctgcgttat cccctgattc tgtggataac cgtattaccg 1920

cctttgagtg agctgatacc gctcgccgca gccgaacgac cgagcgcagc gagtcagtga 1980

gcgaggaagc ggaagagcgc ccaatacgca aaccgcctct ccccgcgcgt tggccgattc 2040

attaatgcag ctgtggaatg tgtgtcagtt agggtgtgga aagtccccag gctccccagc 2100

aggcagaagt atgcaaagca tgcatctcaa ttagtcagca accaggtgtg gaaagtcccc 2160

aggctcccca gcaggcagaa gtatgcaaag catgcatctc aattagtcag caaccatagt 2220

cccgccccta actccgccca tcccgcccct aactccgccc agttccgccc attctccgcc 2280

ccatggctga ctaatttttt ttatttatgc agaggccgag gccgcctcgg cctctgagct 2340

attccagaag tagtgaggag gcttttttgg aggcctaggc ttttgcaaaa agcttggaca 2400

caagacaggc ttgcgagata tgtttgagaa taccacttta tcccgcgtca gggagaggca 2460

gtgcgtaaaa agacgcggac tcatgtgaaa tactggtttt tagtgcgcca gatctctata 2520

atctcgcgca acctattttc ccctcgaaca ctttttaagc cgtagataaa caggctggga 2580

cacttcacat gagcgaaaaa tacatcgtca cctgggacat gttgcagatc catgcacgta 2640

aactcgcaag ccgactgatg ccttctgaac aatggaaagg cattattgcc gtaagccgtg 2700

gcggtctgta ccgggtgcgt tactggcgcg tgaactgggt attcgtcatg tcgataccgt 2760

ttgtatttcc agctacgatc acgacaacca gcgcgagctt aaagtgctga aacgcgcaga 2820

aggcgatggc gaaggcttca tcgttattga tgacctggtg gataccggtg gtactgcggt 2880

tgcgattcgt gaaatgtatc caaaagcgca ctttgtcacc atcttcgcaa aaccggctgg 2940

tcgtccgctg gttgatgact atgttgttga tatcccgcaa gatacctgga ttgaacagcc 3000

gtgggatatg ggcgtcgtat tcgtcccgcc aatctccggt cgctaatctt ttcaacgcct 3060

ggcactgccg ggcgttgttc tttttaactt caggcgggtt acaatagttt ccagtaagta 3120

ttctggaggc tgcatccatg acacaggcaa acctgagcga aaccctgttc aaaccccgct 3180

ttaaacatcc tgaaacctcg acgctagtcc gccgctttaa tcacggcgca caaccgcctg 3240

tgcagtcggc ccttgatggt aaaaccatcc ctcactggta tcgcatgatt aaccgtctga 3300

tgtggatctg gcgcggcatt gacccacgcg aaatcctcga cgtccaggca cgtattgtga 3360

tgagcgatgc cgaacgtacc gacgatgatt tatacgatac ggtgattggc taccgtggcg 3420

gcaactggat ttatgagtgg gccccggatc tttgtgaagg aaccttactt ctgtggtgtg 3480

acataattgg acaaactacc tacagagatt taaagctcta aggtaaatat aaaattttta 3540

agtgtataat gtgttaaact actgattcta attgtttgtg tattttagat tccaacctat 3600

ggaactgatg aatgggagca gtggtggaat gcctttaatg aggaaaacct gttttgctca 3660

gaagaaatgc catctagtga tgatgaggct actgctgact ctcaacattc tactcctcca 3720

aaaaagaaga gaaaggtaga agaccccaag gactttcctt cagaattgct aagttttttg 3780

agtcatgctg tgtttagtaa tagaactctt gcttgctttg ctatttacac cacaaaggaa 3840

aaagctgcac tgctatacaa gaaaattatg gaaaaatatt ctgtaacctt tataagtagg 3900

cataacagtt ataatcataa catactgttt tttcttactc cacacaggca tagagtgtct 3960

gctattaata actatgctca aaaattgtgt acctttagct ttttaatttg taaaggggtt 4020

aataaggaat atttgatgta tagtgccttg actagagatc ataatcagcc ataccacatt 4080

tgtagaggtt ttacttgctt taaaaaacct cccacacctc ccctgaacct gaaacataaa 4140

aatgaatgca attgttgttg ttaacttgtt tattgcagct tataatggtt acaaataaag 4200

caatagcatc acaaatttca caaataaagc atttttttca ctgcattcta gttgtggttt 4260

gtccaaactc atcaatgtat cttatcatgt ctggatcaac tggataactc aagctaacca 4320

aaatcatccc aaacttccca ccccataccc tattaccact gccaattacc tagtggtttc 4380

atttactcta aacctgtgat tcctctgaat tattttcatt ttaaagaaat tgtatttgtt 4440

aaatatgtac tacaaactta gtagttggaa gggctaattc actcccaacg aagacaagat 4500

atccttgatc tgtggatcta ccacacacaa ggctacttcc ctgattagca gaactacaca 4560

ccagggccag ggatcagata tccactgacc tttggatggt gctacaagct agtaccagtt 4620

gagccagaga agttagaaga agccaacaaa ggagagaaca ccagcttgtt acaacctgtg 4680

agcctgcatg ggatggatga cccggagaga gaagtgttag agtggaggtt tgacagccgc 4740

ctagcatttc atcacggtgg cccgagagct gcatccggag tacttcaaga actgctgata 4800

tcgagcttgc tacaagggac tttccgctgg gggactttcc agggaggcgt ggcctgggcg 4860

ggactgggga gtggcgagcc ctcagatcct gcatataagc agctgctttt tgcctgtact 4920

gggtctctct ggttagacca gatctgagcc tgggagctct ctggctaact agggaaccca 4980

ctgcttaagc ctcaataaag cttgccttga gtgcttcaag tagtgtgtgc ccgtctgttg 5040

tgtgactctg gtaactagag atccctcaga cccttttagt cagtgtggaa aatctctagc 5100

agttgaaagc gaaagggaaa ccagaggagc tctctcgacg caggactcgg cttgctgaag 5160

cgcgcacggc aagaggcgag gggcggcgac tggtgagtac gccaaaaatt ttgactagcg 5220

gaggctagaa ggagagagat gggtgcgaga gcgtcagtat taagcggggg agaattagat 5280

cgcgatggga aaaaattcgg ttaaggccag ggggaaagaa aaaatataaa ttaaaacata 5340

tagtatgggc aagcagggag ctagaacgat tcgcagttaa tcctggcctg ttagaaacat 5400

cagaaggctg tagacaaata ctgggacagc tacaaccatc ccttcagaca ggatcagaag 5460

aacttagatc attatataat acagtagcaa ccctctattg tgtgcatcaa aggatagaga 5520

taaaagacac caaggaagct ttagacaaga tagaggaaga gcaaaacaaa agtaagacca 5580

ccgcacagca agcggccgct gatcttcaga cctggaggag gagatatgag ggacaattgg 5640

agaagtgaat tatataaata taaagtagta aaaattgaac cattaggagt agcacccacc 5700

aaggcaaaga gaagagtggt gcagagagaa aaaagagcag tgggaatagg agctttgttc 5760

cttgggttct tgggagcagc aggaagcact atgggcgcag cgtcaatgac gctgacggta 5820

caggccagac aattattgtc tggtatagtg cagcagcaga acaatttgct gagggctatt 5880

gaggcgcaac agcatctgtt gcaactcaca gtctggggca tcaagcagct ccaggcaaga 5940

atcctggctg tggaaagata cctaaaggat caacagctcc tggggatttg gggttgctct 6000

ggaaaactca tttgcaccac tgctgtgcct tggaatgcta gttggagtaa taaatctctg 6060

gaacagattt ggaatcacac gacctggatg gagtgggaca gagaaattaa caattacaca 6120

agcttaatac actccttaat tgaagaatcg caaaaccagc aagaaaagaa tgaacaagaa 6180

ttattggaat tagataaatg ggcaagtttg tggaattggt ttaacataac aaattggctg 6240

tggtatataa aattattcat aatgatagta ggaggcttgg taggtttaag aatagttttt 6300

gctgtacttt ctatagtgaa tagagttagg cagggatatt caccattatc gtttcagacc 6360

cacctcccaa ccccgagggg acccgacagg cccgaaggaa tagaagaaga aggtggagag 6420

agagacagag acagatccat tcgattagtg aacggatctc gacggtatcg ccgaattcac 6480

aaatggcagt attcatccac aattttaaaa gaaaaggggg gattgggggg tacagtgcag 6540

gggaaagaat agtagacata atagcaacag acatacaaac taaagaatta caaaaacaaa 6600

ttacaaaaat tcaaaatttt cgggtttatt acagggacag cagagatcca ctttggctga 6660

tacgcggatc tacgcgtatt aatagtaatc aattacgggg tcattagttc atagcccata 6720

tatggagttc cgcgttacat aacttacggt aaatggcccg cctggctgac cgcccaacga 6780

cccccgccca ttgacgtcaa taatgacgta tgttcccata gtaacgccaa tagggacttt 6840

ccattgacgt caatgggtgg agtatttacg gtaaactgcc cacttggcag tacatcaagt 6900

gtatcatatg ccaagtacgc cccctattga cgtcaatgac ggtaaatggc ccgcctggca 6960

ttatgcccag tacatgacct tatgggactt tcctacttgg cagtacatct acgtattagt 7020

catcgctatt accatggtga tgcggttttg gcagtacatc aatgggcgtg gatagcggtt 7080

tgactcacgg ggatttccaa gtctccaccc cattgacgtc aatgggagtt tgttttggca 7140

ccaaaatcaa cgggactttc caaaatgtcg taacaactcc gccccattga cgcaaatggg 7200

cggtaggcgt gtacggtggg aggtctatat aagcagagct ctctggctaa ctagagaacc 7260

cactgcttac tggcttatcg aaattaatac gactcactat agggagaccc aagcttggta 7320

ccgagctcgg atcgatagct taagcgcatg attgaacaag atggattgca cgcaggttct 7380

ccggccgctt gggtggagag gctattcggc tatgactggg cacaacagac aatcggctgc 7440

tctgatgccg ccgtgttccg gctgtcagcg caggggcgcc cggttctttt tgtcaagacc 7500

gacctgtccg gtgccctgaa tgaactgcag gacgaggcag cgcggctatc gtggctggcc 7560

acgacgggcg ttccttgcgc agctgtgctc gacgttgtca ctgaagcggg aagggactgg 7620

ctgctattgg gcgaagtgcc ggggcaggat ctcctgtcat ctcaccttgc tcctgccgag 7680

aaagtatcca tcatggctga tgcaatgcgg cggctgcata cgcttgatcc ggctacctgc 7740

ccattcgacc accaagcgaa acatcgcatc gagcgagcac gtactcggat ggaagccggt 7800

cttgtcgatc aggatgatct ggacgaagag catcaggggc tcgcgccagc cgaactgttc 7860

gccaggctca aggcgcgcat gcccgacggc gaggatctcg tcgtgaccca tggcgatgcc 7920

tgcttgccga atatcatggt ggaaaatggc cgcttttctg gattcatcga ctgtggccgg 7980

ctgggtgtgg cggaccgcta tcaggacata gcgttggcta cccgtgatat tgctgaagag 8040

cttggcggcg aatgggctga ccgcttcctc gtgctttacg gtatcgccgc tcccgattcg 8100

cagcgcatcg ccttctatcg ccttcttgac gagttcttct gagcggaatt cagtggatcc 8160

actagtctcg agctcaagct tcgaattccc gataatcaac ctctggatta caaaatttgt 8220

gaaagattga ctggtattct taactatgtt gctcctttta cgctatgtgg atacgctgct 8280

ttaatgcctt tgtatcatgc tattgcttcc cgtatggctt tcattttctc ctccttgtat 8340

aaatcctggt tgctgtctct ttatgaggag ttgtggcccg ttgtcaggca acgtggcgtg 8400

gtgtgcactg tgtttgctga cgcaaccccc actggttggg gcattgccac cacctgtcag 8460

ctcctttccg ggactttcgc tttccccctc cctattgcca cggcggaact catcgccgcc 8520

tgccttgccc gctgctggac aggggctcgg ctgttgggca ctgacaattc cgtggtgttg 8580

tcggggaagc tgacgtcctt tccatggctg ctcgcctgtg ttgccacctg gattctgcgc 8640

gggacgtcct tctgctacgt cccttcggcc ctcaatccag cggaccttcc ttcccgcggc 8700

ctgctgccgg ctctgcggcc tcttccgcgt cttcgccttc gccctcagac gagtcggatc 8760

tccctttggg ccgcctcccc gcatcgggaa ttctgcagtc gacggtacct ttaagaccaa 8820

tgacttacaa ggcagctgta gatcttagcc actttttaaa agaaaagggg ggactggaag 8880

ggctaattca ctcccaacga agacaaaatc gtcgagagat gctgcatata agcagctgct 8940

ttttgcttgt actgggtctc tctggttaga ccagatctga gcctgggagc tctctggcta 9000

actagggaac ccactgctta agcctcaata aagcttgcct tgagtgcttc aagtagtgtg 9060

tgcccgtctg ttgtgtgact ctggtaacta gagatccctc agaccctttt agtcagtgtg 9120

gaaaatctct agcagtagta gttcatgtca tcttattatt cagtatttat aacttgcaaa 9180

gaaatgaata tcagagagtg agaggccttg acattataat agatttagca ggaattgaac 9240

taggagtgga gcacacaggc aaagctgcag aagtacttgg aagaagccac cagagatact 9300

cacgattctg cacatacctg gctaatccca gatcctaagg attacattaa gtttactaac 9360

atttatataa tgatttatag tttaaagtat aaacttatct aatttactat tctgacagat 9420

attaattaat cctcaaatat cataagagat gattactatt atccccattt aacacaagag 9480

gaaactgaga gggaaagatg ttgaagtaat tttcccacaa ttacagcatc cgttagttac 9540

gactctatga tcttctgaca caaattccat ttactcctca ccctatgact cagtcgaata 9600

tatcaaagtt atggacatta tgctaagtaa caaattaccc ttttatatag taaatactga 9660

gtagattgag agaagaaatt gtttgcaaac ctgaatagct tcaagaagaa gagaagtgag 9720

gataagaata acagttgtca tttaacaagt tttaacaagt aacttggtta gaaagggatt 9780

caaatgcata aagcaaggga taaatttttc tggcaacaag actatacaat ataaccttaa 9840

atatgacttc aaataattgt tggaacttga taaaactaat taaatattat tgaagattat 9900

caatattata aatgtaattt acttttaaaa agggaacata gaaatgtgta tcattagagt 9960

agaaaacaat ccttattatc acaatttgtc aaaacaagtt tgttattaac acaagtagaa 10020

tactgcattc aattaagttg actgcagatt ttgtgttttg ttaaaattag aaagagataa 10080

caacaatttg aattattgaa agtaacatgt aaatagttct acatacgttc ttttgacatc 10140

ttgttcaatc attgatcgaa gttctttatc ttggaagaat ttgttccaaa gactctgaaa 10200

taaggaaaac aatctattat atagtctcac acctttgttt tacttttagt gatttcaatt 10260

taataatgta aatggttaaa atttattctt ctctgagatc atttcacatt gcagatagaa 10320

aacctgagac tggggtaatt tttattaaaa tctaatttaa tctcagaaac acatctttat 10380

tctaacatca atttttccag tttgatatta tcatataaag tcagccttcc tcatctgcag 10440

gttccacaac aaaaatccaa ccaactgtgg atcaaaaata ttgggaaaaa attaaaaata 10500

gcaatacaac aataaaaaaa tacaaatcag aaaaacagca cagtataaca actttattta 10560

gcatttacaa tctattaggt attataagta atctagccag atcctctacg ccggacgcat 10620

cgtggccggc atcaccggcg ccacaggtgc ggttgctggc gcctatatcg ccgacatcac 10680

cgatggggaa gatcgggctc gccacttcgg gctcatgagc gcttgtttcg gcgtgggtat 10740

ggtggcaggc cccgtggccg ggggactgtt gggcgccatc tccttgcatg caccattcct 10800

tgcggcggcg gtgctcaacg gcctcaacct actactgggc tgcttcctaa tgcaggagtc 10860

gcataaggga gagcgtcgaa tggtgcactc tcagtacaat ctgctctgat gccgcatagt 10920

taagccagcc ccgacacccg ccaacacccg ctgacgcgcc ctgacgggct tgtctgctcc 10980

cggcatccgc ttacagacaa gctgtgaccg tctccgggag ctgcatgtgt cagaggtttt 11040

caccgtcatc accgaaacgc gcgagacgaa 11070

<210> 10
<211> 13862
<212> DNA
<213> Artificial sequence

<220>
<223> SEQ ID NO: 10

<400> 10
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaataaccc 660
   cgccccgttg acgcaaatgg gcggtaggcg tgtacggtgg gaggtctata taagcagagc 720
   tcgtttagtg aaccgtcaga tcactagaag ctttattgcg gtagtttatc acagttaaat 780
   tgctaacgca gtcagtgctt ctgacacaac agtctcgaac ttaagctgca gaagttggtc 840
   gtgaggcact gggcaggtaa gtatcaaggt tacaagacag gtttaaggag accaatagaa 900
   actgggcttg tcgagacaga gaagactctt gcgtttctga taggcaccta ttggtcttac 960
   tgacatccac tttgcctttc tctccacagg tgtccactcc cagttcaatt acagctctta 1020
   aggctagagt acttaatacg actcactata ggcgagttta ctccctatca gtgatagaga 1080
   acgtatgtcg agtttactcc ctatcagtga tagagaacga tgtcgagttt actccctatc 1140
   agtgatagag aacgtatgtc gagtttactc cctatcagtg atagagaacg tatgtcgagt 1200
   ttactcccta tcagtgatag agaacgtatg tcgagtttat ccctatcagt gatagagaac 1260
   gtatgtcgag tttactccct atcagtgata gagaacgtat gtcgaggtag gcgtgtacgg 1320
   tgggaggcct atataagcag agctcgttta gtgaaccgtc agatcgcctg gatgaagtgc 1380
   cttttgtact tagccttttt attcattggg gtgaattgca agttcaccat agtttttcca 1440
   cacaaccaaa aaggaaactg gaaaaatgtt ccttctaatt accattattg cccgtcaagc 1500
   tcagatttaa attggcataa tgacttaata ggcacagcca tacaagtcaa aatgcccaag 1560
   agtcacaagg ctattcaagc agacggttgg atgtgtcatg cttccaaatg ggtcactact 1620
   tgtgatttcc gctggtatgg accgaagtat ataacacagt ccatccgatc cttcactcca 1680
   tctgtagaac aatgcaagga aagcattgaa caaacgaaac aaggaacttg gctgaatcca 1740
   ggcttccctc ctcaaagttg tggatatgca actgtgacgg atgccgaagc agtgattgtc 1800
   caggtgactc ctcaccatgt gctggttgat gaatacacag gagaatgggt tgattcacag 1860
   ttcatcaacg gaaaatgcag caattacata tgccccactg tccataactc tacaacctgg 1920
   cattctgact ataaggtcaa agggctatgt gattctaacc tcatttccat ggacatcacc 1980
   ttcttctcag aggacggaga gctatcatcc ctgggaaagg agggcacagg gttcagaagt 2040
   aactactttg cttatgaaac tggaggcaag gcctgcaaaa tgcaatactg caagcattgg 2100
   ggagtcagac tcccatcagg tgtctggttc gagatggctg ataaggatct ctttgctgca 2160
   gccagattcc ctgaatgccc agaagggtca agtatctctg ctccatctca gacctcagtg 2220
   gatgtaagtc taattcagga cgttgagagg atcttggatt attccctctg ccaagaaacc 2280
   tggagcaaaa tcagagcggg tcttccaatc tctccagtgg atctcagcta tcttgctcct 2340
   aaaaacccag gaaccggtcc tgctttcacc ataatcaatg gtaccctaaa atactttgag 2400
   accagataca tcagagtcga tattgctgct ccaatcctct caagaatggt cggaatgatc 2460
   agtggaacta ccacagaaag ggaactgtgg gatgactggg caccatatga agacgtggaa 2520
   attggaccca atggagttct gaggaccagt tcaggatata agtttccttt atacatgatt 2580
   ggacatggta tgttggactc cgatcttcat cttagctcaa aggctcaggt gttcgaacat 2640
   cctcacattc aagacgctgc ttcgcaactt cctgatgatg agagtttatt ttttggtgat 2700
   actgggctat ccaaaaatcc aatcgagctt gtagaaggtt ggttcagtag ttggaaaagc 2760
   tctattgcct cttttttctt tatcataggg ttaatcattg gactattctt ggttctccga 2820
   gttggtatcc atctttgcat taaattaaag cacaccaaga aaagacagat ttatacagac 2880
   atagagatga accgagaagg atccgcggcc gcgccggctc tagatcgcga acgcgtgaat 2940
   tctaccgggt aggggaggcg cttttcccaa ggcagtctgg agcatgcgct ttagcagccc 3000
   cgctgggcac ttggcgctac acaagtggcc tctggcctcg cacacattcc acatccaccg 3060
   gtaggcgcca accggctccg ttctttggtg gccccttcgc gccaccttct actcctcccc 3120
   tagtcaggaa gttccccccc gccccgcagc tcgcgtcgtg caggacgtga caaatggaag 3180
   tagcacgtct cactagtctc gtgcagatgg acagcaccgc tgagcaatgg aagcgggtag 3240
   gcctttgggg cagcggccaa tagcagcttt gctccttcgc tttctgggct cagaggctgg 3300
   gaaggggtgg gtccgggggc gggctcaggg gcgggctcag gggcggggcg ggcgcccgaa 3360
   ggtcctccgg aggcccggca ttctgcacgc ttcaaaagcg cacgtctgcc gcgctgttct 3420
   cctcttcctc atctccgggc ctttcgtatg ggtgcgagag cgtcagtatt aagcggggga 3480
   gaattagatc gatgggaaaa aattcggtta aggccagggg gaaagaaaaa atataaatta 3540
   aaacatatag tatgggcaag cagggagcta gaacgattcg cagttaatcc tggcctgtta 3600
   gaaacatcag aaggctgtag acaaatactg ggacagctac aaccatccct tcagacagga 3660
   tcagaagaac ttagatcatt atataataca gtagcaaccc tctattgtgt gcatcaaagg 3720
   atagagataa aagacaccaa ggaagcttta gacaagatag aggaagagca aaacaaaagt 3780
   aagaaaaaag cacagcaagc agcagctgac acaggacaca gcaatcaggt cagccaaaat 3840
   taccctatag tgcagaacat ccaggggcaa atggtacatc aggccatatc acctagaact 3900
   ttaaatgcat gggtaaaagt agtagaagag aaggctttca gcccagaagt gatacccatg 3960
   ttttcagcat tatcagaagg agccacccca caagatttaa acaccatgct aaacacagtg 4020
   gggggacatc aagcagccat gcaaatgtta aaagagacca tcaatgagga agctgcagaa 4080
   tgggatagag tgcatccagt gcatgcaggg cctattgcac caggccagat gagagaacca 4140
   aggggaagtg acatagcagg aactactagt acccttcagg aacaaatagg atggatgaca 4200
   cataatccac ctatcccagt aggagaaatc tataaaagat ggataatcct gggattaaat 4260
   aaaatagtaa gaatgtatag ccctaccagc attctggaca taagacaagg accaaaggaa 4320
   ccctttagag actatgtaga ccgattctat aaaactctaa gagccgagca agcttcacaa 4380
   gaggtaaaaa attggatgac agaaaccttg ttggtccaaa atgcgaaccc agattgtaag 4440
   actattttaa aagcattggg accaggagcg acactagaag aaatgatgac agcatgtcag 4500
   ggagtggggg gacccggcca taaagcaaga gttttggctg aagcaatgag ccaagtaaca 4560
   aatccagcta ccataatgat acagaaaggc aattttagga accaaagaaa gactgttaag 4620
   tgtttcaatt gtggcaaaga agggcacata gccaaaaatt gcagggcccc taggaaaaag 4680
   ggctgttgga aatgtggaaa ggaaggacac caaatgaaag attgtactga gagacaggct 4740
   aattttttag ggaagatctg gccttcccac aagggaaggc cagggaattt tcttcagagc 4800
   agaccagagc caacagcccc accagaagag agcttcaggt ttggggaaga gacaacaact 4860
   ccctctcaga agcaggagcc gatagacaag gaactgtatc ctttagcttc cctcagatca 4920
   ctctttggca gcgacccctc gtcacaataa agataggggg gcaattaaag gaagctctat 4980
   tagatacagg agcagatgat acagtattag aagaaatgaa tttgccagga agatggaaac 5040
   caaaaatgat agggggaatt ggaggtttta tcaaagtaag acagtatgat cagatactca 5100
   tagaaatctg cggacataaa gctataggta cagtattagt aggacctaca cctgtcaaca 5160
   taattggaag aaatctgttg actcagattg gctgcacttt aaattttccc attagtccta 5220
   ttgagactgt accagtaaaa ttaaagccag gaatggatgg cccaaaagtt aaacaatggc 5280
   cattgacaga agaaaaaata aaagcattag tagaaatttg tacagaaatg gaaaaggaag 5340
   gaaaaatttc aaaaattggg cctgaaaatc catacaatac tccagtattt gccataaaga 5400
   aaaaagacag tactaaatgg agaaaattag tagatttcag agaacttaat aagagaactc 5460
   aagatttctg ggaagttcaa ttaggaatac cacatcctgc agggttaaaa cagaaaaaat 5520
   cagtaacagt actggatgtg ggcgatgcat atttttcagt tcccttagat aaagacttca 5580
   ggaagtatac tgcatttacc atacctagta taaacaatga gacaccaggg attagatatc 5640
   agtacaatgt gcttccacag ggatggaaag gatcaccagc aatattccag tgtagcatga 5700
   caaaaatctt agagcctttt agaaaacaaa atccagacat agtcatctat caatacatgg 5760
   atgatttgta tgtaggatct gacttagaaa tagggcagca tagaacaaaa atagaggaac 5820
   tgagacaaca tctgttgagg tggggattta ccacaccaga caaaaaacat cagaaagaac 5880
   ctccattcct ttggatgggt tatgaactcc atcctgataa atggacagta cagcctatag 5940
   tgctgccaga aaaggacagc tggactgtca atgacataca gaaattagtg ggaaaattga 6000
   attgggcaag tcagatttat gcagggatta aagtaaggca attatgtaaa cttcttaggg 6060
   gaaccaaagc actaacagaa gtagtaccac taacagaaga agcagagcta gaactggcag 6120
   aaaacaggga gattctaaaa gaaccggtac atggagtgta ttatgaccca tcaaaagact 6180
   taatagcaga aatacagaag caggggcaag gccaatggac atatcaaatt tatcaagagc 6240
   catttaaaaa tctgaaaaca ggaaagtatg caagaatgaa gggtgcccac actaatgatg 6300
   tgaaacaatt aacagaggca gtacaaaaaa tagccacaga aagcatagta atatggggaa 6360
   agactcctaa atttaaatta cccatacaaa aggaaacatg ggaagcatgg tggacagagt 6420
   attggcaagc cacctggatt cctgagtggg agtttgtcaa taccccctcc ttagtgaagt 6480
   tatggtacca gttagagaaa gaacccataa taggagcaga aactttctat gtagatgggg 6540
   cagccaatag ggaaactaaa ttaggaaaag caggatatgt aactgacaga ggaagacaaa 6600
   aagttgtccc cctaacggac acaacaaatc agaagactga gttacaagca attcatctag 6660
   ctttgcagga ttcgggatta gaagtaaaca tagtgacaga ctcacaatat gcattgggaa 6720
   tcattcaagc acaaccagat aagagtgaat cagagttagt cagtcaaata atagagcagt 6780
   taataaaaaa ggaaaaagtc tacctggcat gggtaccagc acacaaagga attggaggaa 6840
   atgaacaagt agataaattg gtcagtgctg gaatcaggaa agtactattt ttagatggaa 6900
   tagataaggc ccaagaagaa catgagaaat atcacagtaa ttggagagca atggctagtg 6960
   attttaacct accacctgta gtagcaaaag aaatagtagc cagctgtgat aaatgtcagc 7020
   taaaagggga agccatgcat ggacaagtag actgtagccc aggaatatgg cagctagatt 7080
   gtacacattt agaaggaaaa gttatcttgg tagcagttca tgtagccagt ggatatatag 7140
   aagcagaagt aattccagca gagacagggc aagaaacagc atacttcctc ttaaaattag 7200
   caggaagatg gccagtaaaa acagtacata cagacaatgg cagcaatttc accagtacta 7260
   cagttaaggc cgcctgttgg tgggcgggga tcaagcagga atttggcatt ccctacaatc 7320
   cccaaagtca aggagtaata gaatctatga ataaagaatt aaagaaaatt ataggacagg 7380
   taagagatca ggctgaacat cttaagacag cagtacaaat ggcagtattc atccacaatt 7440
   ttaaaagaaa aggggggatt ggggggtaca gtgcagggga aagaatagta gacataatag 7500
   caacagacat acaaactaaa gaattacaaa aacaaattac aaaaattcaa aattttcggg 7560
   tttattacag ggacagcaga gatccagttt ggaaaggacc agcaaagctc ctctggaaag 7620
   gtgaaggggc agtagtaata caagataata gtgacataaa agtagtgcca agaagaaaag 7680
   caaagatcat cagggattat ggaaaacaga tggcaggtga tgattgtgtg gcaagtagac 7740
   aggatgagga ttaacacatg gaattctgca acaactgctg tttatccatt tcagaattgg 7800
   gtgtcgacat agcagaatag gcgttactcg acagaggaga gcaagaaatg gagccagtag 7860
   atcctagact agagccctgg aagcatccag gaagtcagcc taaaactgct tgtaccaatt 7920
   gctattgtaa aaagtgttgc tttcattgcc aagtttgttt catgacaaaa gccttaggca 7980
   tctcctatgg caggaagaag cggagacagc gacgaagagc tcatcagaac agtcagactc 8040
   atcaagcttc tctatcaaag cagtaagtag tacatgtaat gcaacctata atagtagcaa 8100
   tagtagcatt agtagtagca ataataatag caatagttgt gtggtccata gtaatcatag 8160
   aatataggaa aatggccgct gatcttcaga cctggaggag gagatatgag ggacaattgg 8220
   agaagtgaat tatataaata taaagtagta aaaattgaac cattaggagt agcacccacc 8280
   aaggcaaaga gaagagtggt gcagagagaa aaaagagcag tgggaatagg agctttgttc 8340
   cttgggttct tgggagcagc aggaagcact atgggcgcag cgtcaatgac gctgacggta 8400
   caggccagac aattattgtc tggtatagtg cagcagcaga acaatttgct gagggctatt 8460
   gaggcgcaac agcatctgtt gcaactcaca gtctggggca tcaagcagct ccaggcaaga 8520
   atcctggctg tggaaagata cctaaaggat caacagctcc tggggatttg gggttgctct 8580
   ggaaaactca tttgcaccac tgctgtgcct tggaatgcta gttggagtaa taaatctctg 8640
   gaacagattt ggaatcacac gacctggatg gagtgggaca gagaaattaa caattacaca 8700
   agcttaatac actccttaat tgaagaatcg caaaaccagc aagaaaagaa tgaacaagaa 8760
   ttattggaat tagataaatg ggcaagtttg tggaattggt ttaacataac aaattggctg 8820
   tggtatataa aattattcat aatgatagta ggaggcttgg taggtttaag aatagttttt 8880
   gctgtacttt ctatagtgaa tagagttagg cagggatatt caccattatc gtttcagacc 8940
   cacctcccaa ccccgagggg acccgacagg cccgaaggaa tagaagaaga aggtggagag 9000
   agagacagag acagatccat tcgattagtg aacggatcct tggcacttat ctgggacgat 9060
   ctgcggagcc tgtgcctctt cagctaccac cgcttgagag acttactctt gattgtaacg 9120
   aggattgtgg aacttctggg acgcaggggg tgggaagccc tcaaatattg gtggaatctc 9180
   ctacaatatt ggagtcagga gctaaagaat agtgctgtta gcttgctcaa tgccacagcc 9240
   atagcagtag ctgaggggac agatagggtt atagaagtag tacaaggagc ttgtagagct 9300
   attcgccaca tacctagaag aataagacag ggcttggaaa ggattttgct atcctgcagc 9360
   tgtctatatg tgattttcca ccatattgcc gtcttttggc aatgtgaggg cccggaaacc 9420
   tggccctgtc ttcttgacga gcattcctag gggtctttcc cctctcgcca aaggaatgca 9480
   aggtctgttg aatgtcgtga aggaagcagt tcctctggaa gcttcttgaa gacaaacaac 9540
   gtctgtagcg accctttgca ggcagcggaa ccccccacct ggcgacaggt gcctctgcgg 9600
   ccaaaagcca cgtgtataag atacacctgc aaaggcggca caaccccagt gccacgttgt 9660
   gagttggata gttgtggaaa gagtcaaatg gctctcctca agcgtattca acaaggggct 9720
   gaaggatgcc cagaaggtac cccattgtat gggatctgat ctggggcctc ggtgcacatg 9780
   ctttacatgt gtttagtcga ggttaaaaaa acgtctaggc cccccgaacc acggggacgt 9840
   ggttttcctt tgaaaaacac gatgataagc tcaagcttac catggataga tccggaaagc 9900
   ctgaactcac cgcgacgtct gtcgagaagt ttctgatcga aaagttcgac agcgtctccg 9960
   acctgatgca gctctcggag ggcgaagaat ctcgtgcttt cagcttcgat gtaggagggc 10020
   gtggatatgt cctgcgggta aatagctgcg ccgatggttt ctacaaagat cgttatgttt 10080
   atcggcactt tgcatcggcc gcgctcccga ttccggaagt gcttgacatt ggggaattca 10140
   gcgagagcct gacctattgc atctcccgcc gtgcacaggg tgtcacgttg caagacctgc 10200
   ctgaaaccga actgcccgct gttctgcagc cggtcgcgga ggccatggat gcgatcgctg 10260
   cggccgatct tagccagacg agcgggttcg gcccattcgg accgcaagga atcggtcaat 10320
   acactacatg gcgtgatttc atatgcgcga ttgctgatcc ccatgtgtat cactggcaaa 10380
   ctgtgatgga cgacaccgtc agtgcgtccg tcgcgcaggc tctcgatgag ctgatgcttt 10440
   gggccgagga ctgccccgaa gtccggcacc tcgtgcacgc ggatttcggc tccaacaatg 10500
   tcctgacgga caatggccgc ataacagcgg tcattgactg gagcgaggcg atgttcgggg 10560
   attcccaata cgaggtcgcc aacatcttct tctggaggcc gtggttggct tgtatggagc 10620
   agcagacgcg ctacttcgag cggaggcatc cggagcttgc aggatcgccg cggctccggg 10680
   cgtatatgct ccgcattggt cttgaccaac tctatcagag cttggttgac ggcaatttcg 10740
   atgatgcagc ttgggcgcag ggtcgatgcg acgcaatcgt ccgatccgga gccgggactg 10800
   tcgggcgtac acaaatcgcc cgcagaagcg cggccgtctg gaccgatggc tgtgtagaag 10860
   tactcgccga tagtggaaac cgacgcccca gcactcgtcc gagggcaaag gaataggaga 10920
   attcacgcgt ggtacctcta gagtcgaccc gggcggccgc ttcgagcaga catgataaga 10980
   tacattgatg agtttggaca aaccacaact agaatgcagt gaaaaaaatg ctttatttgt 11040
   gaaatttgtg atgctattgc tttatttgta accattataa gctgcaataa acaagttaac 11100
   aacaacaatt gcattcattt tatgtttcag gttcaggggg agatgtggga ggttttttaa 11160
   agcaagtaaa acctctacaa atgtggtaaa atcgataagg atccgggctg gcgtaatagc 11220
   gaagaggccc gcaccgatcg cccttcccaa cagttgcgca gcctgaatgg cgaatggacg 11280
   cgccctgtag cggcgcatta agcgcggcgg gtgtggtggt tacgcgcagc gtgaccgcta 11340
   cacttgccag cgccctagcg cccgctcctt tcgctttctt cccttccttt ctcgccacgt 11400
   tcgccggctt tccccgtcaa gctctaaatc gggggctccc tttagggttc cgatttagtg 11460
   ctttacggca cctcgacccc aaaaaacttg attagggtga tggttcacgt agtgggccat 11520
   cgccctgata gacggttttt cgccctttga cgttggagtc cacgttcttt aatagtggac 11580
   tcttgttcca aactggaaca acactcaacc ctatctcggt ctattctttt gatttataag 11640
   ggattttgcc gatttcggcc tattggttaa aaaatgagct gatttaacaa aaatttaacg 11700
   cgaattttaa caaaatatta acgcttacaa tttcctgatg cggtattttc tccttacgca 11760
   tctgtgcggt atttcacacc gcatatggtg cactctcagt acaatctgct ctgatgccgc 11820
   atagttaagc cagccccgac acccgccaac acccgctgac gcgccctgac gggcttgtct 11880
   gctcccggca tccgcttaca gacaagctgt gaccgtctcc gggagctgca tgtgtcagag 11940
   gttttcaccg tcatcaccga aacgcgcgag acgaaagggc ctcgtgatac gcctattttt 12000
   ataggttaat gtcatgataa taatggtttc ttagacgtca ggtggcactt ttcggggaaa 12060
   tgtgcgcgga acccctattt gtttattttt ctaaatacat tcaaatatgt atccgctcat 12120
   gagacaataa ccctgataaa tgcttcaata atattgaaaa aggaagagta tgagtattca 12180
   acatttccgt gtcgccctta ttcccttttt tgcggcattt tgccttcctg tttttgctca 12240
   cccagaaacg ctggtgaaag taaaagatgc tgaagatcag ttgggtgcac gagtgggtta 12300
   catcgaactg gatctcaaca gcggtaagat ccttgagagt tttcgccccg aagaacgttt 12360
   tccaatgatg agcactttta aagttctgct atgtggcgcg gtattatccc gtattgacgc 12420
   cgggcaagag caactcggtc gccgcataca ctattctcag aatgacttgg ttgagtactc 12480
   accagtcaca gaaaagcatc ttacggatgg catgacagta agagaattat gcagtgctgc 12540
   cataaccatg agtgataaca ctgcggccaa cttacttctg acaacgatcg gaggaccgaa 12600
   ggagctaacc gcttttttgc acaacatggg ggatcatgta actcgccttg atcgttggga 12660
   accggagctg aatgaagcca taccaaacga cgagcgtgac accacgatgc ctgtagcaat 12720
   ggcaacaacg ttgcgcaaac tattaactgg cgaactactt actctagctt cccggcaaca 12780
   attaatagac tggatggagg cggataaagt tgcaggacca cttctgcgct cggcccttcc 12840
   ggctggctgg tttattgctg ataaatctgg agccggtgag cgtgggtctc gcggtatcat 12900
   tgcagcactg gggccagatg gtaagccctc ccgtatcgta gttatctaca cgacggggag 12960
   tcaggcaact atggatgaac gaaatagaca gatcgctgag ataggtgcct cactgattaa 13020
   gcattggtaa ctgtcagacc aagtttactc atatatactt tagattgatt taaaacttca 13080
   tttttaattt aaaaggatct aggtgaagat cctttttgat aatctcatga ccaaaatccc 13140
   ttaacgtgag ttttcgttcc actgagcgtc agaccccgta gaaaagatca aaggatcttc 13200
   ttgagatcct ttttttctgc gcgtaatctg ctgcttgcaa acaaaaaaac caccgctacc 13260
   agcggtggtt tgtttgccgg atcaagagct accaactctt tttccgaagg taactggctt 13320
   cagcagagcg cagataccaa atactgttct tctagtgtag ccgtagttag gccaccactt 13380
   caagaactct gtagcaccgc ctacatacct cgctctgcta atcctgttac cagtggctgc 13440
   tgccagtggc gataagtcgt gtcttaccgg gttggactca agacgatagt taccggataa 13500
   ggcgcagcgg tcgggctgaa cggggggttc gtgcacacag cccagcttgg agcgaacgac 13560
   ctacaccgaa ctgagatacc tacagcgtga gctatgagaa agcgccacgc ttcccgaagg 13620
   gagaaaggcg gacaggtatc cggtaagcgg cagggtcgga acaggagagc gcacgaggga 13680
   gcttccaggg ggaaacgcct ggtatcttta tagtcctgtc gggtttcgcc acctctgact 13740
   tgagcgtcga tttttgtgat gctcgtcagg ggggcggagc ctatggaaaa acgccagcaa 13800
   cgcggccttt ttacggttcc tggccttttg ctggcctttt gctcacatgg ctcgacagat 13860
   ct 13862

<210> 11
<211> 5744
<212> DNA
<213> Artificial sequence

<220>
<223> SEQ ID NO: 11

<400> 11
tagactggac aagagcaaag tcataaacgg cgctctggaa ttactcaatg gagtcggtat 60

cgaaggcctg acgacaagga aactcgctca aaagctggga gttgagcagc ctaccctgta 120

ctggcacgtg aagaacaagc gggccctgct cgatgccctg ccaatcgaga tgctggacag 180

gcatcatacc cacttctgcc ccctggaagg cgagtcatgg caagactttc tgcggaacaa 240

cgccaagtca ttccgctgtg ctctcctctc acatcgcgac ggggctaaag tgcatctcgg 300

cacccgccca acagagaaac agtacgaaac cctggaaaat cagctcgcgt tcctgtgtca 360

gcaaggcttc tccctggaga acgcactgta cgctctgtcc gccgtgggcc actttacact 420

gggctgcgta ttggaggaac aggagcatca agtagcaaaa gaggaaagag agacacctac 480

caccgattct atgcccccac ttctgagaca agcaattgag ctgttcgacc ggcagggagc 540

cgaacctgcc ttccttttcg gcctggaact aatcatatgt ggcctggaga aacagctaaa 600

gtgcgaaagc ggcgggccgg ccgacgccct tgacgatttt gacttagaca tgctcccagc 660

cgatgccctt gacgactttg accttgatat gctgcctgct gacgctcttg acgattttga 720

ccttgacatg ctccccgggt aaactagaag ctttattgcg gtagtttatc acagttaaat 780

tgctaacgca gtcagtgctt ctgacacaac agtctcgaac ttaagctgca tgtctatatg 840

tgattttcca ccatattgcc gtcttttggc aatgtgaggg cccggaaacc tggccctgtc 900

ttcttgacga gcattcctag gggtctttcc cctctcgcca aaggaatgca aggtctgttg 960

aatgtcgtga aggaagcagt tcctctggaa gcttcttgaa gacaaacaac gtctgtagcg 1020

accctttgca ggcagcggaa ccccccacct ggcgacaggt gcctctgcgg ccaaaagcca 1080

cgtgtataag atacacctgc aaaggcggca caaccccagt gccacgttgt gagttggata 1140

gttgtggaaa gagtcaaatg gctctcctca agcgtattca acaaggggct gaaggatgcc 1200

cagaaggtac cccattgtat gggatctgat ctggggcctc ggtgcacatg ctttacatgt 1260

gtttagtcga ggttaaaaaa acgtctaggc cccccgaacc acggggacgt ggttttcctt 1320

tgaaaaacac gatgataagc tcaagcttac catgaccgag tacaagccca cggtgcgcct 1380

cgccacccgc gacgacgtcc ccagggccgt acgcaccctc gccgccgcgt tcgccgacta 1440

ccccgccacg cgccacaccg tcgatccgga ccgccacatc gagcgggtca ccgagctgca 1500

agaactcttc ctcacgcgcg tcgggctcga catcggcaag gtgtgggtcg cggacgacgg 1560

cgccgcggtg gcggtctgga ccacgccgga gagcgtcgaa gcgggggcgg tgttcgccga 1620

gatcggcccg cgcatggccg agttgagcgg ttcccggctg gccgcgcagc aacagatgga 1680

aggcctcctg gcgccgcacc ggcccaagga gcccgcgtgg ttcctggcca ccgtcggcgt 1740

ctcgcccgac caccagggca agggtctggg cagcgccgtc gtgctccccg gagtggaggc 1800

ggccgagcgc gccggggtgc ccgccttcct ggagacctcc gcgccccgca acctcccctt 1860

ctacgagcgg ctcggcttca ccgtcaccgc cgacgtcgag gtgcccgaag gaccgcgcac 1920

ctggtgcatg acccgcaagc ccggtgcctg agaagttggt cgtgaggcac tgggcaggta 1980

agtatcaagg ttacaagaca ggtttaagga gaccaataga aactgggctt gtcgagacag 2040

agaagactct tgcgtttctg ataggcacct attggtctta ctgacatcca ctttgccttt 2100

ctctccacag gtgtccactc ccagttcaat tacagctctt aaggctagag tacttaatac 2160

gactcactat aggctagcct cgagaattca cgcgtggtac ctctagagtc gacccgggcg 2220

gccgcttcga gcagacatga taagatacat tgatgagttt ggacaaacca caactagaat 2280

gcagtgaaaa aaatgcttta tttgtgaaat ttgtgatgct attgctttat ttgtaaccat 2340

tataagctgc aataaacaag ttaacaacaa caattgcatt cattttatgt ttcaggttca 2400

gggggagatg tgggaggttt tttaaagcaa gtaaaacctc tacaaatgtg gtaaaatcga 2460

taaggatccg ggctggcgta atagcgaaga ggcccgcacc gatcgccctt cccaacagtt 2520

gcgcagcctg aatggcgaat ggacgcgccc tgtagcggcg cattaagcgc ggcgggtgtg 2580

gtggttacgc gcagcgtgac cgctacactt gccagcgccc tagcgcccgc tcctttcgct 2640

ttcttccctt cctttctcgc cacgttcgcc ggctttcccc gtcaagctct aaatcggggg 2700

ctccctttag ggttccgatt tagtgcttta cggcacctcg accccaaaaa acttgattag 2760

ggtgatggtt cacgtagtgg gccatcgccc tgatagacgg tttttcgccc tttgacgttg 2820

gagtccacgt tctttaatag tggactcttg ttccaaactg gaacaacact caaccctatc 2880

tcggtctatt cttttgattt ataagggatt ttgccgattt cggcctattg gttaaaaaat 2940

gagctgattt aacaaaaatt taacgcgaat tttaacaaaa tattaacgct tacaatttcc 3000

tgatgcggta ttttctcctt acgcatctgt gcggtatttc acaccgcata tggtgcactc 3060

tcagtacaat ctgctctgat gccgcatagt taagccagcc ccgacacccg ccaacacccg 3120

ctgacgcgcc ctgacgggct tgtctgctcc cggcatccgc ttacagacaa gctgtgaccg 3180

tctccgggag ctgcatgtgt cagaggtttt caccgtcatc accgaaacgc gcgagacgaa 3240

agggcctcgt gatacgccta tttttatagg ttaatgtcat gataataatg gtttcttaga 3300

cgtcaggtgg cacttttcgg ggaaatgtgc gcggaacccc tatttgttta tttttctaaa 3360

tacattcaaa tatgtatccg ctcatgagac aataaccctg ataaatgctt caataatatt 3420

gaaaaaggaa gagtatgagt attcaacatt tccgtgtcgc ccttattccc ttttttgcgg 3480

cattttgcct tcctgttttt gctcacccag aaacgctggt gaaagtaaaa gatgctgaag 3540

atcagttggg tgcacgagtg ggttacatcg aactggatct caacagcggt aagatccttg 3600

agagttttcg ccccgaagaa cgttttccaa tgatgagcac ttttaaagtt ctgctatgtg 3660

gcgcggtatt atcccgtatt gacgccgggc aagagcaact cggtcgccgc atacactatt 3720

ctcagaatga cttggttgag tactcaccag tcacagaaaa gcatcttacg gatggcatga 3780

cagtaagaga attatgcagt gctgccataa ccatgagtga taacactgcg gccaacttac 3840

ttctgacaac gatcggagga ccgaaggagc taaccgcttt tttgcacaac atgggggatc 3900

atgtaactcg ccttgatcgt tgggaaccgg agctgaatga agccatacca aacgacgagc 3960

gtgacaccac gatgcctgta gcaatggcaa caacgttgcg caaactatta actggcgaac 4020

tacttactct agcttcccgg caacaattaa tagactggat ggaggcggat aaagttgcag 4080

gaccacttct gcgctcggcc cttccggctg gctggtttat tgctgataaa tctggagccg 4140

gtgagcgtgg gtctcgcggt atcattgcag cactggggcc agatggtaag ccctcccgta 4200

tcgtagttat ctacacgacg gggagtcagg caactatgga tgaacgaaat agacagatcg 4260

ctgagatagg tgcctcactg attaagcatt ggtaactgtc agaccaagtt tactcatata 4320

tactttagat tgatttaaaa cttcattttt aatttaaaag gatctaggtg aagatccttt 4380

ttgataatct catgaccaaa atcccttaac gtgagttttc gttccactga gcgtcagacc 4440

ccgtagaaaa gatcaaagga tcttcttgag atcctttttt tctgcgcgta atctgctgct 4500

tgcaaacaaa aaaaccaccg ctaccagcgg tggtttgttt gccggatcaa gagctaccaa 4560

ctctttttcc gaaggtaact ggcttcagca gagcgcagat accaaatact gttcttctag 4620

tgtagccgta gttaggccac cacttcaaga actctgtagc accgcctaca tacctcgctc 4680

tgctaatcct gttaccagtg gctgctgcca gtggcgataa gtcgtgtctt accgggttgg 4740

actcaagacg atagttaccg gataaggcgc agcggtcggg ctgaacgggg ggttcgtgca 4800

cacagcccag cttggagcga acgacctaca ccgaactgag atacctacag cgtgagctat 4860

gagaaagcgc cacgcttccc gaagggagaa aggcggacag gtatccggta agcggcaggg 4920

tcggaacagg agagcgcacg agggagcttc cagggggaaa cgcctggtat ctttatagtc 4980

ctgtcgggtt tcgccacctc tgacttgagc gtcgattttt gtgatgctcg tcaggggggc 5040

ggagcctatg gaaaaacgcc agcaacgcgg cctttttacg gttcctggcc ttttgctggc 5100

cttttgctca catggctcga cagatctaag cttgggagtt ccgcgttaca taacttacgg 5160

taaatggccc gcctggctga ccgcccaacg acccccgccc attgacgtca ataatgacgt 5220

atgttcccat agtaacgcca atagggactt tccattgacg tcaatgggtg gagtatttac 5280

ggtaaactgc ccacttggca gtacatcaag tgtatcatat gccaagtacg ccccctattg 5340

acgtcaatga cggtaaatgg cccgcctggc attatgccca gtacatgacc ttatgggact 5400

ttcctacttg gcagtacatc tacgtattag tcatcgctat taccatggtg atgcggtttt 5460

ggcagtacat caatgggcgt ggatagcggt ttgactcacg gggatttcca agtctccacc 5520

ccattgacgt caatgggagt ttgttttggc accaaaatca acgggacttt ccaaaatgtc 5580

gtaacaactc cgccccattg acgcaaatgg gcggtaggcg tgtacggtgg gaggtctata 5640

taagcagagc tcgtttagtg aaccgtcaga tcgcctggag acgccatcca cgctgttttg 5700

acctccatag aagacaccga ctctactaga ggatccacca tgtc 5744

<210> 12
<211> 12150
<212> DNA
<213> Artificial sequence

<220>
<223> SEQ ID NO: 12

<400> 12
ttgattattg actagttatt aatagtaatc aattacgggg tcattagttc atagcccata 60

tatggagttc cgcgttacat aacttacggt aaatggcccg cctggctgac cgcccaacga 120

cccccgccca ttgacgtcaa taatgacgta tgttcccata gtaacgccaa tagggacttt 180

ccattgacgt caatgggtgg agtatttacg gtaaactgcc cacttggcag tacatcaagt 240

gtatcatatg ccaagtacgc cccctattga cgtcaatgac ggtaaatggc ccgcctggca 300

ttatgcccag tacatgacct tatgggactt tcctacttgg cagtacatct acgtattagt 360

catcgctatt accatggtga tgcggttttg gcagtacatc aatgggcgtg gatagcggtt 420

tgactcacgg ggatttccaa gtctccaccc cattgacgtc aatgggagtt tgttttggca 480

ccaaaatcaa cgggactttc caaaatgtcg taacaactcc gccccattga cgcaaatggg 540

cggtaggcgt gtacggtggg aggtctatat aagcagagct cgtttagtga accgtcagat 600

cgcctggaga cgccatccac gctgttttga cctccataga agacaccggg accgatccag 660

cctccgcggc cgggaacggt gcattggaac gcggattccc cgtgccaaga gtgacgtaag 720

taccgcctat agagtctata ggcccacccc cttggcttct tatgcgacgg atcgatcccg 780

taataagctt cgaggtccgc ggccggccgc gttgacgcgc acggcaagag gcgaggggcg 840

gcgactggtg agagatgggt gcgagagcgt cagtattaag cgggggagaa ttagatcgat 900

gggaaaaaat tcggttaagg ccagggggaa agaaaaaata taaattaaaa catatagtat 960

gggcaagcag ggagctagaa cgattcgcag ttaatcctgg cctgttagaa acatcagaag 1020

gctgtagaca aatactggga cagctacaac catcccttca gacaggatca gaagaactta 1080

gatcattata taatacagta gcaaccctct attgtgtgca tcaaaggata gagataaaag 1140

acaccaagga agctttagac aagatagagg aagagcaaaa caaaagtaag aaaaaagcac 1200

agcaagcagc agctgacaca ggacacagca atcaggtcag ccaaaattac cctatagtgc 1260

agaacatcca ggggcaaatg gtacatcagg ccatatcacc tagaacttta aatgcatggg 1320

taaaagtagt agaagagaag gctttcagcc cagaagtgat acccatgttt tcagcattat 1380

cagaaggagc caccccacaa gatttaaaca ccatgctaaa cacagtgggg ggacatcaag 1440

cagccatgca aatgttaaaa gagaccatca atgaggaagc tgcagaatgg gatagagtgc 1500

atccagtgca tgcagggcct attgcaccag gccagatgag agaaccaagg ggaagtgaca 1560

tagcaggaac tactagtacc cttcaggaac aaataggatg gatgacacat aatccaccta 1620

tcccagtagg agaaatctat aaaagatgga taatcctggg attaaataaa atagtaagaa 1680

tgtatagccc taccagcatt ctggacataa gacaaggacc aaaggaaccc tttagagact 1740

atgtagaccg attctataaa actctaagag ccgagcaagc ttcacaagag gtaaaaaatt 1800

ggatgacaga aaccttgttg gtccaaaatg cgaacccaga ttgtaagact attttaaaag 1860

cattgggacc aggagcgaca ctagaagaaa tgatgacagc atgtcaggga gtggggggac 1920

ccggccataa agcaagagtt ttggctgaag caatgagcca agtaacaaat ccagctacca 1980

taatgataca gaaaggcaat tttaggaacc aaagaaagac tgttaagtgt ttcaattgtg 2040

gcaaagaagg gcacatagcc aaaaattgca gggcccctag gaaaaagggc tgttggaaat 2100

gtggaaagga aggacaccaa atgaaagatt gtactgagag acaggctaat tttttaggga 2160

agatctggcc ttcccacaag ggaaggccag ggaattttct tcagagcaga ccagagccaa 2220

cagccccacc agaagagagc ttcaggtttg gggaagagac aacaactccc tctcagaagc 2280

aggagccgat agacaaggaa ctgtatcctt tagcttccct cagatcactc tttggcagcg 2340

acccctcgtc acaataaaga taggggggca attaaaggaa gctctattag atacaggagc 2400

agatgataca gtattagaag aaatgaattt gccaggaaga tggaaaccaa aaatgatagg 2460

gggaattgga ggttttatca aagtaagaca gtatgatcag atactcatag aaatctgcgg 2520

acataaagct ataggtacag tattagtagg acctacacct gtcaacataa ttggaagaaa 2580

tctgttgact cagattggct gcactttaaa ttttcccatt agtcctattg agactgtacc 2640

agtaaaatta aagccaggaa tggatggccc aaaagttaaa caatggccat tgacagaaga 2700

aaaaataaaa gcattagtag aaatttgtac agaaatggaa aaggaaggaa aaatttcaaa 2760

aattgggcct gaaaatccat acaatactcc agtatttgcc ataaagaaaa aagacagtac 2820

taaatggaga aaattagtag atttcagaga acttaataag agaactcaag atttctggga 2880

agttcaatta ggaataccac atcctgcagg gttaaaacag aaaaaatcag taacagtact 2940

ggatgtgggc gatgcatatt tttcagttcc cttagataaa gacttcagga agtatactgc 3000

atttaccata cctagtataa acaatgagac accagggatt agatatcagt acaatgtgct 3060

tccacaggga tggaaaggat caccagcaat attccagtgt agcatgacaa aaatcttaga 3120

gccttttaga aaacaaaatc cagacatagt catctatcaa tacatggatg atttgtatgt 3180

aggatctgac ttagaaatag ggcagcatag aacaaaaata gaggaactga gacaacatct 3240

gttgaggtgg ggatttacca caccagacaa aaaacatcag aaagaacctc cattcctttg 3300

gatgggttat gaactccatc ctgataaatg gacagtacag cctatagtgc tgccagaaaa 3360

ggacagctgg actgtcaatg acatacagaa attagtggga aaattgaatt gggcaagtca 3420

gatttatgca gggattaaag taaggcaatt atgtaaactt cttaggggaa ccaaagcact 3480

aacagaagta gtaccactaa cagaagaagc agagctagaa ctggcagaaa acagggagat 3540

tctaaaagaa ccggtacatg gagtgtatta tgacccatca aaagacttaa tagcagaaat 3600

acagaagcag gggcaaggcc aatggacata tcaaatttat caagagccat ttaaaaatct 3660

gaaaacagga aagtatgcaa gaatgaaggg tgcccacact aatgatgtga aacaattaac 3720

agaggcagta caaaaaatag ccacagaaag catagtaata tggggaaaga ctcctaaatt 3780

taaattaccc atacaaaagg aaacatggga agcatggtgg acagagtatt ggcaagccac 3840

ctggattcct gagtgggagt ttgtcaatac ccctccctta gtgaagttat ggtaccagtt 3900

agagaaagaa cccataatag gagcagaaac tttctatgta gatggggcag ccaataggga 3960

aactaaatta ggaaaagcag gatatgtaac tgacagagga agacaaaaag ttgtccccct 4020

aacggacaca acaaatcaga agactgagtt acaagcaatt catctagctt tgcaggattc 4080

gggattagaa gtaaacatag tgacagactc acaatatgca ttgggaatca ttcaagcaca 4140

accagataag agtgaatcag agttagtcag tcaaataata gagcagttaa taaaaaagga 4200

aaaagtctac ctggcatggg taccagcaca caaaggaatt ggaggaaatg aacaagtaga 4260

taaattggtc agtgctggaa tcaggaaagt actattttta gatggaatag ataaggccca 4320

agaagaacat gagaaatatc acagtaattg gagagcaatg gctagtgatt ttaacctacc 4380

acctgtagta gcaaaagaaa tagtagccag ctgtgataaa tgtcagctaa aaggggaagc 4440

catgcatgga caagtagact gtagcccagg aatatggcag ctagtttgta cacatttaga 4500

aggaaaagtt atcttggtag cagttcatgt agccagtgga tatatagaag cagaagtaat 4560

tccagcagag acagggcaag aaacagcata cttcctctta aaattagcag gaagatggcc 4620

agtaaaaaca gtacatacag acaatggcag caatttcacc agtactacag ttaaggccgc 4680

ctgttggtgg gcggggatca agcaggaatt tggcattccc tacaatcccc aaagtcaagg 4740

agtaatagaa tctatgaata aagaattaaa gaaaattata ggacaggtaa gagatcaggc 4800

tgaacatctt aagacagcag tacaaatggc agtattcatc cacaatttta aaagaaaagg 4860

ggggattggg gggtacagtg caggggaaag aatagtagac ataatagcaa cagacataca 4920

aactaaagaa ttacaaaaac aaattacaaa aattcaaaat tttcgggttt attacaggga 4980

cagcagagat ccagtttgga aaggaccagc aaagctcctc tggaaaggtg aaggggcagt 5040

agtaatacaa gataatagtg acataaaagt agtgccaaga agaaaagcaa agatcatcag 5100

ggattatgga aaacagatgg caggtgatga ttgtgtggca agtagacagg atgaggatta 5160

acacatggaa ttctgcaaca actgctgttt atccatttca gaattgggtg tcgacatagc 5220

agaataggcg ttactcgaca gaggagagca agaaatggag ccagtagatc ctagactaga 5280

gccctggaag catccaggaa gtcagcctaa aactgcttgt accaattgct attgtaaaaa 5340

gtgttgcttt cattgccaag tttgtttcat gacaaaagcc ttaggcatct cctatggcag 5400

gaagaagcgg agacagcgac gaagagctca tcagaacagt cagactcatc aagcttctct 5460

atcaaagcag taagtagtac atgtaatgca acctataata gtagcaatag tagcattagt 5520

agtagcaata ataatagcaa tagttgtgtg gtccatagta atcatagaat ataggaaaat 5580

ggccgctgat cttcagacct ggaggaggag atatgaggga caattggaga agtgaattat 5640

ataaatataa agtagtaaaa attgaaccat taggagtagc acccaccaag gcaaagagaa 5700

gagtggtgca gagagaaaaa agagcagtgg gaataggagc tttgttcctt gggttcttgg 5760

gagcagcagg aagcactatg ggcgcagcgt caatgacgct gacggtacag gccagacaat 5820

tattgtctgg tatagtgcag cagcagaaca atttgctgag ggctattgag gcgcaacagc 5880

atctgttgca actcacagtc tggggcatca agcagctcca ggcaagaatc ctggctgtgg 5940

aaagatacct aaaggatcaa cagctcctgg ggatttgggg ttgctctgga aaactcattt 6000

gcaccactgc tgtgccttgg aatgctagtt ggagtaataa atctctggaa cagatttgga 6060

atcacacgac ctggatggag tgggacagag aaattaacaa ttacacaagc ttaatacact 6120

ccttaattga agaatcgcaa aaccagcaag aaaagaatga acaagaatta ttggaattag 6180

ataaatgggc aagtttgtgg aattggttta acataacaaa ttggctgtgg tatataaaat 6240

tattcataat gatagtagga ggcttggtag gtttaagaat agtttttgct gtactttcta 6300

tagtgaatag agttaggcag ggatattcac cattatcgtt tcagacccac ctcccaaccc 6360

cgaggggacc cgacaggccc gaaggaatag aagaagaagg tggagagaga gacagagaca 6420

gatccattcg attagtgaac ggatccttgg cacttatctg ggacgatctg cggagcctgt 6480

gcctcttcag ctaccaccgc ttgagagact tactcttgat tgtaacgagg attgtggaac 6540

ttctgggacg cagggggtgg gaagccctca aatattggtg gaatctccta caatattgga 6600

gtcaggagct aaagaatagt gctgttagct tgctcaatgc cacagccata gcagtagctg 6660

aggggacaga tagggttata gaagtagtac aaggagcttg tagagctatt cgccacatac 6720

ctagaagaat aagacagggc ttggaaagga ttttgctata agctcgaggc cgccccggtg 6780

accttcagac cttggcactg gaggtggccc ggcagaagcg cggcatcgtg gatcagtgct 6840

gcaccagcat ctgctctctc taccaactgg agaactactg caactaggcc caccactacc 6900

ctgtccaccc ctctgcaatg aataaaacct ttgaaagagc actacaagtt gtgtgtacat 6960

gcgtgcatgt gcatatgtgg tgcgggggga acatgagtgg ggctggctgg agtggcgatg 7020

ataagctgtc aaacatgaga attaattctt gaagacgaaa gggcctcgtg atacgcctat 7080

ttttataggt taatgtcatg ataataatgg tttcttagtc tagaattaat tccgtgtatt 7140

ctatagtgtc acctaaatcg tatgtgtatg atacataagg ttatgtatta attgtagccg 7200

cgttctaacg acaatatgta caagcctaat tgtgtagcat ctggcttact gaagcagacc 7260

ctatcatctc tctcgtaaac tgccgtcaga gtcggtttgg ttggacgaac cttctgagtt 7320

tctggtaacg ccgtcccgca cccggaaatg gtcagcgaac caatcagcag ggtcatcgct 7380

agccagatcc tctacgccgg acgcatcgtg gccggcatca ccggcgccac aggtgcggtt 7440

gctggcgcct atatcgccga catcaccgat ggggaagatc gggctcgcca cttcgggctc 7500

atgagcgctt gtttcggcgt gggtatggtg gcaggccccg tggccggggg actgttgggc 7560

gccatctcct tgcatgcacc attccttgcg gcggcggtgc tcaacggcct caacctacta 7620

ctgggctgct tcctaatgca ggagtcgcat aagggagagc gtcgaatggt gcactctcag 7680

tacaatctgc tctgatgccg catagttaag ccagccccga cacccgccaa cacccgctga 7740

cgcgccctga cgggcttgtc tgctcccggc atccgcttac agacaagctg tgaccgtctc 7800

cgggagctgc atgtgtcaga ggttttcacc gtcatcaccg aaacgcgcga gacgaaaggg 7860

cctcgtgata cgcctatttt tataggttaa tgtcatgata ataatggttt cttagacgtc 7920

aggtggcact tttcggggaa atgtgcgcgg aacccctatt tgtttatttt tctaaataca 7980

ttcaaatatg tatccgctca tgagacaata accctgataa atgcttcaat aatattgaaa 8040

aaggaagagt atgagtattc aacatttccg tgtcgccctt attccctttt ttgcggcatt 8100

ttgccttcct gtttttgctc acccagaaac gctggtgaaa gtaaaagatg ctgaagatca 8160

gttgggtgca cgagtgggtt acatcgaact ggatctcaac agcggtaaga tccttgagag 8220

ttttcgcccc gaagaacgtt ttccaatgat gagcactttt aaagttctgc tatgtggcgc 8280

ggtattatcc cgtattgacg ccgggcaaga gcaactcggt cgccgcatac actattctca 8340

gaatgacttg gttgagtact caccagtcac agaaaagcat cttacggatg gcatgacagt 8400

aagagaatta tgcagtgctg ccataaccat gagtgataac actgcggcca acttacttct 8460

gacaacgatc ggaggaccga aggagctaac cgcttttttg cacaacatgg gggatcatgt 8520

aactcgcctt gatcgttggg aaccggagct gaatgaagcc ataccaaacg acgagcgtga 8580

caccacgatg cctgtagcaa tggcaacaac gttgcgcaaa ctattaactg gcgaactact 8640

tactctagct tcccggcaac aattaataga ctggatggag gcggataaag ttgcaggacc 8700

acttctgcgc tcggcccttc cggctggctg gtttattgct gataaatctg gagccggtga 8760

gcgtgggtct cgcggtatca ttgcagcact ggggccagat ggtaagccct cccgtatcgt 8820

agttatctac acgacgggga gtcaggcaac tatggatgaa cgaaatagac agatcgctga 8880

gataggtgcc tcactgatta agcattggta actgtcagac caagtttact catatatact 8940

ttagattgat ttaaaacttc atttttaatt taaaaggatc taggtgaaga tcctttttga 9000

taatctcatg accaaaatcc cttaacgtga gttttcgttc cactgagcgt cagaccccgt 9060

agaaaagatc aaaggatctt cttgagatcc tttttttctg cgcgtaatct gctgcttgca 9120

aacaaaaaaa ccaccgctac cagcggtggt ttgtttgccg gatcaagagc taccaactct 9180

ttttccgaag gtaactggct tcagcagagc gcagatacca aatactgttc ttctagtgta 9240

gccgtagtta ggccaccact tcaagaactc tgtagcaccg cctacatacc tcgctctgct 9300

aatcctgtta ccagtggctg ctgccagtgg cgataagtcg tgtcttaccg ggttggactc 9360

aagacgatag ttaccggata aggcgcagcg gtcgggctga acggggggtt cgtgcacaca 9420

gcccagcttg gagcgaacga cctacaccga actgagatac ctacagcgtg agctatgaga 9480

aagcgccacg cttcccgaag ggagaaaggc ggacaggtat ccggtaagcg gcagggtcgg 9540

aacaggagag cgcacgaggg agcttccagg gggaaacgcc tggtatcttt atagtcctgt 9600

cgggtttcgc cacctctgac ttgagcgtcg atttttgtga tgctcgtcag gggggcggag 9660

cctatggaaa aacgccagca acgcggcctt tttacggttc ctggcctttt gctggccttt 9720

tgctcacatg ttctttcctg cgttatcccc tgattctgtg gataaccgta ttaccgcctt 9780

tgagtgagct gataccgctc gccgcagccg aacgaccgag cgcagcgagt cagtgagcga 9840

ggaagcggaa gagcgcccaa tacgcaaacc gcctctcccc gcgcgttggc cgattcatta 9900

atgcagctgt ggaatgtgtg tcagttaggg tgtggaaagt ccccaggctc cccagcaggc 9960

agaagtatgc aaagcatgca tctcaattag tcagcaacca ggtgtggaaa gtccccaggc 10020

tccccagcag gcagaagtat gcaaagcatg catctcaatt agtcagcaac catagtcccg 10080

cccctaactc cgcccatccc gcccctaact ccgcccagtt ccgcccattc tccgccccat 10140

ggctgactaa ttttttttat ttatgcagag gccgaggccg cctcggcctc tgagctattc 10200

cagaagtagt gaggaggctt ttttggaggc ctaggctttt gcaaaaagct tggacacaag 10260

acaggcttgc gagatatgtt tgagaatacc actttatccc gcgtcaggga gaggcagtgc 10320

gtaaaaagac gcggactcat gtgaaatact ggtttttagt gcgccagatc tctataatct 10380

cgcgcaacct attttcccct cgaacacttt ttaagccgta gataaacagg ctgggacact 10440

tcacatgagc gaaaaataca tcgtcacctg ggacatgttg cagatccatg cacgtaaact 10500

cgcaagccga ctgatgcctt ctgaacaatg gaaaggcatt attgccgtaa gccgtggcgg 10560

tctgtaccgg gtgcgttact ggcgcgtgaa ctgggtattc gtcatgtcga taccgtttgt 10620

atttccagct acgatcacga caaccagcgc gagcttaaag tgctgaaacg cgcagaaggc 10680

gatggcgaag gcttcatcgt tattgatgac ctggtggata ccggtggtac tgcggttgcg 10740

attcgtgaaa tgtatccaaa agcgcacttt gtcaccatct tcgcaaaacc ggctggtcgt 10800

ccgctggttg atgactatgt tgttgatatc ccgcaagata cctggattga acagccgtgg 10860

gatatgggcg tcgtattcgt cccgccaatc tccggtcgct aatcttttca acgcctggca 10920

ctgccgggcg ttgttctttt taacttcagg cgggttacaa tagtttccag taagtattct 10980

ggaggctgca tccatgacac aggcaaacct gagcgaaacc ctgttcaaac cccgctttaa 11040

acatcctgaa acctcgacgc tagtccgccg ctttaatcac ggcgcacaac cgcctgtgca 11100

gtcggccctt gatggtaaaa ccatccctca ctggtatcgc atgattaacc gtctgatgtg 11160

gatctggcgc ggcattgacc cacgcgaaat cctcgacgtc caggcacgta ttgtgatgag 11220

cgatgccgaa cgtaccgacg atgatttata cgatacggtg attggctacc gtggcggcaa 11280

ctggatttat gagtgggccc cggatctttg tgaaggaacc ttacttctgt ggtgtgacat 11340

aattggacaa actacctaca gagatttaaa gctctaaggt aaatataaaa tttttaaccc 11400

ggatctttgt gaaggaacct tacttctgtg gtgtgacata attggacaaa ctacctacag 11460

agatttaaag ctctaaggta aatataaaat ttttaagtgt ataatgtgtt aaactactga 11520

ttctaattgt ttgtgtattt tagattccaa cctatggaac tgatgaatgg gagcagtggt 11580

ggaatgcctt taatgaggaa aacctgtttt gctcagaaga aatgccatct agtgatgatg 11640

aggctactgc tgactctcaa cattctactc ctccaaaaaa gaagagaaag gtagaagacc 11700

ccaaggactt tccttcagaa ttgctaagtt ttttgagtca tgctgtgttt agtaatagaa 11760

ctcttgcttg ctttgctatt tacaccacaa aggaaaaagc tgcactgcta tacaagaaaa 11820

ttatggaaaa atattctgta acctttataa gtaggcataa cagttataat cataacatac 11880

tgttttttct tactccacac aggcatagag tgtctgctat taataactat gctcaaaaat 11940

tgtgtacctt tagcttttta atttgtaaag gggttaataa ggaatatttg atgtatagtg 12000

ccttgactag agatcataat cagccatacc acatttgtag aggttttact tgctttaaaa 12060

aacctcccac acctccccct gaacctgaaa cataaaatga atgcaattgt tgttgttggg 12120

ctgcaggaat taattcgagc tcgcccgaca 12150

<210> 13
<211> 11520
<212> DNA
<213> Artificial sequence

<220>
<223> SEQ ID NO: 13

<400> 13
ccagatcctc tacgccggac gcatcgtggc cggcatcacc ggcgccacag gtgcggttgc 60

tggcgcctat atcgccgaca tcaccgatgg ggaagatcgg gctcgccact tcgggctcat 120

gagcgcttgt ttcggcgtgg gtatggtggc aggccccgtg gccgggggac tgttgggcgc 180

catctccttg catgcaccat tccttgcggc ggcggtgctc aacggcctca acctactact 240

gggctgcttc ctaatgcagg agtcgcataa gggagagcgt cgaatggtgc actctcagta 300

caatctgctc tgatgccgca tagttaagcc agccccgaca cccgccaaca cccgctgacg 360

cgccctgacg ggcttgtctg ctcccggcat ccgcttacag acaagctgtg accgtctccg 420

ggagctgcat gtgtcagagg ttttcaccgt catcaccgaa acgcgcgaga cgaaagggcc 480

tcgtgatacg cctattttta taggttaatg tcatgataat aatggtttct tagacgtcag 540

gtggcacttt tcggggaaat gtgcgcggaa cccctatttg tttatttttc taaatacatt 600

caaatatgta tccgctcatg agacaataac cctgataaat gcttcaataa tattgaaaaa 660

ggaagagtat gagtattcaa catttccgtg tcgcccttat tccctttttt gcggcatttt 720

gccttcctgt ttttgctcac ccagaaacgc tggtgaaagt aaaagatgct gaagatcagt 780

tgggtgcacg agtgggttac atcgaactgg atctcaacag cggtaagatc cttgagagtt 840

ttcgccccga agaacgtttt ccaatgatga gcacttttaa agttctgcta tgtggcgcgg 900

tattatcccg tattgacgcc gggcaagagc aactcggtcg ccgcatacac tattctcaga 960

atgacttggt tgagtactca ccagtcacag aaaagcatct tacggatggc atgacagtaa 1020

gagaattatg cagtgctgcc ataaccatga gtgataacac tgcggccaac ttacttctga 1080

caacgatcgg aggaccgaag gagctaaccg cttttttgca caacatgggg gatcatgtaa 1140

ctcgccttga tcgttgggaa ccggagctga atgaagccat accaaacgac gagcgtgaca 1200

ccacgatgcc tgtagcaatg gcaacaacgt tgcgcaaact attaactggc gaactactta 1260

ctctagcttc ccggcaacaa ttaatagact ggatggaggc ggataaagtt gcaggaccac 1320

ttctgcgctc ggcccttccg gctggctggt ttattgctga taaatctgga gccggtgagc 1380

gtgggtctcg cggtatcatt gcagcactgg ggccagatgg taagccctcc cgtatcgtag 1440

ttatctacac gacggggagt caggcaacta tggatgaacg aaatagacag atcgctgaga 1500

taggtgcctc actgattaag cattggtaac tgtcagacca agtttactca tatatacttt 1560

agattgattt aaaacttcat ttttaattta aaaggatcta ggtgaagatc ctttttgata 1620

atctcatgac caaaatccct taacgtgagt tttcgttcca ctgagcgtca gaccccgtag 1680

aaaagatcaa aggatcttct tgagatcctt tttttctgcg cgtaatctgc tgcttgcaaa 1740

caaaaaaacc accgctacca gcggtggttt gtttgccgga tcaagagcta ccaactcttt 1800

ttccgaaggt aactggcttc agcagagcgc agataccaaa tactgtcctt ctagtgtagc 1860

cgtagttagg ccaccacttc aagaactctg tagcaccgcc tacatacctc gctctgctaa 1920

tcctgttacc agtggctgct gccagtggcg ataagtcgtg tcttaccggg ttggactcaa 1980

gacgatagtt accggataag gcgcagcggt cgggctgaac ggggggttcg tgcacacagc 2040

ccagcttgga gcgaacgacc tacaccgaac tgagatacct acagcgtgag cattgagaaa 2100

gcgccacgct tcccgaaggg agaaaggcgg acaggtatcc ggtaagcggc agggtcggaa 2160

caggagagcg cacgagggag cttccagggg gaaacgcctg gtatctttat agtcctgtcg 2220

ggtttcgcca cctctgactt gagcgtcgat ttttgtgatg ctcgtcaggg gggcggagcc 2280

tatggaaaaa cgccagcaac gcggcctttt tacggttcct ggccttttgc tggccttttg 2340

ctcacatgtt ctttcctgcg ttatcccctg attctgtgga taaccgtatt accgcctttg 2400

agtgagctga taccgctcgc cgcagccgaa cgaccgagcg cagcgagtca gtgagcgagg 2460

aagcggaaga gcgcccaata cgcaaaccgc ctctccccgc gcgttggccg attcattaat 2520

gcagctgtgg aatgtgtgtc agttagggtg tggaaagtcc ccaggctccc cagcaggcag 2580

aagtatgcaa agcatgcatc tcaattagtc agcaaccagg tgtggaaagt ccccaggctc 2640

cccagcaggc agaagtatgc aaagcatgca tctcaattag tcagcaacca tagtcccgcc 2700

cctaactccg cccatcccgc ccctaactcc gcccagttcc gcccattctc cgccccatgg 2760

ctgactaatt ttttttattt atgcagaggc cgaggccgcc tcggcctctg agctattcca 2820

gaagtagtga ggaggctttt ttggaggcct aggcttttgc aaaaagcttg gacacaagac 2880

aggcttgcga gatatgtttg agaataccac tttatcccgc gtcagggaga ggcagtgcgt 2940

aaaaagacgc ggactcatgt gaaatactgg tttttagtgc gccagatctc tataatctcg 3000

cgcaacctat tttcccctcg aacacttttt aagccgtaga taaacaggct gggacacttc 3060

acatgagcga aaaatacatc gtcacctggg acatgttgca gatccatgca cgtaaactcg 3120

caagccgact gatgccttct gaacaatgga aaggcattat tgccgtaagc cgtggcggtc 3180

tgtaccgggt gcgttactgg cgcgtgaact gggtattcgt catgtcgata ccgtttgtat 3240

ttccagctac gatcacgaca accagcgcga gcttaaagtg ctgaaacgcg cagaaggcga 3300

tggcgaaggc ttcatcgtta ttgatgacct ggtggatacc ggtggtactg cggttgcgat 3360

tcgtgaaatg tatccaaaag cgcactttgt caccatcttc gcaaaaccgg ctggtcgtcc 3420

gctggttgat gactatgttg ttgatatccc gcaagatacc tggattgaac agccgtggga 3480

tatgggcgtc gtattcgtcc cgccaatctc cggtcgctaa tcttttcaac gcctggcact 3540

gccgggcgtt gttcttttta acttcaggcg ggttacaata gtttccagta agtattctgg 3600

aggctgcatc catgacacag gcaaacctga gcgaaaccct gttcaaaccc cgctttaaac 3660

atcctgaaac ctcgacgcta gtccgccgct ttaatcacgg cgcacaaccg cctgtgcagt 3720

cggcccttga tggtaaaacc atccctcact ggtatcgcat gattaaccgt ctgatgtgga 3780

tctggcgcgg cattgaccca cgcgaaatcc tcgacgtcca ggcacgtatt gtgatgagcg 3840

atgccgaacg taccgacgat gatttatacg atacggtgat tggctaccgt ggcggcaact 3900

ggatttatga gtgggccccg gatctttgtg aaggaacctt acttctgtgg tgtgacataa 3960

ttggacaaac tacctacaga gatttaaagc tctaaggtaa atataaaatt tttaagtgta 4020

taatgtgtta aactactgat tctaattgtt tgtgtatttt agattccaac ctatggaact 4080

gatgaatggg agcagtggtg gaatgccttt aatgaggaaa acctgttttg ctcagaagaa 4140

atgccatcta gtgatgatga ggctactgct gactctcaac attctactcc tccaaaaaag 4200

aagagaaagg tagaagaccc caaggacttt ccttcagaat tgctaagttt tttgagtcat 4260

gctgtgttta gtaatagaac tcttgcttgc tttgctattt acaccacaaa ggaaaaagct 4320

gcactgctat acaagaaaat tatggaaaaa tattctgtaa cctttataag taggcataac 4380

agttataatc ataacatact gttttttctt actccacaca ggcatagagt gtctgctatt 4440

aataactatg ctcaaaaatt gtgtaccttt agctttttaa tttgtaaagg ggttaataag 4500

gaatatttga tgtatagtgc cttgactaga gatcataatc agccatacca catttgtaga 4560

ggttttactt gctttaaaaa acctcccaca cctccccctg aacctgaaac ataaaatgaa 4620

tgcaattgtt gttgttaact tgtttattgc agcttataat ggttacaaat aaagcaatag 4680

catcacaaat ttcacaaata aagcattttt ttcactgcat tctagttgtg gtttgtccaa 4740

actcatcaat gtatcttatc atgtctggat caactggata actcaagcta accaaaatca 4800

tcccaaactt cccaccccat accctattac cactgccaat tacctagtgg tttcatttac 4860

tctaaacctg tgattcctct gaattatttt cattttaaag aaattgtatt tgttaaatat 4920

gtactacaaa cttagtagtt ggaagggcta attcactccc aacgaagaca agatatcctt 4980

gatctgtgga tctaccacac acaaggctac ttccctgatt agcagaacta cacaccaggg 5040

ccagggatca gatatccact gacctttgga tggtgctaca agctagtacc agttgagcca 5100

gagaagttag aagaagccaa caaaggagag aacaccagct tgttacaacc tgtgagcctg 5160

catgggatgg atgacccgga gagagaagtg ttagagtgga ggtttgacag ccgcctagca 5220

tttcatcacg gtggcccgag agctgcatcc ggagtacttc aagaactgct gatatcgagc 5280

ttgctacaag ggactttccg ctggggactt tccagggagg cgtggcctgg gcgggactg 5340

gggagtggcg agccctcaga tcctgcatat aagcagctgc tttttgcctg tactgggtct 5400

ctctggttag accagatctg agcctgggag ctctctggct aactagggaa cccactgctt 5460

aagcctcaat aaagcttgcc ttgagtgctt caagtagtgt gtgcccgtct gttgtgtgac 5520

tctggtaact agagatccct cagacccttt tagtcagtgt ggaaaatctc tagcagttga 5580

aagcgaaagg gaaaccagag gagctctctc gacgcaggac tcggcttgct gaagcgcgca 5640

cggcaagagg cgaggggcgg cgactggtga gtacgccaaa aattttgact agcggaggct 5700

agaaggagag agatgggtgc gagagcgtca gtattaagcg ggggagaatt agatcgcgat 5760

gggaaaaaat tcggttaagg ccagggggaa agaaaaaata taaattaaaa catatagtat 5820

gggcaagcag ggagctagaa cgattcgcag ttaatcctgg cctgttagaa acatcagaag 5880

gctgtagaca aatactggga cagctacaac catcccttca gacaggatca gaagaactta 5940

gatcattata taatacagta gcaaccctct attgtgtgca tcaaaggata gagataaaag 6000

acaccaagga agctttagac aagatagagg aagagcaaaa caaaagtaag accaccgcac 6060

agcaagcggc cgctgatctt cagacctgga ggaggagata tgagggacaa ttggagaagt 6120

gaattatata aatataaagt agtaaaaatt gaaccattag gagtagcacc caccaaggca 6180

aagagaagag tggtgcagag agaaaaaaga gcagtgggaa taggagcttt gttccttggg 6240

ttcttgggag cagcaggaag cactatgggc gcagcgtcaa tgacgctgac ggtacaggcc 6300

agacaattat tgtctggtat agtgcagcag cagaacaatt tgctgagggc tattgaggcg 6360

caacagcatc tgttgcaact cacagtctgg ggcatcaagc agctccaggc aagaatcctg 6420

gctgtggaaa gatacctaaa ggatcaacag ctcctgggga tttggggttg ctctggaaaa 6480

ctcatttgca ccactgctgt gccttggaat gctagttgga gtaataaatc tctggaacag 6540

atttggaatc acacgacctg gatggagtgg gacagagaaa ttaacaatta cacaagctta 6600

atacactcct taattgaaga atcgcaaaac cagcaagaaa agaatgaaca agaattattg 6660

gaattagata aatgggcaag tttgtggaat tggtttaaca taacaaattg gctgtggtat 6720

ataaaattat tcataatgat agtaggaggc ttggtaggtt taagaatagt ttttgctgta 6780

ctttctatag tgaatagagt taggcaggga tattcaccat tatcgtttca gacccacctc 6840

ccaaccccga ggggacccga caggcccgaa ggaatagaag aagaaggtgg agagagagac 6900

agagacagat ccattcgatt agtgaacgga tctcgacggt atcgccgaat tcacaaatgg 6960

cagtattcat ccacaatttt aaaagaaaag gggggattgg ggggtacagt gcaggggaaa 7020

gaatagtaga cataatagca acagacatac aaactaaaga attacaaaaa caaattacaa 7080

aaattcaaaa ttttcgggtt tattacaggg acagcagaga tccactttgg ctgatacgcg 7140

gatctacgcg tggatccgcg gcccgggtca ccatctagat ggccatatga tcgatacgta 7200

ctagtcgtag ggtcgccgac atgacacaag gggttgtgac cggggtggac acgtacgcgg 7260

gtgcttacga ccgtcagtcg cgcgagcgcg agaattcgag cgcagcaagc ccagcgacac 7320

agcgtagcgc caacgaagac aaggcggccg accttcagcg cgaagtcgag cgcgacgggg 7380

gccggttcag gttcgtcggg catttcagcg aagcgccggg cacgtcggcg ttcgggacgg 7440

cggagcgccc ggagttcgaa cgcatcctga acgaatgccg cgccgggcgg ctcaacatga 7500

tcattgtcta tgacgtgtcg cgcttctcgc gcctgaaggt catggacgcg attccgattg 7560

tctcggaatt gctcgccctg ggcgtgacga ttgtttccac tcaggaaggc gtcttccggc 7620

agggaaacgt catggacctg attcacctga ttatgcggct cgacgcgtcg cacaaagaat 7680

cttcgctgaa gtcggcgaag attctcgaca cgaagaacct tcagcgcgaa ttgggcgggt 7740

acgtcggcgg gaaggcgcct tacggcttcg agcttgtttc ggagacgaag gagatcacgc 7800

gcaacggccg aatggtcaat gtcgtcatca acaagcttgc gcactcgacc actcccctta 7860

ccggaccctt cgagttcgag cccgacgtaa tccggtggtg gtggcgtgag atcaagacgc 7920

acaaacacct tcccttcaag ccgggcagtc aagccgccat tcacccgggc agcatcacgg 7980

ggctttgtaa gcgcatggac gctgacgccg tgccgacccg gggcgagacg attgggaaga 8040

agaccgcttc aagcgcctgg gacccggcaa ccgttatgcg aatccttcgg gacccgcgta 8100

ttgcgggctt cgccgctgag gtgatctaca agaagaagcc ggacggcacg ccgaccacga 8160

agattgaggg ttaccgcatt cagcgcgacc cgatcacgct ccggccggtc gagcttgatt 8220

gcggaccgat catcgagccc gctgagtggt atgagcttca ggcgtggttg gacggcaggg 8280

ggcgcggcaa ggggctttcc cgggggcaag ccattctgtc cgccatggac aagctgtact 8340

gcgagtgtgg cgccgtcatg acttcgaagc gcggggaaga atcgatcaag gactcttacc 8400

gctgccgtcg ccggaaggtg gtcgacccgt ccgcacctgg gcagcacgaa ggcacgtgca 8460

acgtcagcat ggcggcactc gacaagttcg ttgcggaacg catcttcaac aagatcaggc 8520

acgccgaagg cgacgaagag acgttggcgc ttctgtggga agccgcccga cgcttcggca 8580

agctcactga ggcgcctgag aagagcggcg aacgggcgaa ccttgttgcg gagcgcgccg 8640

acgccctgaa cgcccttgaa gagctgtacg aagaccgcgc ggcaggcgcg tacgacggac 8700

ccgttggcag gaagcacttc cggaagcaac aggcagcgct gacgctccgg cagcaagggg 8760

cggaagagcg gcttgccgaa cttgaagccg ccgaagcccc gaagcttccc cttgaccaat 8820

ggttccccga agacgccgac gctgacccga ccggccctaa gtcgtggtgg gggcgcgcgt 8880

cagtagacga caagcgcgtg ttcgtcgggc tcttcgtaga caagatcgtt gtcacgaagt 8940

cgactacggg cagggggcag ggaacgccca tcgagaagcg cgcttcgatc acgtgggcga 9000

agccgccgac cgacgacgac gaagacgacg cccaggacgg cacggaagac gtagcggcgc 9060

ctaagaagaa gaggaaggtt tagcggatcc ctcgagctca agcttcgaat tcccgataat 9120

caacctctgg attacaaaat ttgtgaaaga ttgactggta ttcttaacta tgttgctcct 9180

tttacgctat gtggatacgc tgctttaatg cctttgtatc atgctattgc ttcccgtatg 9240

gctttcattt tctcctcctt gtataaatcc tggttgctgt ctctttatga ggagttgtgg 9300

cccgttgtca ggcaacgtgg cgtggtgtgc actgtgtttg ctgacgcaac ccccactggt 9360

tggggcattg ccaccacctg tcagctcctt tccgggactt tcgctttccc cctccctatt 9420

gccacggcgg aactcatcgc cgcctgcctt gcccgctgct ggacaggggc tcggctgttg 9480

ggcactgaca attccgtggt gttgtcgggg aagctgacgt cctttccatg gctgctcgcc 9540

tgtgttgcca cctggattct gcgcgggacg tccttctgct acgtcccttc ggccctcaat 9600

ccagcggacc ttccttcccg cggcctgctg ccggctctgc ggcctcttcc gcgtcttcgc 9660

cttcgccctc agacgagtcg gatctccctt tgggccgcct ccccgcatcg ggaattctgc 9720

agtcgacggt acctttaaga ccaatgactt acaaggcagc tgtagatctt agccactttt 9780

taaaagaaaa ggggggactg gaagggctaa ttcactccca acgaagacaa aatcgtcgag 9840

agatgctgca tataagcagc tgctttttgc ttgtactggg tctctctggt tagaccagat 9900

ctgagcctgg gagctctctg gctaactagg gaacccactg cttaagcctc aataaagctt 9960

gccttgagtg cttcaagtag tgtgtgcccg tctgttgtgt gactctggta actagagatc 10020

cctcagaccc ttttagtcag tgtggaaaat ctctagcagt agtagttcat gtcatcttat 10080

tattcagtat ttataacttg caaagaaatg aatatcagag agtgagaggc cttgacatta 10140

taatagattt agcaggaatt gaactaggag tggagcacac aggcaaagct gcagaagtac 10200

ttggaagaag ccaccagaga tactcacgat tctgcacata cctggctaat cccagatcct 10260

aaggattaca ttaagtttac taacatttat ataatgattt atagtttaaa gtataaactt 10320

atctaattta ctattctgac agatattaat taatcctcaa atatcataag agatgattac 10380

tattatcccc atttaacaca agaggaaact gagagggaaa gatgttgaag taattttccc 10440

acaattacag catccgttag ttacgactct atgatcttct gacacaaatt ccatttactc 10500

ctcaccctat gactcagtcg aatatatcaa agttatggac attatgctaa gtaacaaatt 10560

acccttttat atagtaaata ctgagtagat tgagagaaga aattgtttgc aaacctgaat 10620

agcttcaaga agaagagaag tgaggataag aataacagtt gtcatttaac aagttttaac 10680

aagtaacttg gttagaaagg gattcaaatg cataaagcaa gggataaatt tttctggcaa 10740

caagactata caatataacc ttaaatatga cttcaaataa ttgttggaac ttgataaaac 10800

taattaaata ttattgaaga ttatcaatat tataaatgta atttactttt aaaaagggaa 10860

catagaaatg tgtatcatta gagtagaaaa caatccttat tatcacaatt tgtcaaaaca 10920

agtttgttat taacacaagt agaatactgc attcaattaa gttgactgca gattttgtgt 10980

tttgttaaaa ttagaaagag ataacaacaa tttgaattat tgaaagtaac atgtaaatag 11040

ttctacatac gttcttttga catcttgttc aatcattgat cgaagttctt tatcttggaa 11100

gaatttgttc caaagactct gaaataagga aaacaatcta ttatatagtc tcacaccttt 11160

gttttacttt tagtgatttc aatttaataa tgtaaatggt taaaatttat tcttctctga 11220

gatcatttca cattgcagat agaaaacctg agactggggt aatttttatt aaaatctaat 11280

ttaatctcag aaacacatct ttattctaac atcaattttt ccagtttgat attatcatat 11340

aaagtcagcc ttcctcatct gcaggttcca caacaaaaat ccaaccaact gtggatcaaa 11400

aatattggga aaaaattaaa aatagcaata caacaataaa aaaatacaaa tcagaaaaac 11460

agcacagtat aacaacttta tttagcattt acaatctatt aggtattata agtaatctag 11520

<210> 14
<211> 10711
<212> DNA
<213> Artificial sequence

<220>
<223> SEQ ID NO: 14

<400> 14
   ccagatcctc tacgccggac gcatcgtggc cggcatcacc ggcgccacag gtgcggttgc 60
   tggcgcctat atcgccgaca tcaccgatgg ggaagatcgg gctcgccact tcgggctcat 120
   gagcgcttgt ttcggcgtgg gtatggtggc aggccccgtg gccgggggac tgttgggcgc 180
   catctccttg catgcaccat tccttgcggc ggcggtgctc aacggcctca acctactact 240
   gggctgcttc ctaatgcagg agtcgcataa gggagagcgt cgaatggtgc actctcagta 300
   caatctgctc tgatgccgca tagttaagcc agccccgaca cccgccaaca cccgctgacg 360
   cgccctgacg ggcttgtctg ctcccggcat ccgcttacag acaagctgtg accgtctccg 420
   ggagctgcat gtgtcagagg ttttcaccgt catcaccgaa acgcgcgaga cgaaagggcc 480
   tcgtgatacg cctattttta taggttaatg tcatgataat aatggtttct tagacgtcag 540
   gtggcacttt tcggggaaat gtgcgcggaa cccctatttg tttatttttc taaatacatt 600
   caaatatgta tccgctcatg agacaataac cctgataaat gcttcaataa tattgaaaaa 660
   ggaagagtat gagtattcaa catttccgtg tcgcccttat tccctttttt gcggcatttt 720
   gccttcctgt ttttgctcac ccagaaacgc tggtgaaagt aaaagatgct gaagatcagt 780
   tgggtgcacg agtgggttac atcgaactgg atctcaacag cggtaagatc cttgagagtt 840
   ttcgccccga agaacgtttt ccaatgatga gcacttttaa agttctgcta tgtggcgcgg 900
   tattatcccg tattgacgcc gggcaagagc aactcggtcg ccgcatacac tattctcaga 960
   atgacttggt tgagtactca ccagtcacag aaaagcatct tacggatggc atgacagtaa 1020
   gagaattatg cagtgctgcc ataaccatga gtgataacac tgcggccaac ttacttctga 1080
   caacgatcgg aggaccgaag gagctaaccg cttttttgca caacatgggg gatcatgtaa 1140
   ctcgccttga tcgttgggaa ccggagctga atgaagccat accaaacgac gagcgtgaca 1200
   ccacgatgcc tgtagcaatg gcaacaacgt tgcgcaaact attaactggc gaactactta 1260
   ctctagcttc ccggcaacaa ttaatagact ggatggaggc ggataaagtt gcaggaccac 1320
   ttctgcgctc ggcccttccg gctggctggt ttattgctga taaatctgga gccggtgagc 1380
   gtgggtctcg cggtatcatt gcagcactgg ggccagatgg taagccctcc cgtatcgtag 1440
   ttatctacac gacggggagt caggcaacta tggatgaacg aaatagacag atcgctgaga 1500
   taggtgcctc actgattaag cattggtaac tgtcagacca agtttactca tatatacttt 1560
   agattgattt aaaacttcat ttttaattta aaaggatcta ggtgaagatc ctttttgata 1620
   atctcatgac caaaatccct taacgtgagt tttcgttcca ctgagcgtca gaccccgtag 1680
   aaaagatcaa aggatcttct tgagatcctt tttttctgcg cgtaatctgc tgcttgcaaa 1740
   caaaaaaacc accgctacca gcggtggttt gtttgccgga tcaagagcta ccaactcttt 1800
   ttccgaaggt aactggcttc agcagagcgc agataccaaa tactgtcctt ctagtgtagc 1860
   cgtagttagg ccaccacttc aagaactctg tagcaccgcc tacatacctc gctctgctaa 1920
   tcctgttacc agtggctgct gccagtggcg ataagtcgtg tcttaccggg ttggactcaa 1980
   gacgatagtt accggataag gcgcagcggt cgggctgaac ggggggttcg tgcacacagc 2040
   ccagcttgga gcgaacgacc tacaccgaac tgagatacct acagcgtgag cattgagaaa 2100
   gcgccacgct tcccgaaggg agaaaggcgg acaggtatcc ggtaagcggc agggtcggaa 2160
   caggagagcg cacgagggag cttccagggg gaaacgcctg gtatctttat agtcctgtcg 2220
   ggtttcgcca cctctgactt gagcgtcgat ttttgtgatg ctcgtcaggg gggcggagcc 2280
   tatggaaaaa cgccagcaac gcggcctttt tacggttcct ggccttttgc tggccttttg 2340
   ctcacatgtt ctttcctgcg ttatcccctg attctgtgga taaccgtatt accgcctttg 2400
   agtgagctga taccgctcgc cgcagccgaa cgaccgagcg cagcgagtca gtgagcgagg 2460
   aagcggaaga gcgcccaata cgcaaaccgc ctctccccgc gcgttggccg attcattaat 2520
   gcagctgtgg aatgtgtgtc agttagggtg tggaaagtcc ccaggctccc cagcaggcag 2580
   aagtatgcaa agcatgcatc tcaattagtc agcaaccagg tgtggaaagt ccccaggctc 2640
   cccagcaggc agaagtatgc aaagcatgca tctcaattag tcagcaacca tagtcccgcc 2700
   cctaactccg cccatcccgc ccctaactcc gcccagttcc gcccattctc cgccccatgg 2760
   ctgactaatt ttttttattt atgcagaggc cgaggccgcc tcggcctctg agctattcca 2820
   gaagtagtga ggaggctttt ttggaggcct aggcttttgc aaaaagcttg gacacaagac 2880
   aggcttgcga gatatgtttg agaataccac tttatcccgc gtcagggaga ggcagtgcgt 2940
   aaaaagacgc ggactcatgt gaaatactgg tttttagtgc gccagatctc tataatctcg 3000
   cgcaacctat ttcccctcga acactttttt aagccgtaga taaacaggct gggacacttc 3060
   acatgagcga aaaatacatc gtcacctggg acatgttgca gatccatgca cgtaaactcg 3120
   caagccgact gatgccttct gaacaatgga aaggcattat tgccgtaagc cgtggcggtc 3180
   tgtaccgggt gcgttactgg cgcgtgaact gggtattcgt catgtcgata ccgtttgtat 3240
   ttccagctac gatcacgaca accagcgcga gcttaaagtg ctgaaacgcg cagaaggcga 3300
   tggcgaaggc ttcatcgtta ttgatgacct ggtggatacc ggtggtactg cggttgcgat 3360
   tcgtgaaatg tatccaaaag cgcactttgt caccatcttc gcaaaaccgg ctggtcgtcc 3420
   gctggttgat gactatgttg ttgatatccc gcaagatacc tggattgaac agccgtggga 3480
   tatgggcgtc gtattcgtcc cgccaatctc cggtcgctaa tcttttcaac gcctggcact 3540
   gccgggcgtt gttcttttta acttcaggcg ggttacaata gtttccagta agtattctgg 3600
   aggctgcatc catgacacag gcaaacctga gcgaaaccct gttcaaaccc cgctttaaac 3660
   atcctgaaac ctcgacgcta gtccgccgct ttaatcacgg cgcacaaccg cctgtgcagt 3720
   cggcccttga tggtaaaacc atccctcact ggtatcgcat gattaaccgt ctgatgtgga 3780
   tctggcgcgg cattgaccca cgcgaaatcc tcgacgtcca ggcacgtatt gtgatgagcg 3840
   atgccgaacg taccgacgat gatttatacg atacggtgat tggctaccgt ggcggcaact 3900
   ggatttatga gtgggccccg gatctttgtg aaggaacctt acttctgtgg tgtgacataa 3960
   ttggacaaac tacctacaga gatttaaagc tctaaggtaa atataaaatt tttaagtgta 4020
   taatgtgtta aactactgat tctaattgtt tgtgtatttt agattccaac ctatggaact 4080
   gatgaatggg agcagtggtg gaatgccttt aatgaggaaa acctgttttg ctcagaagaa 4140
   atgccatcta gtgatgatga ggctactgct gactctcaac attctactcc tccaaaaaag 4200
   aagagaaagg tagaagaccc caaggacttt ccttcagaat tgctaagttt tttgagtcat 4260
   gctgtgttta gtaatagaac tcttgcttgc tttgctattt acaccacaaa ggaaaaagct 4320
   gcactgctat acaagaaaat tatggaaaaa tattctgtaa cctttataag taggcataac 4380
   agttataatc ataacatact gttttttctt actccacaca ggcatagagt gtctgctatt 4440
   aataactatg ctcaaaaatt gtgtaccttt agctttttaa tttgtaaagg ggttaataag 4500
   gaatatttga tgtatagtgc cttgactaga gatcataatc agccatacca catttgtaga 4560
   ggttttactt gctttaaaaa acctcccaca cctccccctg aacctgaaac ataaaatgaa 4620
   tgcaattgtt gttgttaact tgtttattgc agcttataat ggttacaaat aaagcaatag 4680
   catcacaaat ttcacaaata aagcattttt ttcactgcat tctagttgtg gtttgtccaa 4740
   actcatcaat gtatcttatc atgtctggat caactggata actcaagcta accaaaatca 4800
   tcccaaactt cccaccccat accctattac cactgccaat tacctagtgg tttcatttac 4860
   tctaaacctg tgattcctct gaattatttt cattttaaag aaattgtatt tgttaaatat 4920
   gtactacaaa cttagtagtt ggaagggcta attcactccc aacgaagaca agatatcctt 4980
   gatctgtgga tctaccacac acaaggctac ttccctgatt agcagaacta cacaccaggg 5040
   ccagggatca gatatccact gacctttgga tggtgctaca agctagtacc agttgagcca 5100
   gagaagttag aagaagccaa caaaggagag aacaccagct tgttacaacc tgtgagcctg 5160
   catgggatgg atgacccgga gagagaagtg ttagagtgga ggtttgacag ccgcctagca 5220
   tttcatcacg gtggcccgag agctgcatcc ggagtacttc aagaactgct gatatcgagc 5280
   ttgctacaag ggactttccg ctggggactt ttccagggag gcgtggcctg ggcgggactg 5340
   gggagtggcg agccctcaga tcctgcatat aagcagctgc tttttgcctg tactgggtct 5400
   ctctggttag accagatctg agcctgggag ctctctggct aactagggaa cccactgctt 5460
   aagcctcaat aaagcttgcc ttgagtgctt caagtagtgt gtgcccgtct gttgtgtgac 5520
   tctggtaact agagatccct cagacccttt tagtcagtgt ggaaaatctc tagcagttga 5580
   aagcgaaagg gaaaccagag gagctctctc gacgcaggac tcggcttgct gaagcgcgca 5640
   cggcaagagg cgaggggcgg cgactggtga gtacgccaaa aattttgact agcggaggct 5700
   agaaggagag agatgggtgc gagagcgtca gtattaagcg ggggagaatt agatcgcgat 5760
   gggaaaaaat tcggttaagg ccagggggaa agaaaaaata taaattaaaa catatagtat 5820
   gggcaagcag ggagctagaa cgattcgcag ttaatcctgg cctgttagaa acatcagaag 5880
   gctgtagaca aatactggga cagctacaac catcccttca gacaggatca gaagaactta 5940
   gatcattata taatacagta gcaaccctct attgtgtgca tcaaaggata gagataaaag 6000
   acaccaagga agctttagac aagatagagg aagagcaaaa caaaagtaag accaccgcac 6060
   agcaagcggc cgctgatctt cagacctgga ggaggagata tgagggacaa ttggagaagt 6120
   gaattatata aatataaagt agtaaaaatt gaaccattag gagtagcacc caccaaggca 6180
   aagagaagag tggtgcagag agaaaaaaga gcagtgggaa taggagcttt gttccttggg 6240
   ttcttgggag cagcaggaag cactatgggc gcagcgtcaa tgacgctgac ggtacaggcc 6300
   agacaattat tgtctggtat agtgcagcag cagaacaatt tgctgagggc tattgaggcg 6360
   caacagcatc tgttgcaact cacagtctgg ggcatcaagc agctccaggc aagaatcctg 6420
   gctgtggaaa gatacctaaa ggatcaacag ctcctgggga tttggggttg ctctggaaaa 6480
   ctcatttgca ccactgctgt gccttggaat gctagttgga gtaataaatc tctggaacag 6540
   atttggaatc acacgacctg gatggagtgg gacagagaaa ttaacaatta cacaagctta 6600
   atacactcct taattgaaga atcgcaaaac cagcaagaaa agaatgaaca agaattattg 6660
   gaattagata aatgggcaag tttgtggaat tggtttaaca taacaaattg gctgtggtat 6720
   ataaaattat tcataatgat agtaggaggc ttggtaggtt taagaatagt ttttgctgta 6780
   ctttctatag tgaatagagt taggcaggga tattcaccat tatcgtttca gacccacctc 6840
   ccaaccccga ggggacccga caggcccgaa ggaatagaag aagaaggtgg agagagagac 6900
   agagacagat ccattcgatt agtgaacgga tctcgacggt atcgccgaat tcacaaatgg 6960
   cagtattcat ccacaatttt aaaagaaaag gggggattgg ggggtacagt gcaggggaaa 7020
   gaatagtaga cataatagca acagacatac aaactaaaga attacaaaaa caaattacaa 7080
   aaattcaaaa ttttcgggtt tattacaggg acagcagaga tccactttgg ctgatacgcg 7140
   gatctacgcg tggatccgcg gcccggccgc ccccttcacc agatctatgc ccaagaagaa 7200
   gaggaaggtg tccaatttac tgaccgtaca ccaaaatttg cctgcattac cggtcgatgc 7260
   aacgagtgat gaggttcgca agaacctgat ggacatgttc agggatcgcc aggcgttttc 7320
   tgagcatacc tggaaaatgc ttctgtccgt ttgccggtcg tgggcggcat ggtgcaagtt 7380
   gaataaccgg aaatggtttc ccgcagaacc tgaagatgtt cgcgattatc ttctatatct 7440
   tcaggcgcgc ggtctggcag taaaaactat ccagcaacat ttgggccagc taaacatgct 7500
   tcatcgtcgg tccgggctgc cacgaccaag tgacagcaat gctgtttcac tggttatgcg 7560
   gcggatccga aaagaaaacg ttgatgccgg tgaacgtgca aaacaggctc tagcgttcga 7620
   acgcactgat ttcgaccagg ttcgttcact catggaaaat agcgatcgct gccaggatat 7680
   acgtaatctg gcatttctgg ggattgctta taacaccctg ttacgtatag ccgaaattgc 7740
   caggatcagg gttaaagata tctcacgtac tgacggtggg agaatgttaa tccatattgg 7800
   cagaacgaaa acgctggtta gcaccgcagg tgtagagaag gcacttagcc tggaggtaac 7860
   taaactggtc gagcgatgga tttccgtctc tggtgtagct gatgatccga ataactacct 7920
   gttttgccgg gtcagaaaaa atggtgttgc cgcgccatct gccaccagcc agctatcaac 7980
   tcgcgccctg gaagggattt ttgaagcaac tcatcgattg atttacggcg ctaaggatga 8040
   ctctggtcag agatacctgg cctggtctgg acacagtgcc cgtgtcggag ccgcgcgaga 8100
   tatggcccgc gctggagttt caataccgga gatcatgcaa gctggtggct ggaccaatgt 8160
   aaatattgtc atgaactata tccgtaacct ggatagtgaa acaggggcaa tggtgcgcct 8220
   gctggaagat ggcgattagc gggtcaccat ctagatggcc atatgatcga tacgtactag 8280
   tctcgagctc aagcttcgaa ttcccgataa tcaacctctg gattacaaaa tttgtgaaag 8340
   attgactggt attcttaact atgttgctcc ttttacgcta tgtggatacg ctgctttaat 8400
   gcctttgtat catgctattg cttcccgtat ggctttcatt ttctcctcct tgtataaatc 8460
   ctggttgctg tctctttatg aggagttgtg gcccgttgtc aggcaacgtg gcgtggtgtg 8520
   cactgtgttt gctgacgcaa cccccactgg ttggggcatt gccaccacct gtcagctcct 8580
   ttccgggact ttcgctttcc ccctccctat tgccacggcg gaactcatcg ccgcctgcct 8640
   tgcccgctgc tggacagggg ctcggctgtt gggcactgac aattccgtgg tgttgtcggg 8700
   gaagctgacg tcctttccat ggctgctcgc ctgtgttgcc acctggattc tgcgcgggac 8760
   gtccttctgc tacgtccctt cggccctcaa tccagcggac cttccttccc gcggcctgct 8820
   gccggctctg cggcctcttc cgcgtcttcg ccttcgccct cagacgagtc ggatctccct 8880
   ttgggccgcc tccccgcatc gggaattctg cagtcgacgg tacctttaag accaatgact 8940
   tacaaggcag ctgtagatct tagccacttt ttaaaagaaa aggggggact ggaagggcta 9000
   attcactccc aacgaagaca aaatcgtcga gagatgctgc atataagcag ctgctttttg 9060
   cttgtactgg gtctctctgg ttagaccaga tctgagcctg ggagctctct ggctaactag 9120
   ggaacccact gcttaagcct caataaagct tgccttgagt gcttcaagta gtgtgtgccc 9180
   gtctgttgtg tgactctggt aactagagat ccctcagacc cttttagtca gtgtggaaaa 9240
   tctctagcag tagtagttca tgtcatctta ttattcagta tttataactt gcaaagaaat 9300
   gaatatcaga gagtgagagg ccttgacatt ataatagatt tagcaggaat tgaactagga 9360
   gtggagcaca caggcaaagc tgcagaagta cttggaagaa gccaccagag atactcacga 9420
   ttctgcacat acctggctaa tcccagatcc taaggattac attaagttta ctaacattta 9480
   tataatgatt tatagtttaa agtataaact tatctaattt actattctga cagatattaa 9540
   ttaatcctca aatatcataa gagatgatta ctattatccc catttaacac aagaggaaac 9600
   tgagagggaa agatgttgaa gtaattttcc cacaattaca gcatccgtta gttacgactc 9660
   tatgatcttc tgacacaaat tccatttact cctcacccta tgactcagtc gaatatatca 9720
   aagttatgga cattatgcta agtaacaaat taccctttta tatagtaaat actgagtaga 9780
   ttgagagaag aaattgtttg caaacctgaa tagcttcaag aagaagagaa gtgaggataa 9840
   gaataacagt tgtcatttaa caagttttaa caagtaactt ggttagaaag ggattcaaat 9900
   gcataaagca agggataaat ttttctggca acaagactat acaatataac cttaaatatg 9960
   acttcaaata attgttggaa cttgataaaa ctaattaaat attattgaag attatcaata 10020
   ttataaatgt aatttacttt taaaaaggga acatagaaat gtgtatcatt agagtagaaa 10080
   acaatcctta ttatcacaat ttgtcaaaac aagtttgtta ttaacacaag tagaatactg 10140
   cattcaatta agttgactgc agattttgtg ttttgttaaa attagaaaga gataacaaca 10200
   atttgaatta ttgaaagtaa catgtaaata gttctacata cgttcttttg acatcttgtt 10260
   caatcattga tcgaagttct ttatcttgga agaatttgtt ccaaagactc tgaaataagg 10320
   aaaacaatct attatatagt ctcacacctt tgttttactt ttagtgattt caatttaata 10380
   atgtaaatgg ttaaaattta ttcttctctg agatcatttc acattgcaga tagaaaacct 10440
   gagactgggg taatttttat taaaatctaa tttaatctca gaaacacatc tttattctaa 10500
   catcaatttt tccagtttga tattatcata taaagtcagc cttcctcatc tgcaggttcc 10560
   acaacaaaaa tccaaccaac tgtggatcaa aaatattggg aaaaaattaa aaatagcaat 10620
   acaacaataa aaaaatacaa atcagaaaaa cagcacagta taacaacttt atttagcatt 10680
   tacaatctat taggtattat aagtaatcta g 10711

<210> 15
<211> 18
<212> DNA
<213> Artificial sequence

<220>
<223> SEQ ID NO: 15

<400> 15

tggcgcccga acagggac                                                    18

<210>  16
<211>  18
<212>  DNA
<213>  Artificial sequence

<220>
<223>  SEQ ID NO: 16

<400>  16
tggcgcccaa cgtggggc                                                    18

<210>  17
<211>  18
<212>  RNA
<213>  Artificial sequence

<220>
<223>  SEQ ID NO: 17

<400>  17
uggcgcccga acagggac                                                    18

<210>  18
<211>  18
<212>  RNA
<213>  Artificial sequence

<220>
<223>  SEQ ID NO: 18

<400>  18
uggcgcccaa cguggggc                                                    18